# EUROPEAN PATENT APPLICATION

(11) **EP 4 821 925 A2**
(43) Date of publication of application: **14.10.2026**
(21) Application number: 24900005.0
(22) Date of filing: 06.12.2024
(51) Int. Cl.: C07D 401/12, C07D 471/04, C07D 215/42, A61K 31/4709, A61P 17/04, A61P 29/00, A61P 37/06, A61P 37/08

(54) **MRGPRX2 ANTAGONIST AND USE THEREOF**

(30) Priority: 08.12.2023 CN 202311693381; 05.02.2024 CN 202410166370; 30.09.2024 CN 202411392820; 25.10.2024 CN 202411507978; 29.11.2024 CN 202411754629
(71) Applicant: Hubei Bio-Pharmaceutical Industrial Technological Institute Inc., Wuhan, Hubei 430075 (CN)
(72) Inventor: ZHANG, Xuejun, Wuhan, Hubei 430075 (CN); ZANG, Yang, Wuhan, Hubei 430075 (CN); YANG, Hui, Wuhan, Hubei 430075 (CN); ZHU, Haiyong, Wuhan, Hubei 430075 (CN); LI, Xiaopei, Wuhan, Hubei 430075 (CN); LI, Jinping, Wuhan, Hubei 430075 (CN); LIU, Lifei, Wuhan, Hubei 430075 (CN); QIAN, Lina, Wuhan, Hubei 430075 (CN); ZHU, Shengji, Wuhan, Hubei 430075 (CN); LI, Yuqiong, Wuhan, Hubei 430075 (CN)
(74) Representative: Gille Hrabal Partnerschaftsgesellschaft mbB Patentanwälte
(86) International application number: PCT/CN2024/137602
(87) International publication number: WO 2025/119372

(57) **Abstract**

Provided in the present invention are a compound as shown in formula IA, a tautomer, stereoisomer, hydrate, solvate, pharmaceutically acceptable salt or prodrug thereof. The compound has a good MRGPRX2 antagonistic effect.

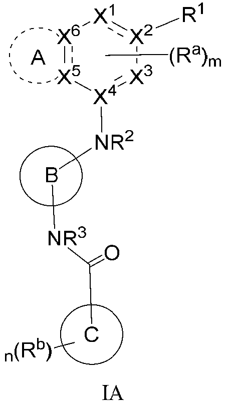

## Description

The present application claims priority to:
a prior application with the patent application No. 202311693381.1 entitled "MRGPRX2 ANTAGONIST AND USE THEREOF" and filed with China National Intellectual Property Administration by the applicant on December 8, 2023;
a prior application with the patent application No. 202410166370.6 entitled "MRGPRX2 ANTAGONIST AND USE THEREOF" and filed with China National Intellectual Property Administration by the applicant on February 5, 2024;
a prior application with the patent application No. 202411392820.X entitled "MRGPRX2 ANTAGONIST AND USE THEREOF" and filed with China National Intellectual Property Administration by the applicant on September 30, 2024;
a prior application with the patent application No. 202411507978.7 entitled "MRGPRX2 ANTAGONIST AND USE THEREOF" and filed with China National Intellectual Property Administration by the applicant on October 25, 2024; and
a prior application with the patent application No. 202411754629.5 entitled "MRGPRX2 ANTAGONIST AND USE THEREOF" and filed with China National Intellectual Property Administration by the applicant on November 29, 2024,
which are incorporated herein by reference in their entirety.

### TECHNICAL FIELD

The present disclosure pertains to the field of pharmaceuticals, and particularly relates to an MRGPRX2 antagonist and use thereof.

### BACKGROUND

Mast cells (MCs) are tissue-resident immune cells originating from the hematopoietic lineage. MCs reside in a variety of connective tissues and vascularized organs, and are present in the greatest number and highest density at interfaces between the internal and external environments, where they respond to foreign organisms and antigens and function as sentinels. These sites include the dermis, skeletal muscle, oral mucosa, gastrointestinal mucosa and submucosa, conjunctiva, pulmonary alveoli and airways, as well as the atrial appendage. MCs in the dermis are typically located in close proximity to blood vessels, nerves, and lymphatic vessels. Upon activation via the human high-affinity immunoglobulin E (IgE) receptor (FcεRI) and Mas-related G protein-coupled receptor X2 (MRGPRX2), MCs undergo degranulation, thereby releasing a variety of bioactive substances (such as histamine and proteases) or *de novo* synthesizing new prostaglandins, leukotrienes, and certain cytokines, which play central roles in host defense, inflammation, and allergic responses.

Mas-related G protein-coupled receptors (MRGPRs) are divided, based on different receptor members, into nine subfamilies, namely MRGPRA, B, C, D, E, F, G, H, and the primate-specific MRGPRX. Each subfamily further includes different subtypes, such as MRGPRX1, MRGPRX2, MRGPRX3, and MRGPRX4. Transcriptomic analyses have shown that most members of the MRGPR family are expressed in peripheral neurons, whereas MRGPRX2 is predominantly expressed in skin MCs, with an expression level even higher than that of Fcε-RI. MRGPRX2 can sense various endogenous or exogenous agonists, including polycationic compounds, polypeptides and the like, thereby triggering mast cell degranulation.

Studies have shown that inappropriate activation of MRGPRX2 may lead to mast cell-related diseases, including drug-induced pseudo-allergic reactions, rosacea, atopic dermatitis, allergic contact dermatitis, urticaria, pruritus, mastocytosis, interstitial cystitis, pain, rheumatoid arthritis, asthma, ulcerative colitis, and the like. Currently, an Investigational New Drug (IND) application for the MRGPRX2 antagonist EP262 has been approved by the FDA for the treatment of mast cell-related diseases (e.g., chronic urticaria). Preclinical studies have demonstrated that EP262 is capable of effectively inhibiting mast cell activation and degranulation induced by various MRGPRX2 agonists, and blocking the release of trypsin and inflammatory factors in human mast cells. Oral administration of EP262 can efficiently inhibit agonist-induced mast cell degranulation and increases in vascular permeability in MRGPRX2 KI mice, indicating significant potential efficacy in the treatment of mast cell-related diseases. These findings indicate that the development of novel MRGPRX2 antagonists for treating a variety of mast cell-related diseases represents a promising approach.

Currently, no drug acting as an MRGPRX2 antagonist has been approved for marketing. Therefore, the development of novel compounds capable of antagonizing MRGPRX2 activity is of positive significance for disease treatment.

### SUMMARY

The object of the present disclosure is to provide a novel compound for use as an MRGPRX2 antagonist.

In a first aspect of the present disclosure, provided is a compound represented by formula IA, or a tautomer, a stereoisomer, a hydrate, a solvate, a pharmaceutically acceptable salt, or a prodrug thereof: 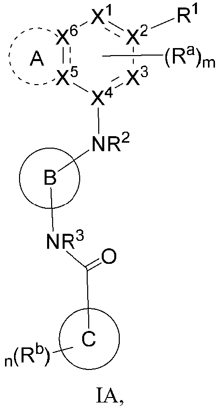 wherein
X¹, X², X³, X⁴, X⁵, and X⁶ each independently represent a ring atom;
X¹, X², X³, X⁵, and X⁶ are each independently N, CH₂, CH, or C;
X⁴ is C;
each of the bonds linked between X¹ and X² and between X⁵ and X⁶ is a unsubstituted bond or a double bond;
in a group fragment 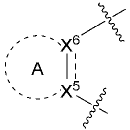 linked by X⁵ and X⁶, A is absent, and X⁵ and X⁶ are each independently substituted with R⁴ or R⁵; or when A is present, A, together with X⁵ and X⁶ ring atoms, forms 6- to 10-membered aryl, 3- to 11-membered heterocycloalkyl, or 5- to 10-membered heteroaryl, and A is further substituted with R^{c}; one or more R^{c} substitutions are present, and when a plurality of R^{c} substituents are present, the substituents are identical or different; heteroatoms in the heterocycloalkyl and heteroaryl are selected from one or more of N, O, and S, and the number of the heteroatoms is 1, 2, or 3;
and when A, together with X⁵ and X⁶ ring atoms, forms 6-membered aryl, R^{a} is oxo;
or, when A, together with X⁵ and X⁶ ring atoms, forms 5-membered heteroaryl, and when R^{c} is not H, R¹ is C₁₋₆ haloalkyl; heteroatoms in the heteroaryl are selected from one or more of N, O, and S, and the number of the heteroatoms is 1, 2, or 3;
ring B is C₃₋₁₂ cycloalkyl;
ring C is 6- to 10-membered aryl or 5- to 10-membered heteroaryl; heteroatoms in the heteroaryl are selected from one or more of N, O, and S, and the number of the heteroatoms is 1, 2, or 3;
R^{a}, R^{c}, R¹, R², R³, R⁴, and R⁵ are each independently H, halogen, cyano, amino, oxo, C₁₋₆ alkyl, C₁₋₆ alkyl substituted with hydroxyl, C₂₋₆ alkenyl, C₂₋₆ alkynyl, C₁₋₆ haloalkyl, C₃₋₈ cycloalkyl, halogenated C₃₋₈ cycloalkyl, -OR^{f}, - C(O)OR^{f}, -OC(O)R^{f}, -N(R^{f})₂, -N(R^{f})C(O)R^{f}, -N(R^{f})S(O)₂R^{f}, or -S(O)₂R^{f}; R^{f} is selected from H, C₁₋₆ alkyl, C₂₋₆ alkenyl, C₂₋₆ alkynyl, C₁₋₆ alkylamino, -(CH₂)rR^{g}, 6- to 10-membered aryl, C₃₋₈ cycloalkyl, 5- to 10-membered heteroaryl, or 5- to 10-membered heterocycloalkyl, or two R^{f} groups, together with an atom attached thereto, form 5- to 11-membered heterocycloalkyl; R^{g} is H, C₁₋₆ alkyl, C₂₋₆ alkenyl, C₂₋₆ alkynyl, C₃₋₈ cycloalkyl, 6- to 10-membered aryl, 5- to 10-membered heteroaryl, or 5- to 10-membered heterocycloalkyl; heteroatoms in the heterocycloalkyl and heteroaryl are selected from one or more of N, O, and S, and the number of the heteroatoms is 1, 2, or 3;
R^{b} is H, halogen, hydroxyl, amino, cyano, carbonyl, oxo, C₁₋₆ alkyl, C₃₋₈ cycloalkyl, C₂₋₆ alkenyl, C₂₋₆ alkynyl, C₁₋₆ haloalkyl, C₃₋₈ halocycloalkyl, C₁₋₆ hydroxyalkyl, C₁₋₆ alkylcarbonyl, C₁₋₆ alkoxy, C₁₋₆ haloalkoxy or 3- to 10-membered heterocycloalkyl, -(CHR^{j})₀₋₃-S(=O)(-NR^{j})-R^{k}, -(CHR^{j})₁₋₃-S(O)₂-R^{k}, -(CHR^{j})₁₋₃-OC(O)-N(R^{k})₂, - (CHR^{j})₀₋₃-(N=)S(=O)(R^{k})₂, or -(CHR^{j})₀₋₃-P(=O)(R^{k})₂; the C₁₋₆ alkyl, C₃₋₈ cycloalkyl, C₂₋₆ alkenyl, C₂₋₆ alkynyl, C₁₋₆ haloalkyl, C₃₋₈ halocycloalkyl, C₁₋₆ hydroxyalkyl, C₁₋₆ alkylcarbonyl, C₁₋₆ alkoxy, and C₁₋₆ haloalkoxy are optionally substituted with one or more R^{d}; R^{d} is selected from the following substituents: halogen, hydroxyl, amino, nitro, cyano, carbonyl, oxo, carboxyl, C₁₋₆ alkyl, C₂₋₆ alkenyl, C₂₋₆ alkynyl, C₁₋₆ haloalkyl, C₁₋₆ hydroxyalkyl, C₁₋₆ alkylcarbonyl, C₁₋₆ alkoxy, and C₁₋₆ haloalkoxy; when a plurality of R^{d} substituents are present, the R^{d} substituents are identical or different; heteroatoms in the heterocycloalkyl are selected from one or more of N, O, and S, and the number of the heteroatoms is 1, 2, or 3;
R^{j} and R^{k} are each independently H, cyano, amino, C₁₋₆ alkyl, C₁₋₆ alkylamino, -(CH₂)₀₋₃C₃₋₈ cycloalkyl, -COC₁₋₆ alkyl, C₁₋₆ haloalkyl, C₁₋₆ alkyl substituted with hydroxyl, or unsubstituted 5- to 10-membered heterocycloalkyl; heteroatoms in the heterocycloalkyl are selected from one or more of N, O, and S, and the number of the heteroatoms is 1, 2, or 3;
m, n, and r are 0, 1, 2, or 3, respectively.

According to an embodiment of the present disclosure, R^{b} is H, F, Cl, -CN, methyl, amino, methoxy, oxo, -CH₂CF₃, -CF₃, -CHF₂, -CH₂CHF₂, -CH₂-SO₂-CH₃, -CH(CH₃)SO₂CH₃, -CH₂S(=O)(=NCH₃)-CH₃,

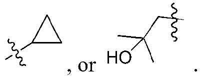

According to an embodiment of the present disclosure, R^{a}, R^{c}, R¹, R², and R³ are each independently H, F, Cl, methyl, ethyl, amino, oxo, -CHF₂, -CH₂CF₃, -CF₃,

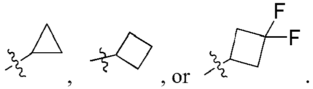

According to an embodiment of the present disclosure, in the group fragment 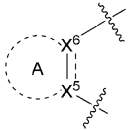 linked by X⁵ and X⁶, when A is absent and X⁵ and X⁶ are each independently substituted with R⁴ or R⁵, a structural fragment 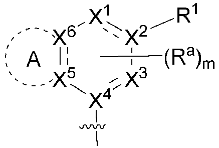 is selected from 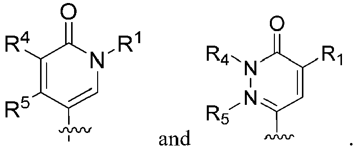 In some embodiments, R⁴ and R⁵ are each independently H, F, Cl, methyl, -CF₃, or -CHF₂.

In some embodiments, in the group fragment 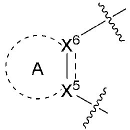 linked by X⁵ and X⁶, when A is absent and X⁵ and X⁶ are each independently substituted with R⁴ or R⁵, a structural fragment 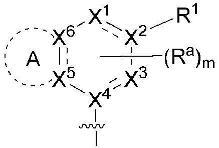 is selected from

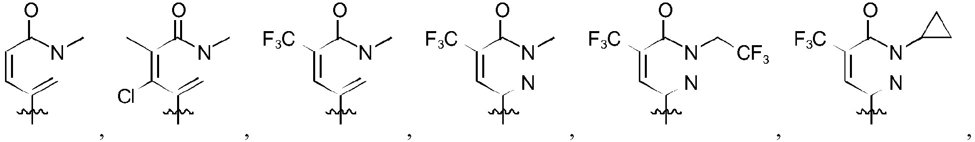

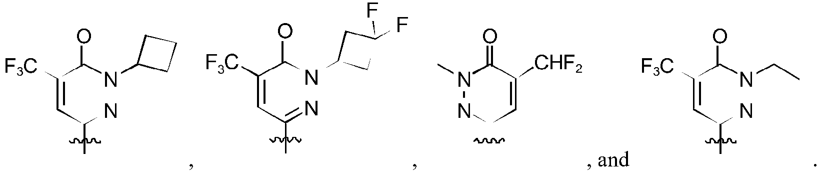

According to an embodiment of the present disclosure, in the group fragment 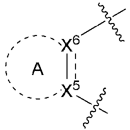 linked by X⁵ and X⁶, when A is present, A, together with X⁵ and X⁶ ring atoms, forms 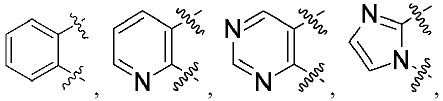 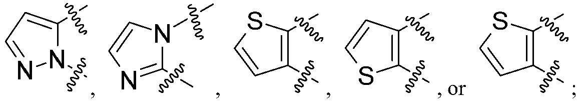 and A is further substituted with R^{c}.

In some embodiments, when A is present, the structural fragment 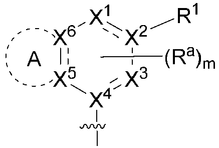 is selected from 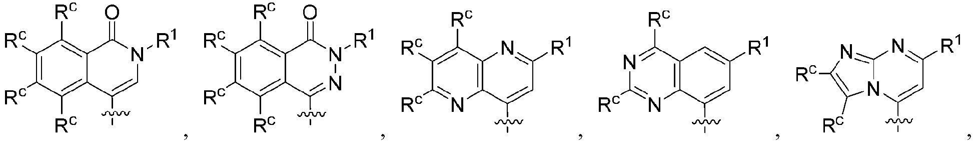 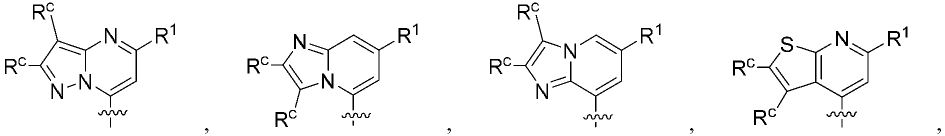 and

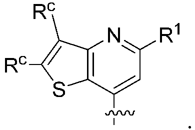

In some embodiments, when A is present, the structural fragment 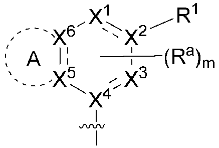 is selected from 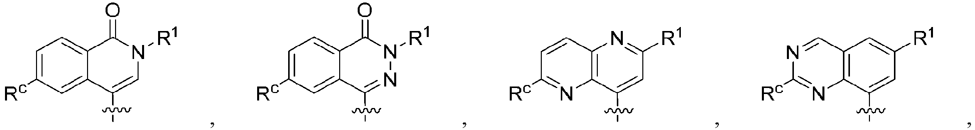 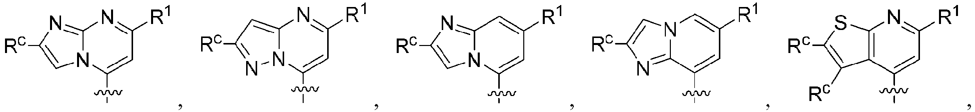 and

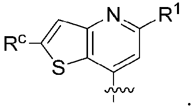

In some embodiments, the structural fragment 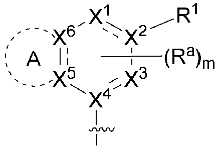 is selected from the following structures:

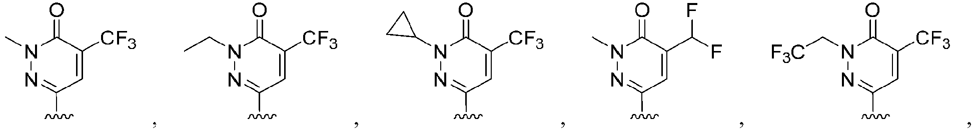

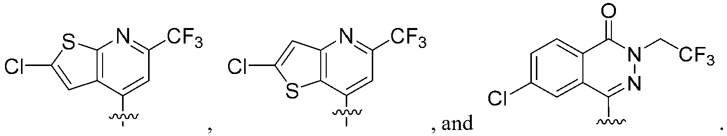

According to an embodiment of the present disclosure, ring C is 6- to 8-membered aryl or 5- to 10-membered heteroaryl; heteroatoms in the heteroaryl are selected from one or more of N, O, and S, and the number of the heteroatoms is 1, 2, or 3.

According to an embodiment of the present disclosure, ring C is 5- to 6-membered nitrogen-containing heteroaryl, and the number of the nitrogen atoms is 1, 2, or 3.

According to an embodiment of the present disclosure, ring C is phenyl, pyrazolyl, or pyridinyl.

According to an embodiment of the present disclosure, ring B is cyclopropyl, cyclobutyl, cyclopentyl, cyclohexyl, or cycloheptyl;

According to an embodiment of the present disclosure, the structural fragment 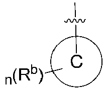 is selected from

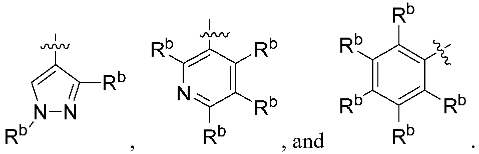

In some embodiments, the structural fragment 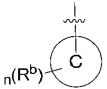 is selected from 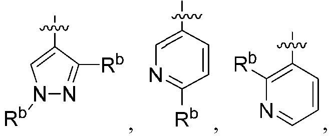 and

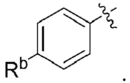

In some embodiments, the formula IA may be selected from IA-1 as follows: 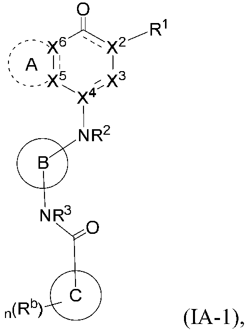 wherein A, ring B, ring C, X², X³, X⁴, X⁵, X⁶, R¹, R², R³, R^{b}, m, and n are as defined in formula IA.

In a second aspect of the present disclosure, provided is a compound represented by formula I, or a tautomer, a stereoisomer, a hydrate, a solvate, a pharmaceutically acceptable salt, or a prodrug thereof: 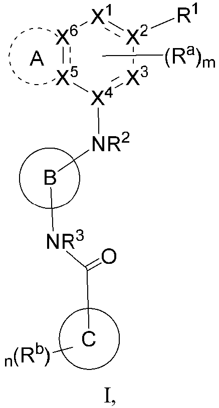
according to an embodiment of the present disclosure, the formula IA according to the first aspect is selected from formula I, and the groups in the formula I are as defined in formula IA;
according to an embodiment of the present disclosure,
in the formula I,
X¹, X², X³, X⁴, X⁵, and X⁶ each independently represent a ring atom;
X¹, X², X³, X⁵, and X⁶ are each independently N, CH₂, CH, or C;
X⁴ is C;
each of the bonds linked between X¹ and X² and between X⁵ and X⁶ is a single bond or a double bond;
in a group fragment 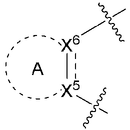 linked by X⁵ and X⁶, A is absent, and X⁵ and X⁶ are each independently substituted with R⁴ or R⁵; or when A is present, A, together with X⁵ and X⁶ ring atoms, forms 6- to 10-membered aryl, 3- to 11-membered heterocycloalkyl, or 5- to 10-membered heteroaryl; the heteroatoms are independently selected from one or more of N, O, and S; and A is further substituted with R^{c}; one or more R^{c} substitutions are present, and when a plurality of R^{c} substituents are present, the substituents are identical or different; heteroatoms in the heterocycloalkyl and heteroaryl are selected from one or more of N, O, and S, and the number of the heteroatoms is 1, 2, or 3;
and when A, together with X⁵ and X⁶ ring atoms, forms 6-membered aryl, R^{a} is oxo;
or, when A, together with X⁵ and X⁶ ring atoms, forms 5-membered heteroaryl, and when R^{c} is not H, R¹ is C₁₋₆ haloalkyl; heteroatoms in the heteroaryl are selected from one or more of N, O, and S, and the number of the heteroatoms is 1, 2, or 3;
ring B is C₃₋₁₂ cycloalkyl;
ring C is 6- to 10-membered aryl or 5- to 10-membered heteroaryl; heteroatoms in the heteroaryl are selected from one or more of N, O, and S, and the number of the heteroatoms is 1, 2, or 3;
R^{a}, R^{c}, R¹, R², R³, R⁴, and R⁵ are each independently H, halogen, cyano, amino, oxo, C₁₋₆ alkyl, C₁₋₆ alkyl substituted with hydroxyl, C₂₋₆ alkenyl, C₂₋₆ alkynyl, C₁₋₆ haloalkyl, C₃₋₈ cycloalkyl, halogenated C₃₋₈ cycloalkyl, -OR^{f}, - C(O)OR^{f}, -OC(O)R^{f}, -N(R^{f})₂, -N(R^{f})C(O)R^{f}, -N(R^{f})S(O)₂R^{f}, or -S(O)₂R^{f}; R^{f} is selected from H, C₁₋₆ alkyl, C₂₋₆ alkenyl, C₂₋₆ alkynyl, C₁₋₆ alkylamino, -(CH₂)rR^{g}, 6- to 10-membered aryl, C₃₋₈ cycloalkyl, 5- to 10-membered heteroaryl, or 5- to 10-membered heterocycloalkyl, or two R^{f} groups, together with an atom attached thereto, form 5- to 11-membered heterocycloalkyl; R^{g} is H, C₁₋₆ alkyl, C₂₋₆ alkenyl, C₂₋₆ alkynyl, C₃₋₈ cycloalkyl, 6- to 10-membered aryl, 5- to 10-membered heteroaryl, or 5- to 10-membered heterocycloalkyl; heteroatoms in the heterocycloalkyl and heteroaryl are selected from one or more of N, O, and S, and the number of the heteroatoms is 1, 2, or 3;
R^{b} is H, halogen, hydroxyl, amino, cyano, carbonyl, oxo, C₁₋₆ alkyl, C₃₋₈ cycloalkyl, C₂₋₆ alkenyl, C₂₋₆ alkynyl, C₁₋₆ haloalkyl, C₃₋₈ halocycloalkyl, C₁₋₆ hydroxyalkyl, C₁₋₆ alkylcarbonyl, C₁₋₆ alkoxy, C₁₋₆ haloalkoxy or 3- to 10-membered heterocycloalkyl; the C₁₋₆ alkyl, C₃₋₈ cycloalkyl, C₂₋₆ alkenyl, C₂₋₆ alkynyl, C₁₋₆ haloalkyl, C₃₋₈ halocycloalkyl, C₁₋₆ hydroxyalkyl, C₁₋₆ alkylcarbonyl, C₁₋₆ alkoxy, and C₁₋₆ haloalkoxy are optionally substituted with one or more R^{d}; R^{d} is selected from the following substituents: halogen, hydroxyl, amino, nitro, cyano, carbonyl, oxo, carboxyl, C₁₋₆ alkyl, C₂₋₆ alkenyl, C₂₋₆ alkynyl, C₁₋₆ haloalkyl, C₁₋₆ hydroxyalkyl, C₁₋₆ alkylcarbonyl, C₁₋₆ alkoxy, and C₁₋₆ haloalkoxy; when a plurality of R^{d} substituents are present, the R^{d} substituents are identical or different; heteroatoms in the heterocycloalkyl are selected from one or more of N, O, and S, and the number of the heteroatoms is 1, 2, or 3;
m, n, and r are 0, 1, 2, or 3, respectively.

In the present disclosure, some substituents in the compound represented by formula IA or formula I may be defined as follows, and the substituents which are not mentioned are defined as in any one of the above schemes.

In a preferred embodiment of the present disclosure, the compound represented by formula IA or formula I is selected from the following structure: 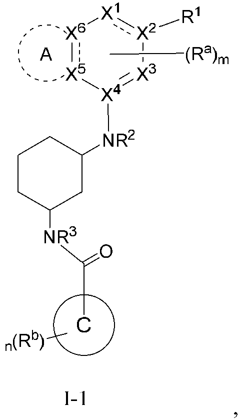 wherein in the group fragment 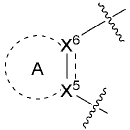 linked by X⁵ and X⁶, A is as defined in the first aspect of the present disclosure; ring C is 6- to 10-membered aryl or 5- to 10-membered heteroaryl; heteroatoms in the heteroaryl are selected from one or more of N, O, and S, and the number of the heteroatoms is 1, 2, or 3; X¹, X², X³, X⁴, X⁵, X⁶, R¹, R², R³, R^{a}, R^{b}, m, and n are as defined in the first aspect of the present disclosure.

In a preferred embodiment of the present disclosure, the compound represented by formula IA is selected from the following structure: 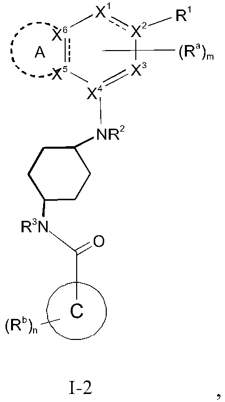 wherein in the group fragment 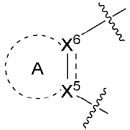 linked by X⁵ and X⁶, A is as defined in the first aspect of the present disclosure; ring C is 6- to 10-membered aryl or 5- to 10-membered heteroaryl; heteroatoms in the heteroaryl are selected from one or more of N, O, and S, and the number of the heteroatoms is 1, 2, or 3; X¹, X², X³, X⁴, X⁵, X⁶, R¹, R², R³, R^{a}, R^{b}, m, and n are as defined in the first aspect of the present disclosure.

In a preferred embodiment of the present disclosure, the compound represented by formula I is selected from the following structure: 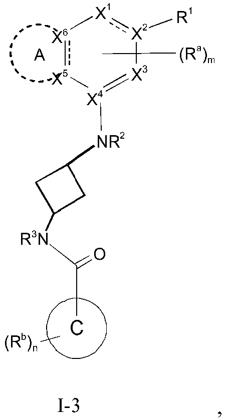 wherein in the group fragment 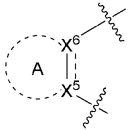 linked by X⁵ and X⁶, A is as defined in the first aspect of the present disclosure; ring C is 6- to 10-membered aryl or 5- to 10-membered heteroaryl; heteroatoms in the heteroaryl are selected from one or more of N, O, and S, and the number of the heteroatoms is 1, 2, or 3; X¹, X², X³, X⁴, X⁵, X⁶, R¹, R², R³, R^{a}, R^{b}, m, and n are as defined in the first aspect of the present disclosure.

In a preferred embodiment of the present disclosure, the compound represented by formula IA is selected from the following structure: 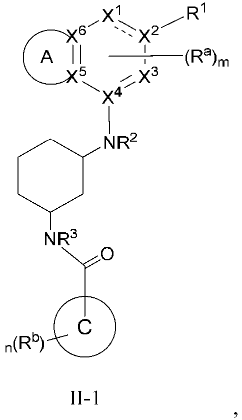 wherein A, together with X⁵ and X⁶ ring atoms, forms 6- to 8-membered aryl, 3- to 8-membered heterocycloalkyl, 5-membered heteroaryl, or 6-membered heteroaryl; the heteroatoms are independently selected from one or more of N, O, and S; A is further substituted with R^{c}; one or more R^{c} substitutions are present, and when a plurality of R^{c} substituents are present, the substituents are identical or different; and when A, together with X⁵ and X⁶ ring atoms, forms 6-membered aryl, R^{a} is oxo; or, when A, together with X⁵ and X⁶ ring atoms, forms 5-membered heteroaryl and R^{c} is not H, R¹ is C₁₋₆ haloalkyl; heteroatoms in the heterocycloalkyl and heteroaryl are selected from one or more of N, O, and S, and the number of the heteroatoms is 1, 2, or 3; ring C is 6- to 10-membered aryl or 5- to 8-membered heteroaryl; heteroatoms in the heteroaryl are selected from one or more of N, O, and S, and the number of the heteroatoms is 1, 2, or 3; R^{b} is H, halogen, hydroxyl, amino, cyano, carbonyl, oxo, C₁₋₆ alkyl, C₃₋₈ cycloalkyl, C₂₋₆ alkenyl, C₂₋₆ alkynyl, C₁₋₆ haloalkyl, C₃₋₈ halocycloalkyl, C₁₋₆ hydroxyalkyl, C₁₋₆ alkylcarbonyl, C₁₋₆ alkoxy, C₁₋₆ haloalkoxy or 3- to 8-membered heterocycloalkyl, -(CHR^{j})₀₋₃-S(=O)(=NR^{j})-R^{k}, -(CHR^{j})₁₋₃-S(O)₂-R^{k}, -(CHR^{j})₁₋₃-OC(O)-N(R^{k})₂, -(CHR^{j})₀₋₃-(N=)S(=O)(R^{k})₂, or -(CHR^{j})₀₋₃-P(=O)(R^{k})₂; the C₁₋₆ alkyl, C₃₋₈ cycloalkyl, C₂₋₆ alkenyl, C₂₋₆ alkynyl, C₁₋₆ haloalkyl, C₃₋₈ halocycloalkyl, C₁₋₆ hydroxyalkyl, C₁₋₆ alkylcarbonyl, C₁₋₆ alkoxy, and C ₁₋₆ haloalkoxy are optionally substituted with one or more R^{d}; R^{d} is selected from the following substituents: halogen, hydroxyl, amino, nitro, cyano, carbonyl, oxo, carboxyl, C₁₋₆ alkyl, C₂₋₆ alkenyl, C₂₋₆ alkynyl, C₁₋₆ haloalkyl, C₁₋₆ hydroxyalkyl, C₁₋₆ alkylcarbonyl, C₁₋₆ alkoxy, and C₁₋₆ haloalkoxy; heteroatoms in the heterocycloalkyl are selected from one or more of N, O, and S, and the number of the heteroatoms is 1, 2, or 3; R^{j} and R^{k} are each independently H, cyano, amino, C₁₋₆ alkyl, C₁₋₆ alkylamino, -(CH₂)₀₋₃C₃₋₈ cycloalkyl, -COC₁₋₆ alkyl, C₁₋₆ haloalkyl, C₁₋₆ alkyl substituted with hydroxyl, or unsubstituted 5- to 10-membered heterocycloalkyl; R^{a}, R^{c}, R¹, R², and R³ are each independently H, halogen, cyano, C₁₋₆ alkyl, C₂₋₆ alkenyl, C₂₋₆ alkynyl, C₁₋₆ haloalkyl, C₃₋₈ cycloalkyl, or halogenated C₃₋₈ cycloalkyl; X¹, X², X³, X⁴, X⁵, X⁶, m, and n are as defined in the first aspect of the present disclosure. In a preferred embodiment of the present disclosure, R^{b} is H, F, Cl, -CN, methyl, amino, oxo, -CH₂CF₃, -CF₃, -CHF₂, -CH₂CHF₂, -CH₂-SO₂-CH₃, -CH(CH₃)SO₂CH₃, - CH₂S(=O)(-NCH₃)-CH₃, 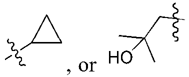 . In a preferred embodiment of the present disclosure, R^{a}, R^{c}, R¹, R², and R³ are each independently H, F, Cl, methyl, amino, oxo, -CHF₂, -CH₂CF₃, -CF₃, 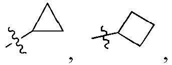 or

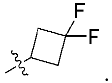

In a preferred embodiment of the present disclosure, the compound represented by formula IA is selected from the following structure: 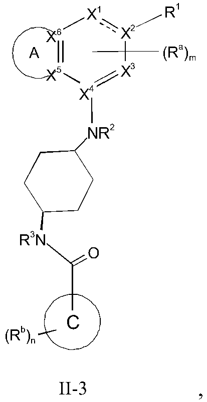 wherein A, together with X⁵ and X⁶ ring atoms, forms 6- to 8-membered aryl, 3- to 8-membered heterocycloalkyl, 5-membered heteroaryl, or 6-membered heteroaryl; the heteroatoms are independently selected from one or more of N, O, and S; A is further substituted with R^{c}; one or more R^{c} substitutions are present, and when a plurality of R^{c} substituents are present, the substituents are identical or different; and when A, together with X⁵ and X⁶ ring atoms, forms 6-membered aryl, R^{a} is oxo; or, when A, together with X⁵ and X⁶ ring atoms, forms 5-membered heteroaryl and R^{c} is not H, R¹ is C₁₋₆ haloalkyl; heteroatoms in the heterocycloalkyl and heteroaryl are selected from one or more of N, O, and S, and the number of the heteroatoms is 1, 2, or 3; ring C is 6- to 10-membered aryl or 5- to 8-membered heteroaryl; heteroatoms in the heteroaryl are selected from one or more of N, O, and S, and the number of the heteroatoms is 1, 2, or 3; R^{b} is H, halogen, hydroxyl, amino, cyano, carbonyl, oxo, C₁₋₆ alkyl, C₃₋₈ cycloalkyl, C₂₋₆ alkenyl, C₂₋₆ alkynyl, C₁₋₆ haloalkyl, C₃₋₈ halocycloalkyl, C₁₋₆ hydroxyalkyl, C₁₋₆ alkylcarbonyl, C₁₋₆ alkoxy, C₁₋₆ haloalkoxy or 3- to 8-membered heterocycloalkyl, -(CHR^{j})₀₋₃-S(=O)(=NR^{j})-R^{k}, -(CHR^{j})₁₋₃-S(O)₂-R^{k}, -(CHR^{j})₁₋₃-OC(O)-N(R^{k})₂, -(CHR^{j})₀₋₃-(N=)S(=O)(R^{k})₂, or -(CHR^{j})₀₋₃-P(=O)(R^{k})₂; the C₁₋₆ alkyl, C₃₋₈ cycloalkyl, C₂₋₆ alkenyl, C₂₋₆ alkynyl, C₁₋₆ haloalkyl, C₃₋₈ halocycloalkyl, C₁₋₆ hydroxyalkyl, C₁₋₆ alkylcarbonyl, C₁₋₆ alkoxy, and C ₁₋₆ haloalkoxy are optionally substituted with one or more R^{d}; R^{d} is selected from the following substituents: halogen, hydroxyl, amino, nitro, cyano, carbonyl, oxo, carboxyl, C₁₋₆ alkyl, C₂₋₆ alkenyl, C₂₋₆ alkynyl, C₁₋₆ haloalkyl, C₁₋₆ hydroxyalkyl, C₁₋₆ alkylcarbonyl, C₁₋₆ alkoxy, and C₁₋₆ haloalkoxy; heteroatoms in the heterocycloalkyl are selected from one or more of N, O, and S, and the number of the heteroatoms is 1, 2, or 3; R^{j} and R^{k} are each independently H, cyano, amino, C₁₋₆ alkyl, C₁₋₆ alkylamino, - (CH₂)₀₋₃C₃₋₈ cycloalkyl, -COC₁₋₆ alkyl, C₁₋₆ haloalkyl, C₁₋₆ alkyl substituted with hydroxyl, or unsubstituted 5- to 10-membered heterocycloalkyl; R^{a}, R^{c}, R¹, R², and R³ are each independently H, halogen, cyano, C₁₋₆ alkyl, C₂₋₆ alkenyl, C₂₋₆ alkynyl, C₁₋₆ haloalkyl, C₃₋₈ cycloalkyl, or halogenated C₃₋₈ cycloalkyl; X¹, X², X³, X⁴, X⁵, X⁶, m, and n are as defined in the first aspect of the present disclosure. In a preferred embodiment of the present disclosure, R^{b} is H, F, Cl, -CN, methyl, methoxy, amino, oxo, -CH₂CF₃, -CF₃, -CHF₂, -CH₂CHF₂, -CH₂-SO₂-CH₃, - CH(CH₃)SO₂CH₃, -CH₂S(=O)(=NCH₃)-CH₃, 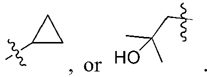 In a preferred embodiment of the present disclosure, R^{a}, R^{c}, R¹, R², and R³ are each independently H, F, Cl, methyl, ethyl, amino, oxo, -CHF₂, -CH₂CF₃, - CF₃,

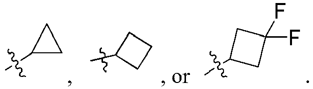

In a preferred embodiment of the present disclosure, the compound represented by formula I is selected from the following structure: 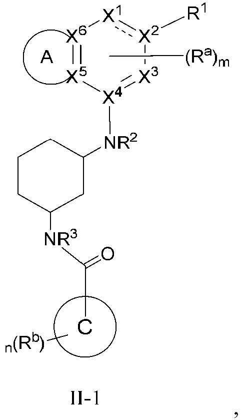 wherein A, together with X⁵ and X⁶ ring atoms, forms 6- to 8-membered aryl, 3- to 8-membered heterocycloalkyl, 5-membered heteroaryl, or 6-membered heteroaryl; the heteroatoms are independently selected from one or more of N, O, and S; A is further substituted with R^{c}; one or more R^{c} substitutions are present, and when a plurality of R^{c} substituents are present, the substituents are identical or different; and when A, together with X⁵ and X⁶ ring atoms, forms 6-membered aryl, R^{a} is oxo; or, when A, together with X⁵ and X⁶ ring atoms, forms 5-membered heteroaryl and R^{c} is not H, R¹ is C₁₋₆ haloalkyl; heteroatoms in the heterocycloalkyl and heteroaryl are selected from one or more of N, O, and S, and the number of the heteroatoms is 1, 2, or 3; ring C is 6- to 10-membered aryl or 5- to 8-membered heteroaryl; heteroatoms in the heteroaryl are selected from one or more of N, O, and S, and the number of the heteroatoms is 1, 2, or 3; R^{b} is H, halogen, hydroxyl, amino, cyano, carbonyl, oxo, C₁₋₆ alkyl, C₃₋₈ cycloalkyl, C₂₋₆ alkenyl, C₂₋₆ alkynyl, C₁₋₆ haloalkyl, C₃₋₈ halocycloalkyl, C₁₋₆ hydroxyalkyl, C₁₋₆ alkylcarbonyl, C₁₋₆ alkoxy, C₁₋₆ haloalkoxy, or 3- to 8-membered heterocycloalkyl; heteroatoms in the heterocycloalkyl are selected from one or more of N, O, and S, and the number of the heteroatoms is 1, 2, or 3; R^{a}, R^{c}, R¹, R², and R³ are each independently H, halogen, cyano, C₁₋₆ alkyl, C₂₋₆ alkenyl, C₂₋₆ alkynyl, C₁₋₆ haloalkyl, C₃₋₈ cycloalkyl, and halogenated C₃₋₈ cycloalkyl; X¹, X², X³, X⁴, X⁵, X⁶, m, and n are as defined in the first aspect of the present disclosure. In a preferred embodiment of the present disclosure, R^{b} is H, F, Cl, methyl, amino, oxo, -CH₂CF₃, or -CF₃. In a preferred embodiment of the present disclosure, R^{a}, R^{c}, R¹, R², and R³ are each independently H, F, Cl, methyl, amino, oxo, -CH₂CF₃, -CF₃,

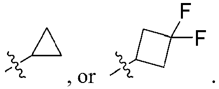

In a preferred embodiment of the present disclosure, the compound represented by formula IA is selected from the following structure: 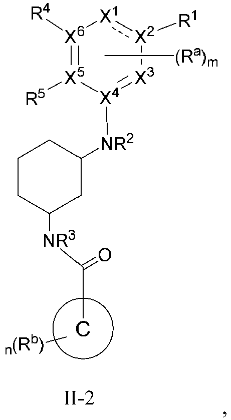 wherein A, together with X⁵ and X⁶ ring atoms, forms 6- to 8-membered aryl, 3- to 8-membered heterocycloalkyl, 5-membered heteroaryl, or 6-membered heteroaryl; the heteroatoms are independently selected from one or more of N, O, and S; A is further substituted with R^{c}; one or more R^{c} substitutions are present, and when a plurality of R^{c} substituents are present, the substituents are identical or different; and when A, together with X⁵ and X⁶ ring atoms, forms 6-membered aryl, R^{a} is oxo; or, when A, together with X⁵ and X⁶ ring atoms, forms 5-membered heteroaryl and R^{c} is not H, R¹ is C₁₋₆ haloalkyl; heteroatoms in the heterocycloalkyl and heteroaryl are selected from one or more of N, O, and S, and the number of the heteroatoms is 1, 2, or 3; ring C is 6- to 10-membered aryl or 5- to 8-membered heteroaryl; heteroatoms in the heteroaryl are selected from one or more of N, O, and S, and the number of the heteroatoms is 1, 2, or 3; R^{b} is H, halogen, hydroxyl, amino, cyano, carbonyl, oxo, C₁₋₆ alkyl, C₃₋₈ cycloalkyl, C₂₋₆ alkenyl, C₂₋₆ alkynyl, C₁₋₆ haloalkyl, C₃₋₈ halocycloalkyl, C₁₋₆ hydroxyalkyl, C₁₋₆ alkylcarbonyl, C₁₋₆ alkoxy, C₁₋₆ haloalkoxy or 3- to 8-membered heterocycloalkyl, -(CHR^{j})₀₋₃-S(=O)(=NR^{j})-R^{k}, -(CHR^{j})₁₋₃-S(O)₂-R^{k}, -(CHR^{j})₁₋₃-OC(O)-N(R^{k})₂, -(CHR^{j})₀₋₃-(N=)S(=O)(R^{k})₂, or -(CHR^{j})₀₋₃-P(=O)(R^{k})₂; the C₁₋₆ alkyl, C₃₋₈ cycloalkyl, C₂₋₆ alkenyl, C₂₋₆ alkynyl, C₁₋₆ haloalkyl, C₃₋₈ halocycloalkyl, C₁₋₆ hydroxyalkyl, C₁₋₆ alkylcarbonyl, C₁₋₆ alkoxy, and C ₁₋₆ haloalkoxy are optionally substituted with one or more R^{d}; R^{d} is selected from the following substituents: halogen, hydroxyl, amino, nitro, cyano, carbonyl, oxo, carboxyl, C₁₋₆ alkyl, C₂₋₆ alkenyl, C₂₋₆ alkynyl, C₁₋₆ haloalkyl, C₁₋₆ hydroxyalkyl, C₁₋₆ alkylcarbonyl, C₁₋₆ alkoxy, and C₁₋₆ haloalkoxy; heteroatoms in the heterocycloalkyl are selected from one or more of N, O, and S, and the number of the heteroatoms is 1, 2, or 3; R^{j} and R^{k} are each independently H, cyano, amino, C₁₋₆ alkyl, C₁₋₆ alkylamino, - (CH₂)₀₋₃C₃₋₈ cycloalkyl, -COC₁₋₆ alkyl, C₁₋₆ haloalkyl, C₁₋₆ alkyl substituted with hydroxyl, or unsubstituted 5- to 10-membered heterocycloalkyl; R^{a}, R^{c}, R¹, R², and R³ are each independently H, halogen, cyano, C₁₋₆ alkyl, C₂₋₆ alkenyl, C₂₋₆ alkynyl, C₁₋₆ haloalkyl, C₃₋₈ cycloalkyl, or halogenated C₃₋₈ cycloalkyl; X¹, X², X³, X⁴, X⁵, X⁶, m, and n are as defined in the first aspect of the present disclosure. In a preferred embodiment of the present disclosure, R^{b} is H, F, Cl, -CN, methyl, methoxy, amino, oxo, -CH₂CF₃, -CF₃, -CHF₂, -CH₂CHF₂, -CH₂-SO₂-CH₃, - CH(CH₃)SO₂CH₃, -CH₂S(=O)(=NCH₃)-CH₃, 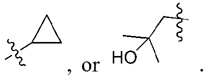 In a preferred embodiment of the present disclosure, R^{a}, R^{c}, R¹, R², and R³ are each independently H, F, Cl, methyl, ethyl, amino, oxo, -CHF₂, -CH₂CF₃, - CF₃,

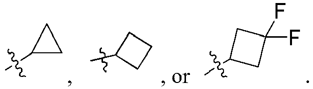

In a preferred embodiment of the present disclosure, the compound represented by formula IA is selected from the following structure: 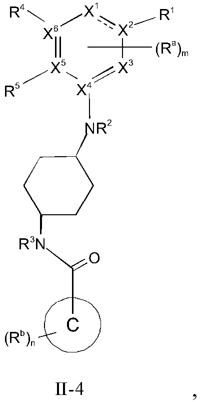 wherein A, together with X⁵ and X⁶ ring atoms, forms 6- to 8-membered aryl, 3- to 8-membered heterocycloalkyl, 5-membered heteroaryl, or 6-membered heteroaryl; the heteroatoms are independently selected from one or more of N, O, and S; A is further substituted with R^{c}; one or more R^{c} substitutions are present, and when a plurality of R^{c} substituents are present, the substituents are identical or different; and when A, together with X⁵ and X⁶ ring atoms, forms 6-membered aryl, R^{a} is oxo; or, when A, together with X⁵ and X⁶ ring atoms, forms 5-membered heteroaryl and R^{c} is not H, R¹ is C₁₋₆ haloalkyl; heteroatoms in the heterocycloalkyl and heteroaryl are selected from one or more of N, O, and S, and the number of the heteroatoms is 1, 2, or 3; ring C is 6- to 10-membered aryl or 5- to 8-membered heteroaryl; heteroatoms in the heteroaryl are selected from one or more of N, O, and S, and the number of the heteroatoms is 1, 2, or 3; R^{b} is H, halogen, hydroxyl, amino, cyano, carbonyl, oxo, C₁₋₆ alkyl, C₃₋₈ cycloalkyl, C₂₋₆ alkenyl, C₂₋₆ alkynyl, C₁₋₆ haloalkyl, C₃₋₈ halocycloalkyl, C₁₋₆ hydroxyalkyl, C₁₋₆ alkylcarbonyl, C₁₋₆ alkoxy, C₁₋₆ haloalkoxy or 3- to 8-membered heterocycloalkyl, -(CHR^{j})₀₋₃-S(=O)(=NR^{j})-R^{k}, -(CHR^{j})₁₋₃-S(O)₂-R^{k}, -(CHR^{j})₁₋₃-OC(O)-N(R^{k})₂, -(CHR^{j})₀₋₃-(N=)S(=O)(R^{k})₂, or -(CHR^{j})₀₋₃-P(=O)(R^{k})₂; the C₁₋₆ alkyl, C₃₋₈ cycloalkyl, C₂₋₆ alkenyl, C₂₋₆ alkynyl, C₁₋₆ haloalkyl, C₃₋₈ halocycloalkyl, C₁₋₆ hydroxyalkyl, C₁₋₆ alkylcarbonyl, C₁₋₆ alkoxy, and C ₁₋₆ haloalkoxy are optionally substituted with one or more R^{d}; R^{d} is selected from the following substituents: halogen, hydroxyl, amino, nitro, cyano, carbonyl, oxo, carboxyl, C₁₋₆ alkyl, C₂₋₆ alkenyl, C₂₋₆ alkynyl, C₁₋₆ haloalkyl, C₁₋₆ hydroxyalkyl, C₁₋₆ alkylcarbonyl, C₁₋₆ alkoxy, and C₁₋₆ haloalkoxy; heteroatoms in the heterocycloalkyl are selected from one or more of N, O, and S, and the number of the heteroatoms is 1, 2, or 3; R^{j} and R^{k} are each independently H, cyano, amino, C₁₋₆ alkyl, C₁₋₆ alkylamino, - (CH₂)₀₋₃C₃₋₈ cycloalkyl, -COC₁₋₆ alkyl, C₁₋₆ haloalkyl, C₁₋₆ alkyl substituted with hydroxyl, or unsubstituted 5- to 10-membered heterocycloalkyl; R^{a}, R^{c}, R¹, R², and R³ are each independently H, halogen, cyano, C₁₋₆ alkyl, C₂₋₆ alkenyl, C₂₋₆ alkynyl, C₁₋₆ haloalkyl, C₃₋₈ cycloalkyl, or halogenated C₃₋₈ cycloalkyl; X¹, X², X³, X⁴, X⁵, X⁶, m, and n are as defined in the first aspect of the present disclosure. In a preferred embodiment of the present disclosure, R^{b} is H, F, Cl, -CN, methyl, methoxy, amino, oxo, -CH₂CF₃, -CF₃, -CHF₂, -CH₂CHF₂, -CH₂-SO₂-CH₃, - CH(CH₃)SO₂CH₃, -CH₂S(=O)(=NCH₃)-CH₃, 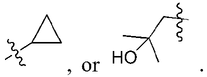 In a preferred embodiment of the present disclosure, R^{a}, R^{c}, R¹, R², and R³ are each independently H, F, Cl, methyl, ethyl, amino, oxo, -CHF₂, -CH₂CF₃, - CF₃,

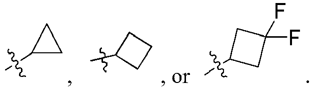

In a preferred embodiment of the present disclosure, the compound represented by formula IA is selected from the following structure: 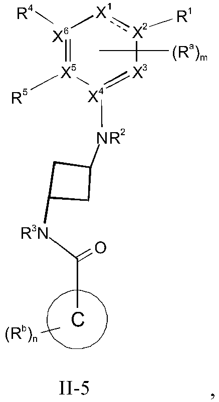 wherein A, together with X⁵ and X⁶ ring atoms, forms 6- to 8-membered aryl, 3- to 8-membered heterocycloalkyl, 5-membered heteroaryl, or 6-membered heteroaryl; the heteroatoms are independently selected from one or more of N, O, and S; A is further substituted with R^{c}; one or more R^{c} substitutions are present, and when a plurality of R^{c} substituents are present, the substituents are identical or different; and when A, together with X⁵ and X⁶ ring atoms, forms 6-membered aryl, R^{a} is oxo; or, when A, together with X⁵ and X⁶ ring atoms, forms 5-membered heteroaryl and R^{c} is not H, R¹ is C₁₋₆ haloalkyl; heteroatoms in the heterocycloalkyl and heteroaryl are selected from one or more of N, O, and S, and the number of the heteroatoms is 1, 2, or 3; ring C is 6- to 10-membered aryl or 5- to 8-membered heteroaryl; heteroatoms in the heteroaryl are selected from one or more of N, O, and S, and the number of the heteroatoms is 1, 2, or 3; R^{b} is H, halogen, hydroxyl, amino, cyano, carbonyl, oxo, C₁₋₆ alkyl, C₃₋₈ cycloalkyl, C₂₋₆ alkenyl, C₂₋₆ alkynyl, C₁₋₆ haloalkyl, C₃₋₈ halocycloalkyl, C₁₋₆ hydroxyalkyl, C₁₋₆ alkylcarbonyl, C₁₋₆ alkoxy, C₁₋₆ haloalkoxy or 3- to 8-membered heterocycloalkyl, -(CHR^{j})₀₋₃-S(=O)(=NR^{j})-R^{k}, -(CHR^{j})₁₋₃-S(O)₂-R^{k}, -(CHR^{j})₁₋₃-OC(O)-N(R^{k})₂, -(CHR^{j})₀₋₃-(N=)S(=O)(R^{k})₂, or -(CHR^{j})₀₋₃-P(=O)(R^{k})₂; the C₁₋₆ alkyl, C₃₋₈ cycloalkyl, C₂₋₆ alkenyl, C₂₋₆ alkynyl, C₁₋₆ haloalkyl, C₃₋₈ halocycloalkyl, C₁₋₆ hydroxyalkyl, C₁₋₆ alkylcarbonyl, C₁₋₆ alkoxy, and C ₁₋₆ haloalkoxy are optionally substituted with one or more R^{d}; R^{d} is selected from the following substituents: halogen, hydroxyl, amino, nitro, cyano, carbonyl, oxo, carboxyl, C₁₋₆ alkyl, C₂₋₆ alkenyl, C₂₋₆ alkynyl, C₁₋₆ haloalkyl, C₁₋₆ hydroxyalkyl, C₁₋₆ alkylcarbonyl, C₁₋₆ alkoxy, and C₁₋₆ haloalkoxy; heteroatoms in the heterocycloalkyl are selected from one or more of N, O, and S, and the number of the heteroatoms is 1, 2, or 3; R^{j} and R^{k} are each independently H, cyano, amino, C₁₋₆ alkyl, C₁₋₆ alkylamino, - (CH₂)₀₋₃C₃₋₈ cycloalkyl, -COC₁₋₆ alkyl, C₁₋₆ haloalkyl, C₁₋₆ alkyl substituted with hydroxyl, or unsubstituted 5- to 10-membered heterocycloalkyl; R^{a}, R^{c}, R¹, R², and R³ are each independently H, halogen, cyano, C₁₋₆ alkyl, C₂₋₆ alkenyl, C₂₋₆ alkynyl, C₁₋₆ haloalkyl, C₃₋₈ cycloalkyl, or halogenated C₃₋₈ cycloalkyl; X¹, X², X³, X⁴, X⁵, X⁶, m, and n are as defined in the first aspect of the present disclosure. In a preferred embodiment of the present disclosure, R^{b} is H, F, Cl, -CN, methyl, methoxy, amino, oxo, -CH₂CF₃, -CF₃, -CHF₂, -CH₂CHF₂, -CH₂-SO₂-CH₃, - CH(CH₃)SO₂CH₃, -CH₂S(=O)(=NCH₃)-CH₃, 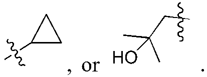 In a preferred embodiment of the present disclosure, R^{a}, R^{c}, R¹, R², and R³ are each independently H, F, Cl, methyl, ethyl, amino, oxo, -CHF₂, -CH₂CF₃, - CF₃,

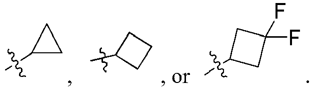

In some embodiments of the present disclosure, the compound represented by formula I is selected from the following structures: 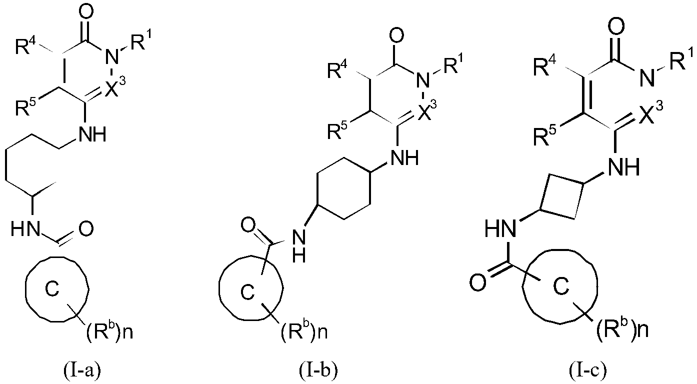 wherein ring C, X³, R¹, R⁴, R⁵, R^{b}, and n are as defined above.

In a preferred embodiment of the present disclosure, the compound represented by formula I is selected from the following structure: 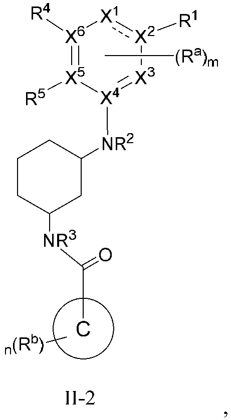 wherein ring C is 6- to 10-membered aryl or 5- to 8-membered heteroaryl; heteroatoms in the heteroaryl are selected from one or more of N, O, and S, and the number of the heteroatoms is 1, 2, or 3; R^{b} is H, halogen, hydroxyl, amino, cyano, carbonyl, oxo, C₁₋₆ alkyl, C₃₋₈ cycloalkyl, C₂₋₆ alkenyl, C₂₋₆ alkynyl, C₁₋₆ haloalkyl, C₃₋₈ halocycloalkyl, C₁₋₆ hydroxyalkyl, C₁₋₆ alkylcarbonyl, C₁₋₆ alkoxy, C₁₋₆ haloalkoxy, or 3- to 8-membered heterocycloalkyl; heteroatoms in the heterocycloalkyl are selected from one or more of N, O, and S, and the number of the heteroatoms is 1, 2, or 3; R^{a}, R¹, R², R³, R⁴, and R⁵ are each independently H, halogen, cyano, amino, oxo, C₁₋₆ alkyl, C₂₋₆ alkenyl, C₂₋₆ alkynyl, C₁₋₆ haloalkyl, C₃₋₈ cycloalkyl, or halogenated C₃₋₈ cycloalkyl; X¹, X², X³, X⁴, X⁵, X⁶, m, and n are as defined in the first aspect of the present disclosure. In a preferred embodiment of the present disclosure, R^{b} is H, F, Cl, methyl, amino, oxo, -CH₂CF₃, or -CF₃. In a preferred embodiment of the present disclosure, R^{a}, R¹, R², R³, R⁴, and R⁵ are each independently H, F, Cl, methyl, amino, oxo, -CH₂CF₃, -CF₃,

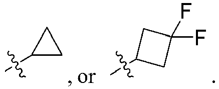

In a preferred embodiment of the present disclosure, ring C is 6- to 8-membered aryl or 5- to 10-membered heteroaryl. Heteroatoms in the heteroaryl are selected from one or more of N, O, and S, and the number of the heteroatoms is 1, 2, or 3.

In a preferred embodiment of the present disclosure, ring C is 5- to 6-membered nitrogen-containing heteroaryl, and the number of the nitrogen atoms is 1, 2, or 3.

In a preferred embodiment of the present disclosure, ring C is phenyl, pyrazolyl, or pyridinyl.

In a preferred embodiment of the present disclosure, the compound represented by formula IA is selected from any one of the following compounds:

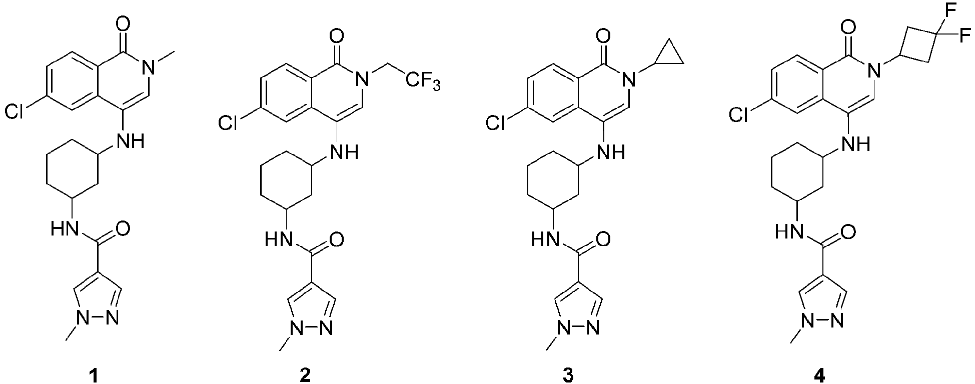

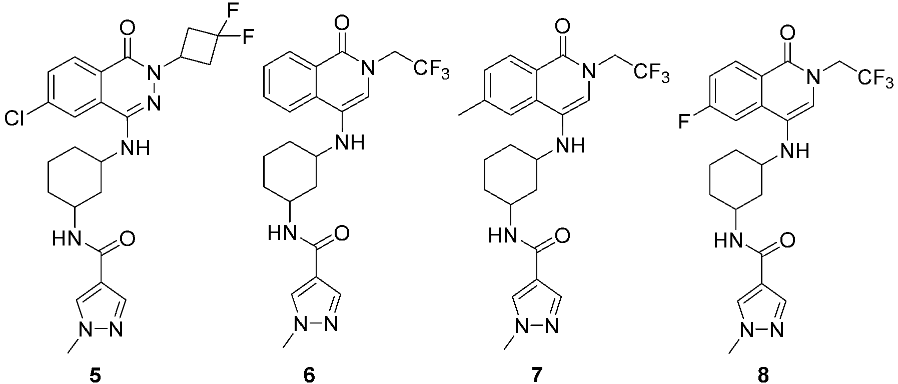

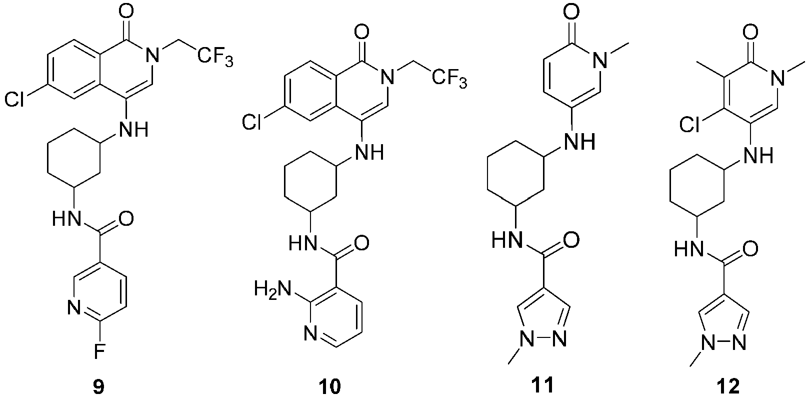

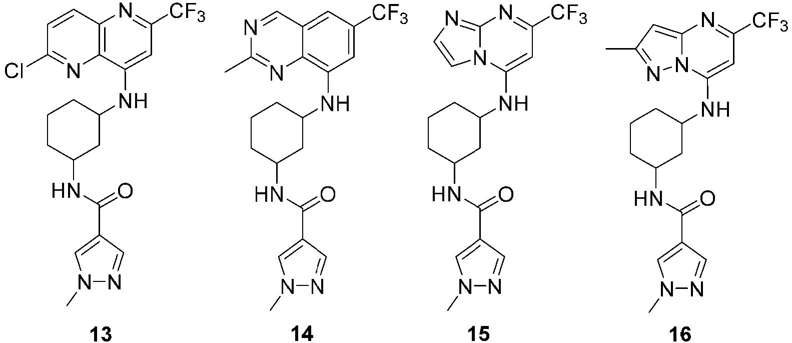

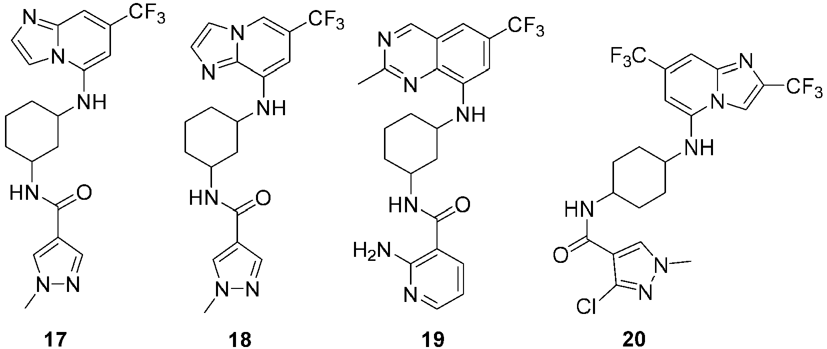

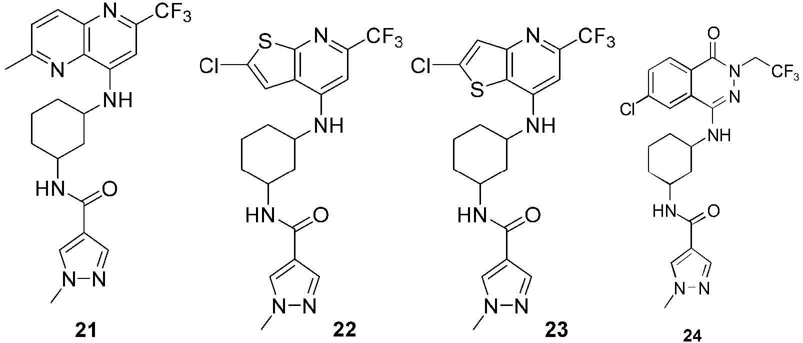

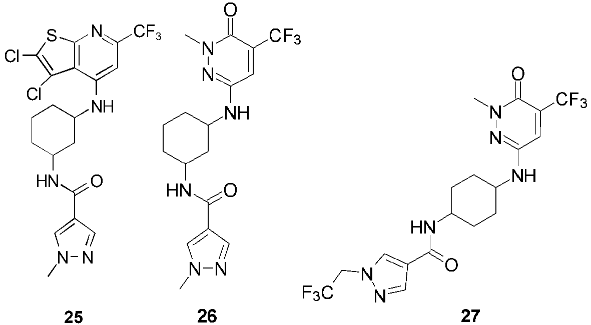

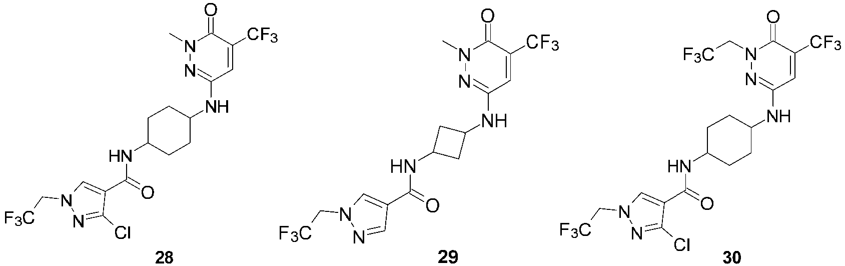

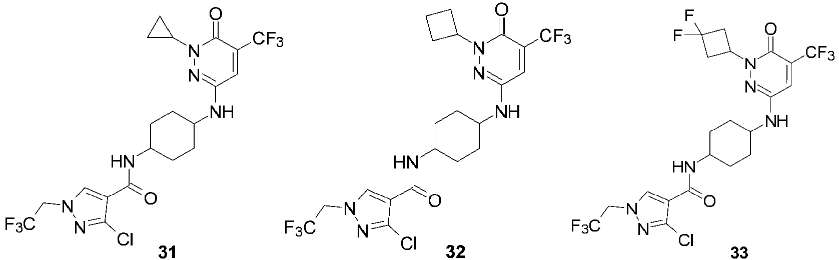

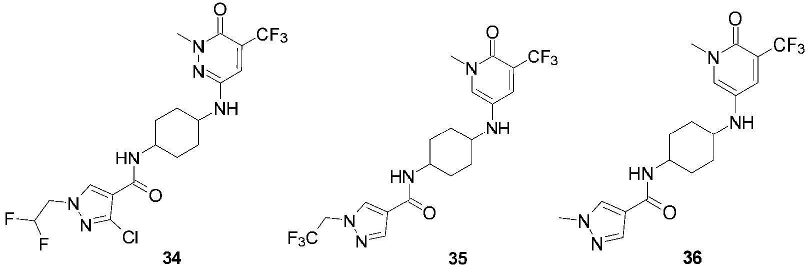

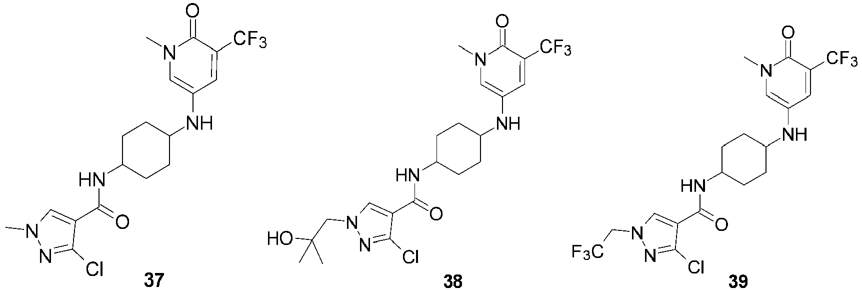

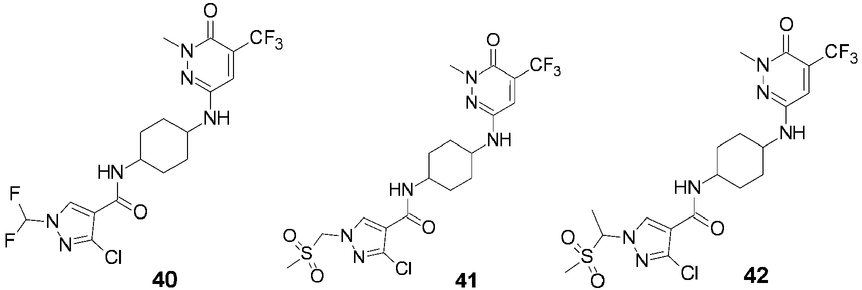

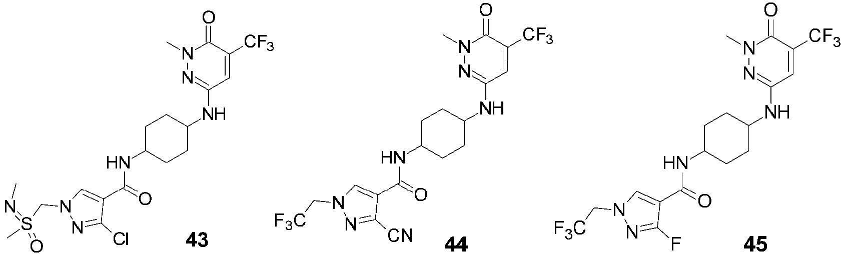

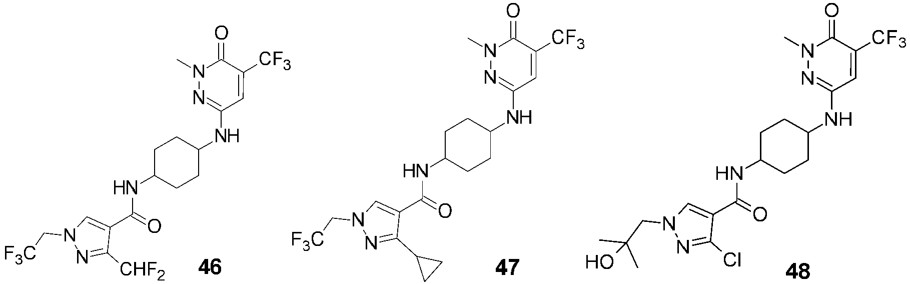

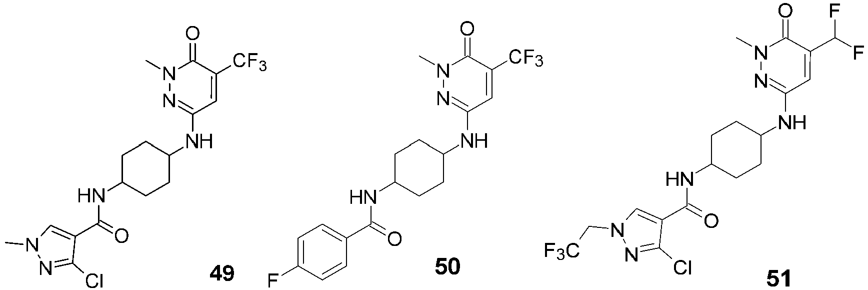

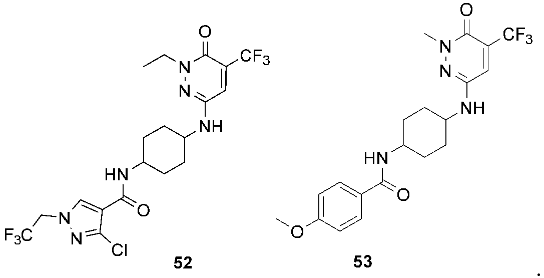

In a preferred embodiment of the present disclosure, the pharmaceutically acceptable salt of the compound represented by formula IA is a trifluoroacetate.

In a preferred embodiment of the present disclosure, the compound represented by formula IA is selected from any one of the following compounds:

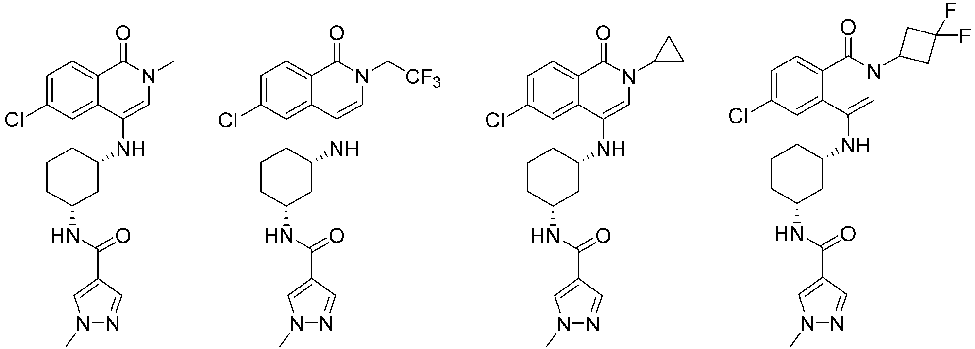

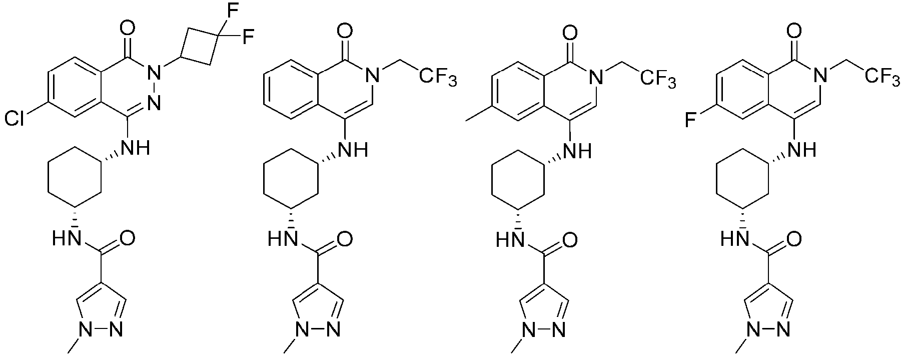

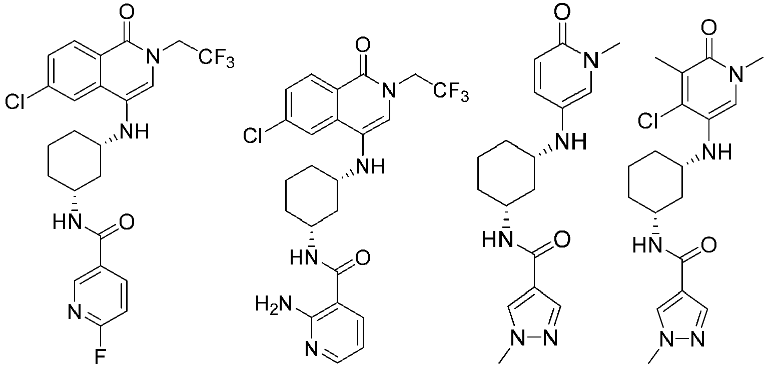

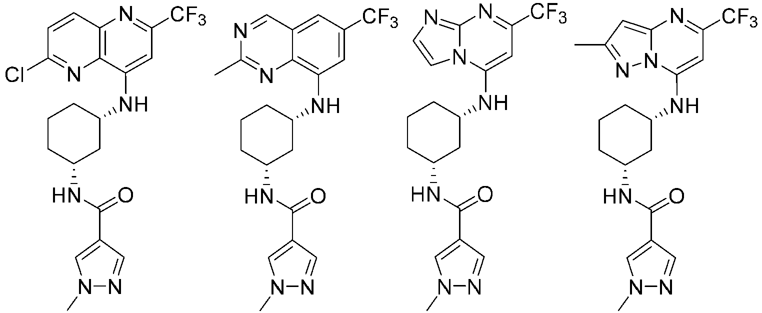

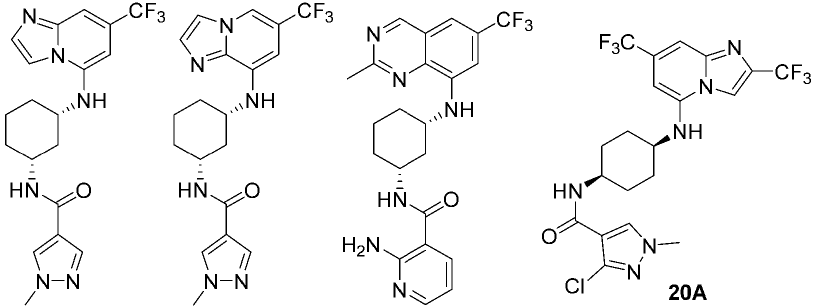

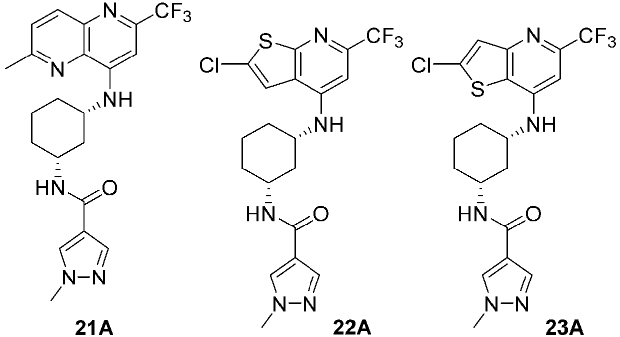

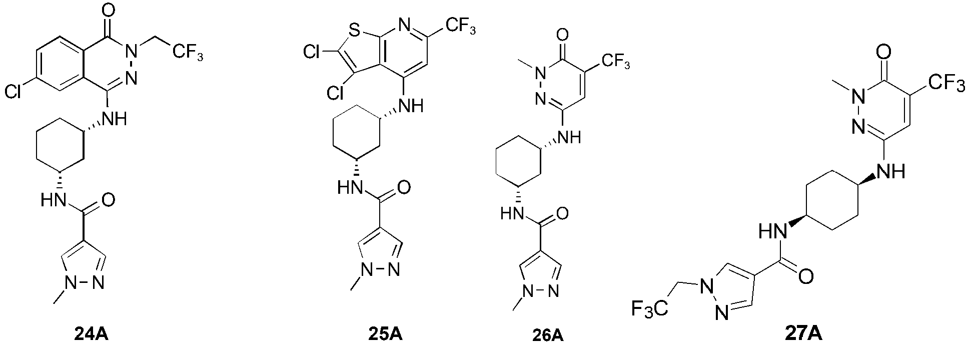

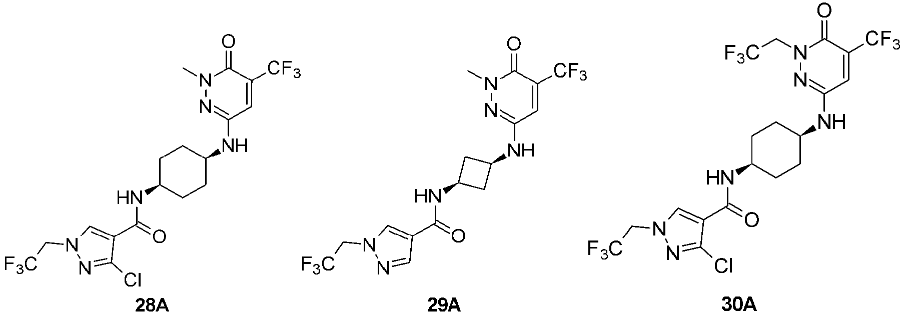

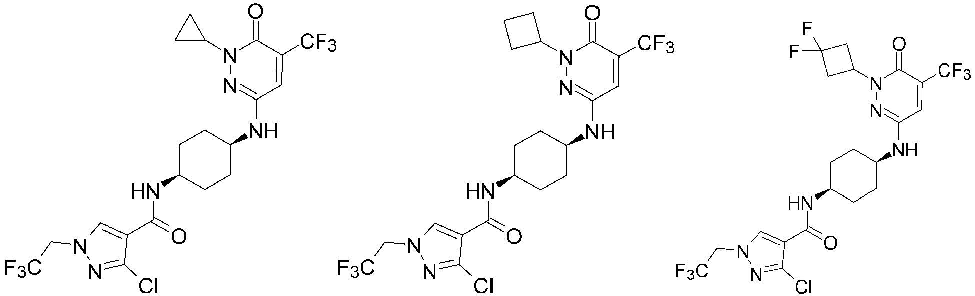

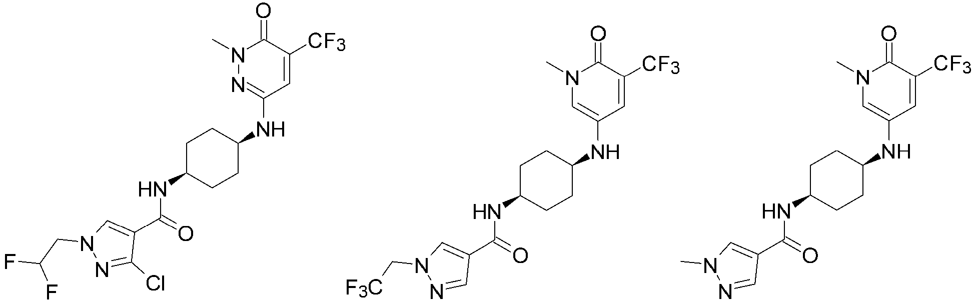

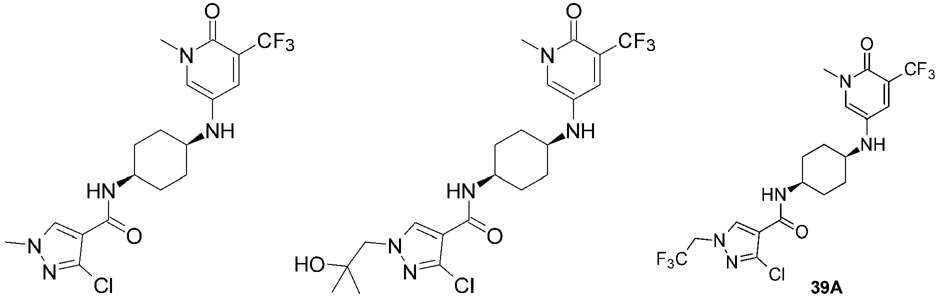

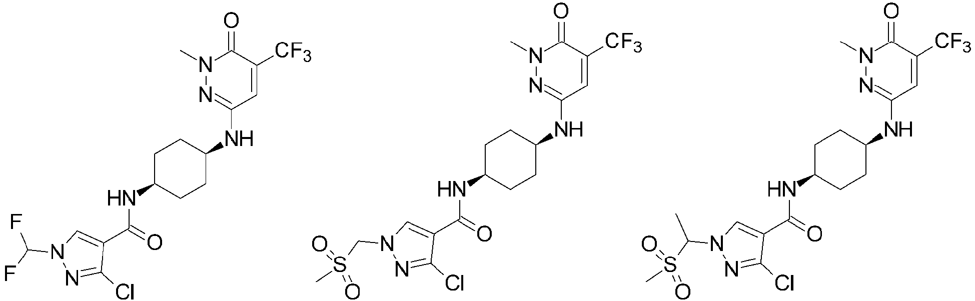

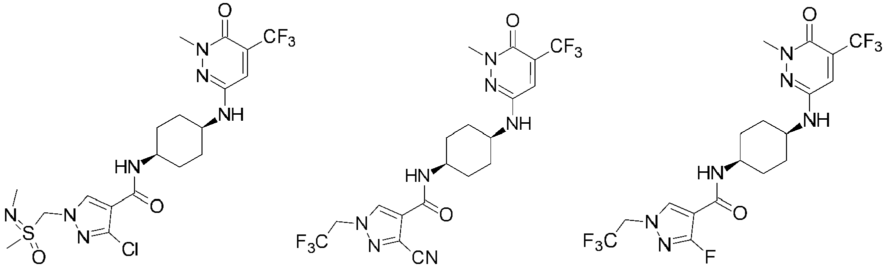

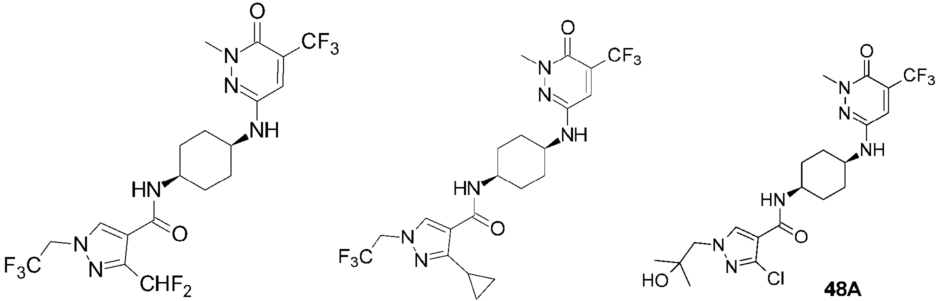

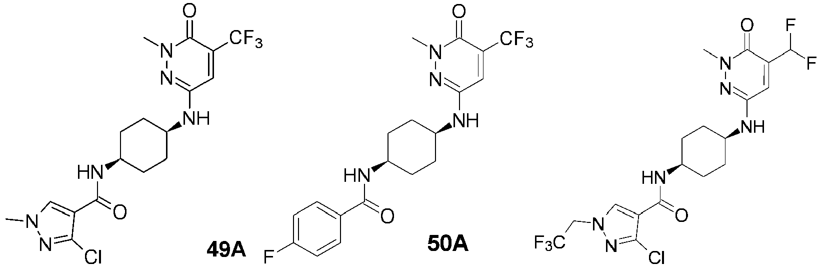

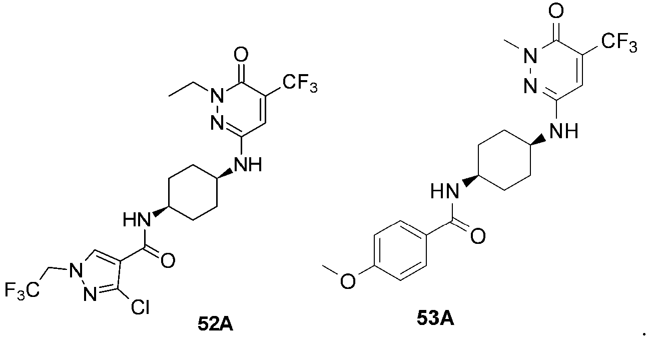

In a preferred embodiment of the present disclosure, the pharmaceutically acceptable salt of the compound represented by formula IA or formula I is a trifluoroacetate.

In a third aspect of the present disclosure, provided is a pharmaceutical composition comprising: the compound represented by formula IA or formula I, or the tautomer, the stereoisomer, the hydrate, the solvate, the pharmaceutically acceptable salt, or the prodrug thereof according to the first or second aspect of the present disclosure, and a pharmaceutically acceptable carrier.

In a fourth aspect of the present disclosure, provided is use of the compound represented by formula IA or formula I, or
the tautomer, the stereoisomer, the hydrate, the solvate, the pharmaceutically acceptable salt, or the prodrug thereof according to the first or second aspect of the present disclosure, or use of the pharmaceutical composition according to the third aspect of the present disclosure, and the use comprises: antagonizing MRGPRX2; and/or preventing and/or treating a disease associated with MRGPRX2; and/or, manufacturing a medicament, a pharmaceutical composition, or a formulation for use in antagonizing MRGPRX2 and/or for use in preventing and/or treating a disease associated with MRGPRX2.

Preferably, the disease associated with MRGPRX2 comprises: a mast cell-related disease.

Preferably, the disease associated with MRGPRX2 comprises: skin diseases, autoimmune diseases, and nervous system diseases.

Preferably, the skin disease is selected from atopic dermatitis, contact dermatitis, urticaria, and pruritus.

Preferably, the urticaria is selected from chronic spontaneous urticaria and induced urticaria.

Preferably, the autoimmune disease is selected from allergy, mastocytosis, rheumatoid arthritis, asthma, ulcerative colitis, and interstitial cystitis.

Preferably, the nervous system disease is selected from pain.

In a fifth aspect of the present disclosure, provided is a method for antagonizing MRGPRX2, or preventing and/or treating a disease associated with MRGPRX2, and the method comprises the step of: administering to a subject in need thereof the compound represented by formula IA or formula I, or the tautomer, the stereoisomer, the hydrate, the solvate, the pharmaceutically acceptable salt, or the prodrug thereof according to the first or second aspect of the present disclosure.

Additional aspects and advantages of the present disclosure will be set forth in part in the following description and, in part, will be apparent from the following description, or may be learned by practice of the present disclosure.

### Terms and Definitions

Unless otherwise stated, the terms and definitions described in the specification and claims of the present application and used in the present disclosure are included as follows. It will be understood by those skilled in the art that, according to conventions used in the art, in the structural formulas of the present application, 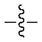 is used to depict a chemical bond that is the point at which a moiety or substituent is linked to a core structure or framework.

Unless otherwise specified, the term "pharmaceutically acceptable" refers to those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

Unless otherwise specified, the term "pharmaceutically acceptable salt" refers to a pharmaceutically acceptable salt of a non-toxic acid or base, including salts of inorganic acids and bases, and organic acids and bases.

Unless otherwise specified, the term "pharmaceutical composition" refers to a mixture of one or more of the compounds described herein or a physiologically/pharmaceutically acceptable salt or prodrug thereof, and other chemical components, for example, physiologically/pharmaceutically acceptable carriers and excipients. The purpose of the pharmaceutical composition is to facilitate the administration of the compound to an organism.

Unless otherwise specified, the term "prodrug" can be converted to the compound disclosed herein with biological activity under physiological conditions or by solvolysis. The prodrug disclosed herein is prepared by modifying a functional group in the compound, wherein the modification may be removed by conventional procedures or be removed *in vivo* to obtain the parent compound. The prodrug includes a compound formed by linking a hydroxyl or amino group of the compound disclosed herein to any group. When the prodrug of the compound disclosed herein is administered to a mammalian individual, the prodrug is cleaved to form a free hydroxyl group or a free amino group.

Unless otherwise specified, the term "stereoisomer" refers to isomers resulting from different spatial arrangements of atoms in a molecule, including cis-trans isomers, enantiomers, diastereoisomers, and conformational isomers.

Based on the choice of starting materials and methods, the compound disclosed herein may be present in the form of one of the possible isomers or a mixture thereof, for example, as a pure optical isomer, or as an isomer mixture, such as a racemic mixture and a diastereoisomer mixture, depending on the number of asymmetric carbon atoms. When describing optically active compounds, the prefixes D and L or R and S are used to denote the absolute configuration of the molecule with respect to the chiral center(s) in the molecule. The prefixes D and L or (+) and (-) are the symbols used to designate the rotation of plane polarized light by a compound, where (-) or L indicates that the compound is levorotatory. Compounds with the prefix (+) or D are dextrorotatory. For a given chemical structure, the stereoisomers are identical except that they are mirror images of one another. Specific stereoisomers may also be referred to as enantiomers, and mixtures of such isomers are generally referred to as mixtures of enantiomers. A 50:50 mixture of enantiomers is referred to as a racemic mixture or a racemate, and such a racemic mixture or racemate may be formed when no stereoselectivity or stereospecificity is present in a chemical reaction or process. Many geometric isomers, such as those of alkenes and C=N double bonds, may also exist in the compound described herein, and all such stable isomers are contemplated in the present disclosure. When the compound described herein contains a carbon-carbon double bond, unless otherwise stated, such double bond includes both E and Z geometric isomers. If a compound contains a disubstituted cycloalkyl group, the substituents on the cycloalkyl group may be in cis- or trans-configurations.

When the bond to a chiral carbon in the formula of the present disclosure is depicted as a direct line, it should be understood that both the (R) and (S) configurations of the chiral carbon, as well as the resulting enantiomerically pure compounds and a mixture thereof, are encompassed within the scope of the general formula. The method of depicting the racemic or enantiomerically pure compounds herein is from Maehr, J. Chem. Ed. 1985, 62:114-120. Unless otherwise stated, the absolute configuration of a stereogenic center is represented by a wedged bond and a dashed bond.

Optically active (R)- or (S)-isomers may be prepared using chiral synthons or chiral reagents, or may be obtained by resolution using conventional techniques. The compound of the present disclosure containing asymmetrically substituted carbon atoms can be separated in optically active forms or in racemic forms. Resolution of racemic mixtures of compounds can be performed by any one of a variety of methods known in the art. Exemplary methods include fractional recrystallization using a chiral resolving acid, which is an optically active salt-forming organic acid. Suitable resolving agents for use in the fractional recrystallization method are, for example, optically active acids such as tartaric acid, diacetyltartaric acid, dibenzoyltartaric acid, mandelic acid, malic acid, lactic acid, or various optically active camphorsulfonic acids, such as the D- and L-forms of β-camphorsulfonic acid. Other resolving agents that are suitable for use in the fractional crystallization method include stereomerically pure forms of α-methyl-benzylamine (e.g., S- and R-forms or diastereomerically pure forms), 2-phenylglycinol, norephedrine, ephedrine, N-methylephedrine, cyclohexylethylamine, 1,2-diaminocyclohexane, and the like. Resolution of racemic mixtures may also be performed by elution on a chromatographic column packed with an optically active resolving agent (e.g., dinitrobenzoylphenylglycine). High performance liquid chromatography (HPLC) or supercritical fluid chromatography (SFC) may be employed to perform the resolution. The selection of specific methods, as well as elution conditions and selection of chromatographic columns, may be determined by those skilled in the art based on the structure of the compound and experimental results. Furthermore, any enantiomer or diastereomer of the compound described herein can be obtained by stereochemical organic synthesis using optically pure starting raw materials or reagents of known configuration.

Unless otherwise specified, the term "tautomer" refers to functional isomers resulting from the rapid movement of an atom in a molecule between two positions. The compound disclosed herein may exhibit tautomerism. A tautomeric compound may be present in two or more interconvertible forms. Prototropic tautomers result from the migration of a covalently bonded hydrogen atom between two atoms. Tautomers are generally present in an equilibrium form. Efforts to separate a single tautomer usually lead to a mixture, the physicochemical properties of which are consistent with those of the mixture of the compound. The position of the equilibrium depends on the chemical properties of the molecule. For example, in many aliphatic aldehydes and ketones such as acetaldehyde, the keto form predominates; whereas in phenols, the enol form predominates. In the present disclosure, all tautomeric forms of the compound are included.

Unless otherwise stated, the absolute configuration of a stereogenic center is represented by a wedged solid bond ( 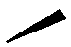 ) and a wedged dashed bond ( 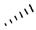 ), and the relative configuration of a stereogenic center is represented by a straight solid bond ( 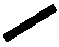 ) and a straight dashed bond ( 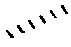 ).

Unless otherwise stated, the term "solvate" means that the compound disclosed herein or a salt thereof comprises a stoichiometric or non-stoichiometric amount of a solvent bonded by non-covalent intermolecular forces, and when the solvent is water, the solvate is a hydrate.

In the context of a drug or pharmacologically active agent, the term "effective amount" or "therapeutically effective amount" refers to an amount of the drug or medicament that is sufficient to achieve the desired effects without producing toxic effects. For oral dosage forms of the present disclosure, an "effective amount" of one active substance in the composition is the amount required to achieve the desired effect when the active substance is combined with another active substance in the composition. The effective amount varies from person to person, depending on the age and general condition of the subject and also on the particular active substance. The suitable effective amount in a case can be determined by those skilled in the art through routine tests.

Unless otherwise specified, the term "active ingredient", "therapeutic agent", "active substance", or "active agent" refers to a chemical entity that is effective in treating a target disorder, disease, or condition.

Unless otherwise specified, the term "substituted" means that one or more hydrogen atoms on a specific atom are substituted by substituents, including deuterium and hydrogen variants, as long as the valence of the specific atom is normal and the substituted compound is stable. When the substituent is keto (i.e., =O), it means that two hydrogen atoms are substituted. Substitution with keto does not occur on aromatic groups.

In the present application, "optional" or "optionally" means that the subsequently described event or circumstance may occur or may not occur, and the description includes instances where the event or circumstance occurs or does not occur. For example, "optionally substituted aryl" means that the aryl is substituted or unsubstituted, and the description includes both substituted aryl and unsubstituted aryl.

Unless otherwise specified, the term "C₁₋₆ alkyl" refers to a linear or branched saturated hydrocarbon group consisting of 1 to 6 carbon atoms. The C₁₋₆ alkyl includes C₁₋₅ alkyl, C₁₋₄ alkyl, C₁₋₃ alkyl, C₁₋₂ alkyl, C₂₋₆ alkyl, C₂₋₄ alkyl, C₆ alkyl, and C₅ alkyl, and the like, and may be monovalent (e.g., methyl), divalent (e.g., methylene), or polyvalent (e.g., methenyl). Examples of C₁₋₆ alkyl include, but are not limited to, methyl (Me), ethyl (Et), propyl (including n-propyl and isopropyl), butyl (including n-butyl, isobutyl, s-butyl, and t-butyl), pentyl (including n-pentyl, isopentyl, and neopentyl), hexyl, and the like.

Unless otherwise specified, the term "C₁₋₃ alkyl" refers to a linear or branched saturated hydrocarbon group consisting of 1 to 3 carbon atoms. The C₁₋₃ alkyl includes C₁₋₂ alkyl and C₂₋₃ alkyl and the like, and may be monovalent (e.g., methyl), divalent (e.g., methylene), or polyvalent (e.g., methenyl). Examples of C₁₋₃ alkyl include, but are not limited to, methyl (Me), ethyl (Et), propyl (including n-propyl and isopropyl), and the like.

The term "halo", alone or as part of another substituent, is used interchangeably with the term "halogen-substituted". Unless otherwise specified, "haloalkyl" or "halogen-substituted alkyl" is meant to include branched and linear saturated aliphatic hydrocarbyl having a specified number of carbon atoms and being substituted with one or more halogens.

Unless otherwise specified, "C₂₋₆ alkenyl" refers to a linear or branched hydrocarbon group consisting of 2 to 6 carbon atoms and comprising at least one carbon-carbon double bond, where the carbon-carbon double bond may be located at any position within the group. The C₂₋₆ alkenyl includes C₂₋₄ alkenyl, C₂₋₃ alkenyl, C₄ alkenyl, C₃ alkenyl, and C₂ alkenyl, and the like, and may be monovalent, divalent, or polyvalent. Examples of C₂₋₆ alkenyl include, but are not limited to, ethenyl, propenyl, butenyl, pentenyl, hexenyl, butadienyl, pentadienyl, hexadienyl, and the like.

Unless otherwise specified, "C₂₋₆ alkynyl" refers to a linear or branched hydrocarbon group consisting of 2 to 6 carbon atoms and comprising at least one carbon-carbon triple bond, where the carbon-carbon triple bond may be located at any position within the group. The C₂₋₆ alkynyl includes C₂₋₄ alkynyl, C₂₋₃ alkynyl, C₄ alkynyl, C₃ alkynyl, and C₂ alkynyl, and the like, and may be monovalent, divalent, or polyvalent. Examples of C₂₋₆ alkynyl include, but are not limited to, ethynyl, propynyl, butynyl, pentynyl, and the like.

Unless otherwise specified, the term "C₁₋₆ alkoxy" refers to those alkyl groups that each contain 1 to 6 carbon atoms and are each linked to the rest part of the molecule through an oxygen atom. The C₁₋₆ alkoxy includes C₁₋₄ alkoxy, C₁₋₃ alkoxy, C₁₋₂ alkoxy, C₂₋₆ alkoxy, C₂₋₄ alkoxy, C₆ alkoxy, C₅ alkoxy, C₄ alkoxy, and C₃ alkoxy, and the like. Examples of C₁₋₆ alkoxy include, but are not limited to, methoxy, ethoxy, propoxy (including n-propoxy and isopropoxy), butoxy (including n-butoxy, isobutoxy, s-butoxy, and t-butoxy), pentyloxy (including n-pentyloxy, isopentyloxy, and neopentyloxy), and hexyloxy.

Unless otherwise specified, the term "C₁₋₃ alkoxy" refers to those alkyl groups that each contain 1 to 3 carbon atoms and are each linked to the rest part of the molecule through an oxygen atom. The C₁₋₃ alkoxy includes C₁₋₂ alkoxy, C₂₋₃ alkoxy, C₃ alkoxy, and C₂ alkoxy, and the like. Examples of C₁₋₃ alkoxy include, but are not limited to, methoxy, ethoxy, propoxy (including n-propoxy and isopropoxy), and the like.

Unless otherwise specified, the term "C₁₋₆ alkylamino" refers to those alkyl groups that each contain 1 to 6 carbon atoms and are each linked to the rest part of the molecule through an amino group. The C₁₋₆ alkylamino includes C₁₋₄ alkylamino, C₁₋₃ alkylamino, C₁₋₂ alkylamino, C₂₋₆ alkylamino, C₂₋₄ alkylamino, C₆ alkylamino, C₅ alkylamino, C₄ alkylamino, C₃ alkylamino, and C₂ alkylamino, and the like. Examples of C₁₋₆ alkylamino include, but are not limited to, -NHCH₃, -N(CH₃)₂, -NHCH₂CH₃, -N(CH₃)CH₂CH₃, -N(CH₂CH₃)(CH₂CH₃), -NHCH₂CH₂CH₃, - NHCH₂(CH₃)₂, -NHCH₂CH₂CH₂CH3, and the like.

Unless otherwise specified, the term "C₃₋₈ cycloalkyl" refers to a saturated cyclic hydrocarbon group consisting of 3 to 8 carbon atoms. This includes monocyclic and bicyclic systems, wherein the bicyclic system includes spirocyclic, fused, and bridged rings. The C₃₋₈ cycloalkyl includes C₃₋₆ cycloalkyl, C₃₋₅ cycloalkyl, C₄₋₈ cycloalkyl, C₄₋₆ cycloalkyl, C₄₋₅ cycloalkyl, C₅₋₈ cycloalkyl, or C₅₋₆ cycloalkyl, or the like, and may be monovalent, divalent, or polyvalent. Examples of C₃₋₈ cycloalkyl include, but are not limited to, cyclopropyl, cyclobutyl, cyclopentyl, cyclohexyl, cycloheptyl, norbornyl, [2.2.2]bicyclooctane, and the like.

Unless otherwise specified, the term "C₃₋₆ cycloalkyl" refers to a saturated cyclic hydrocarbon group consisting of 3 to 6 carbon atoms, including monocyclic and bicyclic ring systems. The C₃₋₆ cycloalkyl includes C₃₋₅ cycloalkyl, C₄₋₅ cycloalkyl, C₅₋₆ cycloalkyl, and the like, and may be monovalent, divalent, or polyvalent. Examples of C₃₋₆ cycloalkyl include, but are not limited to, cyclopropyl, cyclobutyl, cyclopentyl, cyclohexyl, and the like.

Unless otherwise specified, Cₙ₋ₙ₊ₘ or Cₙ-Cₙ₊ₘ includes any one of the specific cases of n to n+m carbon atoms. For example, C₁₋₁₂ includes C₁, C₂, C₃, C₄, C₅, C₆, C₇, C₈, C₉, C₁₀, C₁₁, and C₁₂. Also, any range within n to n+m may be included. For example, C₁₋₁₂ includes C₁₋₃, C₁₋₆, C₁₋₉, C₃₋₆, C₃₋₉, C₃₋₁₂, C₆₋₉, C₆₋₁₂, and C₉₋₁₂, etc. Similarly, n- to n+m-membered represents that the number of atoms on the ring is n to n+m. For example, 3- to 12-membered ring includes 3-membered ring, 4-membered ring, 5-membered ring, 6-membered ring, 7-membered ring, 8-membered ring, 9-membered ring, 10-membered ring, 11-membered ring, and 12-membered ring. n- to n+m-membered also represents any range within n to n+m. For example, 3- to 12-membered ring includes 3- to 6-membered ring, 3- to 9-membered ring, 5- to 6-membered ring, 5- to 7-membered ring, 6- to 7-membered ring, 6- to 8-membered ring, 6- to 10-membered ring, etc.

Unless otherwise specified, the term "aryl" refers to a monocyclic or polycyclic carbocyclic ring having 6 to 20 carbon atoms, wherein at least one ring is an aromatic ring. When one of the rings is a non-aromatic ring, the group may be linked via the aromatic ring or via the non-aromatic ring. "Aryl" may be 6- to 8-membered or 6- to 10-membered aryl. Examples of aryl include, but are not limited to, phenyl, naphthyl, tetrahydronaphthyl, 2,3-dihydroindenyl, biphenyl, phenanthryl, anthryl, and acenaphthyl. The term "6- to 10-membered aryl" refers to a monocyclic or polycyclic carbocyclic ring having 6 to 10 carbon atoms, wherein at least one ring is an aromatic ring.

Unless otherwise specified, the term "heterocycloalkyl" refers to cycloalkyl in which one or more (e.g., 1, 2, 3, or 4, and in some embodiments 1 to 3) carbon atoms are substituted with heteroatoms, wherein the heteroatom is, for example, but not limited to, N, O, S, or P. The term "m- to n-membered heterocycloalkyl" should be understood to refer to a saturated, unsaturated, or partially saturated ring having m to n atoms, wherein the heterocyclic atoms are selected from N, O, S, and P, preferably from N, O, or S. The term "heterocycloalkyl" may refer to 3- to 8-membered, 3- to 11-membered, or 6- to 8-membered heterocycloalkyl. The term "3- to 11-membered heterocycloalkyl", alone or in combination with other terms, refers to a saturated cyclic group consisting of 3 to 11 ring atoms. The term "6-to 8-membered heterocycloalkyl", alone or in combination with other terms, refers to a saturated cyclic group consisting of 6 to 8 ring atoms, of which 1, 2, 3, or 4 ring atoms are heteroatoms independently selected from O, S, and N, with the remaining being carbon atoms. The nitrogen atom is optionally quaternized, and the nitrogen and sulfur heteroatoms can be optionally oxidized (i.e., NO and S(O)p, where p is 1 or 2). This includes monocyclic and bicyclic systems, wherein the bicyclic system includes spirocyclic, fused, and bridged rings. Furthermore, with respect to the "6- to 8-membered heterocycloalkyl", a heteroatom may occupy the position where the heterocycloalkyl is connected to the rest of the molecule. For example, 6- to 8-membered heterocycloalkyl includes, but is not limited to, 6-membered, 7-membered, and 8-membered heterocycloalkyl. Examples of 6- to 8-membered heterocycloalkyl include, but are not limited to, azetidinyl, oxetanyl, thietanyl, pyrrolidinyl, pyrazolidinyl, imidazolidinyl, tetrahydrothienyl (including tetrahydrothien-2-yl, tetrahydrothien-3-yl, and the like), tetrahydrofuranyl (including tetrahydrofuran-2-yl, and the like), tetrahydropyranyl, piperidyl (including 1-piperidyl, 2-piperidyl, 3-piperidyl, and the like), piperazinyl (including 1-piperazinyl, 2-piperazinyl, and the like), morpholinyl (including 3-morpholinyl, 4-morpholinyl, and the like), dioxanyl, dithianyl, isoxazolidinyl, isothiazolidinyl, 1,2-oxazinyl, 1,2-thiazinyl, and hexahydropyridazinyl.

Unless otherwise specified, the term "heteroaromatic ring" or "heteroaryl" refers to a monocyclic or polycyclic carbon ring, wherein at least one ring atom (e.g., 1, 2, or 3) is a heteroatom independently selected from oxygen, sulfur, and nitrogen, the remaining ring atoms are C, and at least one ring is an aromatic ring. The group may be a carbon group or a heteroatom group (that is, it may be C-linked or N-linked as long as such linkage is possible). When one of the rings is a non-aromatic ring, the group may be linked via the aromatic ring or via the non-aromatic ring. "Heteroaryl" may be 5- to 10-membered (e.g., 5-, 6-, 7-, 8-, 9-, or 10-membered) heteroaryl, or 5- to 8-membered heteroaryl. Examples of heteroaryl include, but are not limited to: imidazolyl, acridyl, carbazolyl, cinnolinyl, quinoxalinyl, pyrazolyl, indolyl, benzotriazolyl, furanyl, thienyl, benzothienyl, benzofuranyl, quinolyl, isoquinolyl, oxazolyl, isoxazolyl, indolyl, pyrazinyl, pyridazinyl, pyridinyl, pyrimidinyl, pyrrolyl, N-methylpyrrolyl, and tetrahydroquinolyl. The term "heteroaromatic ring" is used interchangeably with the term "heterocyclic aromatic ring", "heteroaryl", or "heteroaromatic ring group".

Unless otherwise specified, the term "oxo" means that two hydrogens on methylene are substituted with oxygen, i.e., methylene is substituted with carbonyl, represented as =O.

Unless otherwise specified, the term "halo" or "halogen" refers to fluorine, chlorine, bromine, and iodine.

In addition, it should be noted that, unless otherwise explicitly indicated, the description of "... is independently" used herein is to be understood broadly and means that each individual group described is independent from the others and may be independently the same or different specific groups. In more detail, the description of "... is independently" can mean that the specific options expressed by the same symbols in different groups do not affect each other; it can also mean that the specific options expressed by the same symbols in the same group do not affect each other.

In the present application, "pharmaceutically acceptable carrier" includes, but is not limited to, any adjuvant, carrier, excipient, glidant, sweetener, diluent, preservative, dye/colorant, flavoring agent, surfactant, wetting agent, dispersant, suspending agent, stabilizer, isotonic agent, solvent, or emulsifier that is approved by the relevant governmental regulatory agency for human or livestock use.

The term "treatment" refers to therapeutic therapy. Where specific conditions are involved, the treatment refers to: (1) relieving one or more of biological manifestations of a disease or condition, (2) interfering with (a) one or more of points in a biological cascade that causes or contributes to a condition or (b) one or more of biological manifestations of a condition, (3) ameliorating one or more of symptoms, effects, or side effects associated with a condition, or one or more of symptoms, effects, or side effects associated with a condition or treatment thereof, or (4) slowing the progression of a condition or one or more of biological manifestations of a condition.

The term "prevention" refers to a reduced risk of acquiring or developing a disease or disorder.

The term "patient" refers to any animal, preferably a mammal, that is to receive or has received administration of the compound or composition according to the embodiments of the present disclosure. The term "mammal" includes any mammal. Examples of mammals include, but are not limited to, cows, horses, sheep, pigs, cats, dogs, mice, rats, rabbits, guinea pigs, monkeys, humans, and the like, preferably humans.

The term "therapeutically effective amount" refers to an amount of a compound that, when administered to a patient, is sufficient to effectively treat diseases or conditions described herein. The "therapeutically effective amount" varies depending on the compound, the condition and its severity, and the age of the patient to be treated, and can be adjusted as desired by those skilled in the art.

The preferred conditions described above may be combined arbitrarily to obtain preferred embodiments of the present disclosure without departing from the general knowledge in the art.

### Beneficial Effects

After extensive and intensive studies, the inventors surprisingly developed a compound or a pharmaceutically acceptable salt thereof, a preparation method therefor, and use thereof. The present disclosure provides a compound represented by formula IA, or a tautomer, a stereoisomer, a hydrate, a solvate, a pharmaceutically acceptable salt, or a prodrug thereof. The compound of formula IA exhibits significant inhibitory activity against MRGPRX2 and can serve as an antagonist of MRGPRX2. It demonstrates favorable pharmacokinetic properties, good druggability, and shows no significant inhibitory effect on the bile salt export pump (BSEP), without the risk of cholestatic toxicity. Therefore, it can be seen that the compound of the present disclosure possesses relatively high safety and druggability from multiple aspects.

### DETAILED DESCRIPTION

The present disclosure will be further illustrated with reference to the following specific examples. It should be understood that the following descriptions are merely the most preferred embodiments of the present disclosure, and should not be construed as limiting the protection scope of the present disclosure. On the basis of a thorough understanding of the present disclosure, experimental procedures without specified conditions in the following examples are generally conducted according to conventional conditions or according to conditions recommended by the manufacturer. Those skilled in the art can make non-essential changes in the technical solutions of the present disclosure, and such changes should be regarded as included in the protection scope of the present disclosure.
EDCI: 1-(3-dimethylaminopropyl)-3-ethylcarbodiimide hydrochloride
HOBt: 1-hydroxybenzotriazole
DIEA: N,N-diisopropylethylamine
Ruphos Pd G4: methanesulfonato(2-dicyclohexylphosphino-2',4',6'-triisopropyl-1,1'-biphenyl)(2'-amino-1,1'-biphenyl-2-yl)palladium(II)

### Intermediate A1: Preparation of N-((1R,3S)-3-aminocyclohexyl)-1-methyl-1H-pyrazole-4-carboxamide

The synthetic route of target intermediate **A1** was as follows:

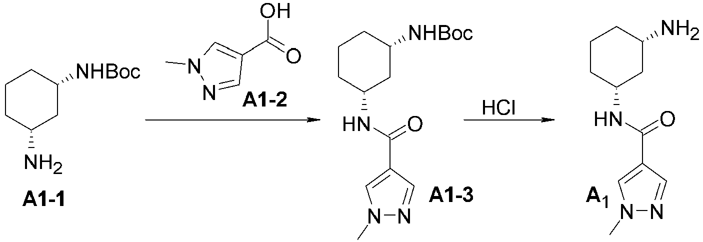

### Step 1: Synthesis of tert-butyl ((1S,3R))-3-(1-methyl-1H-pyrazole-4-carboxamido)cyclohexyl)carbamate

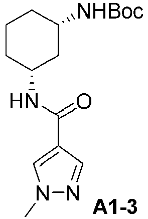

At room temperature, 1-methyl-1*H*-pyrazole-4-carboxylic acid (500 mg, 4 mmol) was dissolved in *N,N-*dimethylformamide (10 mL), and then tert-butyl ((1S,3R)-3-aminocyclohexane (428 mg, 4 mmol), *N,N-*diisopropylethylamine (408 mg, 12 mmol), and O-(7-azabenzotriazol-1-yl)-*N,N,N',N'*-tetramethyluronium hexafluorophosphate (2.38 g, 6 mmol) were sequentially added. Subsequently, the mixture was allowed to react at room temperature for 12 h. After the reaction was completed, the reaction liquid was poured into ice water, and extracted with ethyl acetate (3 × 50 mL). The obtained organic phase was dried over anhydrous sodium sulfate, filtered, and concentrated by rotary evaporation. The obtained crude product was purified by silica gel column chromatography (petroleum ether:ethyl acetate (V/V) = 10:1) to obtain a compound tert-butyl ((1*S,*3*R*))-3-(1-methyl-1*H*-pyrazole-4-carboxamido)cyclohexyl)carbamate **(A1-3)** (830 mg, 2.58 mmol, yield: 64.5%).
LC-MS, M/Z (ESI): 323.20 [M+H]⁺

### Step 2: Synthesis of N-((1R,3S)-3-aminocyclohexyl)-1-methyl-1H-pyrazole-4-carboxamide

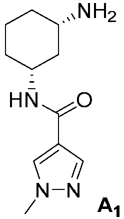

At room temperature, tert-butyl ((1*S*,3*R*))-3-(1-methyl-1*H*-pyrazole-4-carboxamido)cyclohexyl)carbamate (830 mg, 2.58 mmol) was dissolved in anhydrous 1,4-dioxane (10 mL), and the mixture was stirred at room temperature. A solution of hydrogen chloride in 1,4-dioxane (4 mol/L, 1.76 mL, 4 mmol) was then slowly added dropwise, and the reaction liquid was stirred at room temperature for 30 min. After the reaction of the starting material was completed as monitored by thin-layer chromatography, stirring was stopped. A saturated sodium bicarbonate solution was added into the reaction system to adjust the pH to 7-8, and the mixture was extracted with ethyl acetate (30 mL × 3). The organic phase was collected, dried over anhydrous sodium sulfate, and concentrated under reduced pressure. The residue was purified by silica gel column chromatography (dichloromethane:methanol (V/V) = 10: 1) to obtain a compound N-((1R,3S)-3-aminocyclohexyl)-1-methyl-1*H*-pyrazole-4-carboxamide **(intermediate A1**) (213 mg, yield: 36.8%).
LC-MS, M/Z (ESI): 223.13 [M+H]⁺

### Intermediate A2: Preparation of compound 3-chloro-1-(1-(methylsulfonyl)ethyl)-1H-pyrazole-4-carboxylic acid

The synthetic route of target intermediate **A2** was as follows:

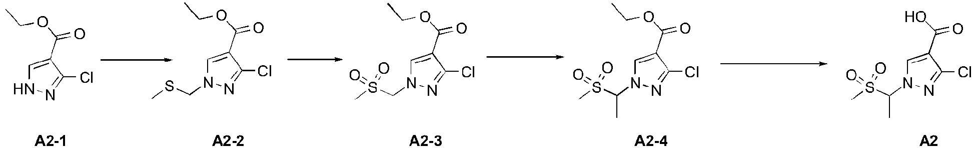

### Step 1: Synthesis of compound ethyl 3-chloro-1-((methylthio)methyl)-1H-pyrazole-4-carboxylate

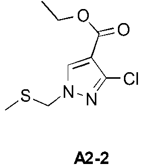

At room temperature, a compound ethyl 3-chloro-1*H*-pyrazole-4-carboxylate (2.00 g, 11.46 mmol) was dissolved in anhydrous tetrahydrofuran (20 mL), and the mixture was stirred in an ice bath at 0 °C for 10 min. Subsequently, sodium hydride (504 mg, 12.60 mmol) was added in batches, and chloromethyl methyl sulfide (1.33 g, 13.75 mmol) was then slowly added. The reaction liquid was slowly warmed to room temperature and successively stirred for 30 min. After the reaction was completed, stirring was stopped, and the reaction liquid was poured into water (30 mL) and then extracted with ethyl acetate (30 mL × 3). The organic phase was collected, dried over anhydrous sodium sulfate, filtered, and concentrated, and the residue was separated and purified by silica gel column chromatography (petroleum ether:ethyl acetate (V/V) = 10:1) to obtain compound ethyl 3-chloro-1-((methylthio)methyl)-1*H-*pyrazole-4-carboxylate (930 mg, yield: 34.59%).
LC-MS, M/Z (ESI): 235.3[M+H]⁺
¹H NMR (400 MHz, DMSO-d6) δ 8.50 (s, 1H), 5.26 (s, 2H), 4.23 (q, 2H), 2.14 (s, 3H), 1.27 (t, 3H).

### Step 2: Synthesis of compound ethyl 3-chloro-1-((methylsulfonylymethyl)-1H-pyrazole-4-carboxylate

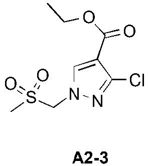

At room temperature, the compound ethyl 3-chloro-1-((methylthio)methyl)-1*H*-pyrazole-4-carboxylate (550 mg, 2.34 mmol) was dissolved in anhydrous dichloromethane (11 mL), and the mixture was stirred at 0 °C for 10 min. Subsequently, m-chloroperoxybenzoic acid (1.21 g, 7.03 mmol) was added, and the reaction liquid was slowly warmed to room temperature and successively stirred for 2 h. After the reaction of the starting material was completed as indicated by thin-layer chromatography, stirring was stopped. The reaction liquid was poured into saturated aqueous sodium thiosulfate solution (30 mL), and then the mixture was extracted with ethyl acetate (30 mL × 3). The organic phase was collected and washed with saturated aqueous sodium carbonate solution (40 mL × 3), and then dried over anhydrous sodium sulfate, filtered, and concentrated to obtain a compound ethyl 3-chloro-1-((methylsulfonyl)methyl)-1*H*-pyrazole-4-carboxylate (615 mg, yield: 98.4%). The obtained crude product could be directly used in the next step.
LC-MS, M/Z (ESI): 267.0[M+H]⁺

### Step 3: Synthesis of ethyl 3-chloro-1-(1-(methylsulfonyl)ethyl)-1H-pyrazole-4-carboxylate

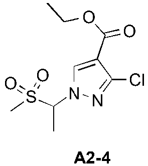

At room temperature, the compound ethyl 3-chloro-1-((methylsulfonyl)methyl)-1*H*-pyrazole-4-carboxylate (200 mg, 0.75 mmol) was dissolved in anhydrous DMF (2 mL), and then cesium carbonate (488.67 mg, 1.50 mmol) was added. The reaction liquid was stirred for 10 min, and iodomethane (212.88 mg, 1.50 mmol) was slowly added dropwise. Subsequently, the mixture was successively stirred at room temperature for 12 h. After the reaction of the starting material was completed as monitored by TLC, stirring was stopped, water was added to quench the reaction (5 mL), and the mixture was then extracted with ethyl acetate (15 mL × 3). The organic phase was collected, dried over anhydrous sodium sulfate, filtered, and concentrated, and the residue was separated and purified by silica gel column chromatography (DCM:MeOH (V/V) = 100:2) to obtain a compound ethyl 3-chloro-1-(1-(methylsulfonyl)ethyl)-1*H*-pyrazole-4-carboxylate (139 mg, yield: 66.03%).
LC-MS, M/Z (ESI): 281.0[M+H]⁺

### Step 4: Synthesis of compound 3-chloro-1-(1-(methylsulfonyl)ethyl)-1H-pyrazole-4-carboxylic acid

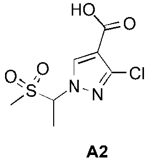

At room temperature, the compound ethyl 3-chloro-1-(1-(methylsulfonyl)ethyl)-1*H*-pyrazole-4-carboxylate (130 mg, 0.46 mmol) was dissolved in a mixed solution of methanol (2 mL) and water (0.8 mL), and sodium hydroxide (148 mg, 3.70 mmol) was then added. The reaction liquid was stirred at room temperature for 2 h. After the reaction of the starting material was completed as monitored by TLC, stirring was stopped, and methanol was removed by distillation under reduced pressure. The residue was added with water (5 mL) for dilution, the pH was adjusted to 2 with diluted hydrochloric acid (2 mol/L), and the mixture was then extracted with ethyl acetate (15 mL × 3). The organic phase was collected, dried over anhydrous sodium sulfate, and concentrated by distillation under reduced pressure to obtain a compound 3-chloro-1-(1-(methylsulfonyl)ethyl)-1*H*-pyrazole-4-carboxylic acid (110 mg, yield: 94.01%).
LC-MS, M/Z (ESI): 252.8[M+H]⁺

### Intermediate A3: Preparation of 3-chloro-1-(2-hydroxy-2-methylpropyl)-1H-pyrazole-4-carboxylic acid

The synthetic route of target intermediate **A3** was as follows:

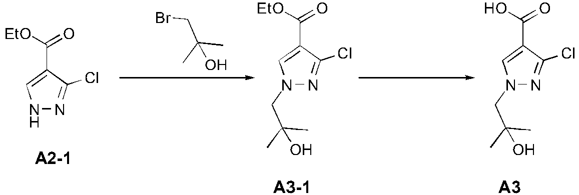

### Step 1: Synthesis of ethyl 3-chloro-1-(2-hydroxy-2-methylpropyl)-1H-pyrazole-4-carboxylate

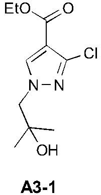

To a solution of ethyl 3-chloro-1H-pyrazole-4-carboxylate (200 mg, 1.15 mmol) in N,N-dimethylformamide (3 mL) were added 1-bromo-2-methyl-2-propanol (351 mg, 2.29 mmol), cesium carbonate (1.12 g, 3.44 mmol), and potassium iodide (190 mg, 1.15 mmol), and the mixture was stirred at 70 °C for 3 h. LCMS analysis showed that the reaction was completed. The reaction liquid was diluted with water (100 mL), and then extracted with ethyl acetate (200 mL × 3). The organic phases were combined and concentrated to obtain a crude product, and the crude product was purified by silica gel column chromatography (petroleum ether:ethyl acetate (V/V) = 100:0 to 0:100) to obtain a compound ethyl 3-chloro-1-(2-hydroxy-2-methylpropyl)-1H-pyrazole-4-carboxylate (181 mg, yield 64.1%).
LC-MS, M/Z (ESI): 247.2 [M+H]⁺.

### Step 2: Synthesis of 3-chloro-1-(2-hydroxy-2-methylpropyl)-1H-pyrazole-4-carboxylic acid

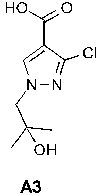

To a solution of ethyl 3-chloro-1-(2-hydroxy-2-methylpropyl)-1H-pyrazole-4-carboxylate (181 mg, 0.73 mmol) in methanol (3 mL) and water (3 mL) was added lithium hydroxide (308 mg, 7.34 mmol), and the resulting mixture was stirred at 25 °C for 2 h. LCMS analysis showed that the reaction was completed. The reaction liquid was concentrated under reduced pressure to obtain a crude product, and the crude product was purified by column chromatography (water:acetonitrile (V/V) = 100:0 to 80:20) to obtain a compound 3-chloro-1-(2-hydroxy-2-methylpropyl)-1H-pyrazole-4-carboxylic acid (150 mg, yield: 93.5%).
LC-MS, M/Z (ESI): 219.0 [M+H]⁺.

### Example 1: Preparation of N-((1S,4S)-4-((2,7-bis(trifluoromethyl))imidazo[1,2-a](pyridin-5-yl)amino)cyclohexyl)-3-chloro-1-methyl-1H-pyrazole-4-carboxamide (compound 20A)

The synthetic route of target **compound 20A** was as follows:

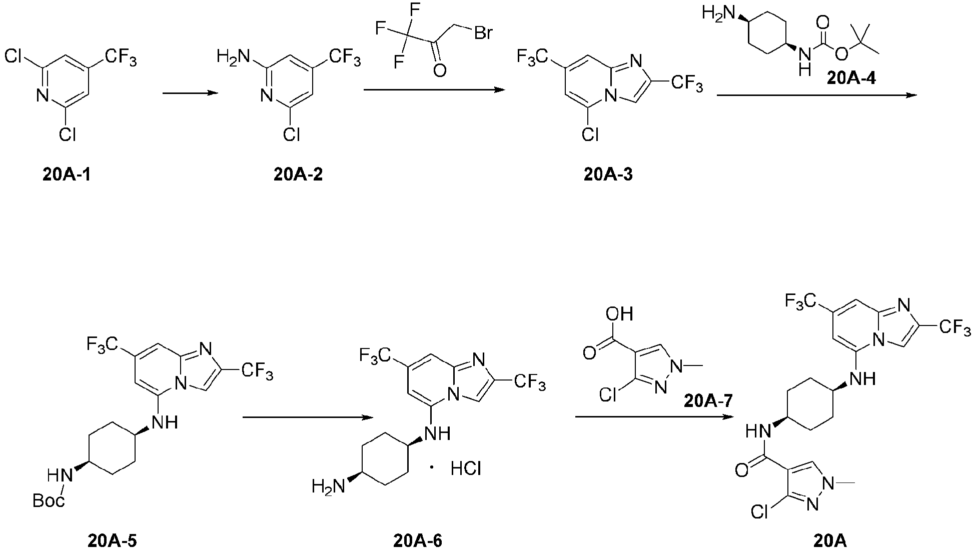

### Step 1: Synthesis of 6-chloro-4-(trifluoromethyl)pyridin-2-amine

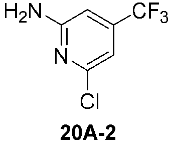

At room temperature, a compound 2,6-dichloro-4-trifluoromethylpyridine **(20A-1)** (2.00 g, 9.26 mmol) was dissolved in 10 mL of aqueous ammonia (25%), and the mixture was stirred in a closed metal container reactor at 120 °C for 12 h. After the reaction was completed, heating was stopped, the reaction system was cooled to room temperature, and the solvent was removed by rotary evaporation under reduced pressure. The resulting residue was separated and purified by silica gel column chromatography (dichloromethane:methanol (V/V) = 100:5) to obtain a compound 6-chloro-4-(trifluoromethyl)pyridin-2-amine **(20A-2)** (1.30 g, yield: 71.43%).
LC-MS, M/Z (ESI):196.80 [M+H]⁺.

### Step 2: Synthesis of 5-chloro-2,7-bis(trifluoromethyl)imidazo[1,2-a]pyridine

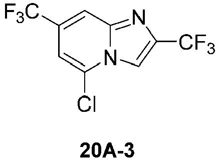

At room temperature, the compound 6-chloro-4-(trifluoromethyl)pyridin-2-amine **(20A-2)** (1.30 g, 6.61 mmol) and a compound 3-bromo-1,1,1-trifluoroacetone (1.89 g, 9.92 mmol) were dissolved in ethylene glycol (15 mL), and the reaction liquid was stirred at 120 °C for 12 h. After the reaction of the starting material was completed as monitored by TLC, stirring was stopped. The reaction liquid was cooled to room temperature, and then added with water (80 mL) for dilution, and extracted with ethyl acetate (40 mL × 5). The organic phase was collected, dried over anhydrous sodium sulfate, filtered, and concentrated. The resulting residue was separated and purified by silica gel column chromatography (petroleum ether:ethyl acetate (V/V) = 100:5) to obtain a compound 5-chloro-2,7-bis(trifluoromethyl)imidazo[1,2-*a*]pyridine **(20A-3)** (1.29 g, yield: 67.59%).
LC-MS, M/Z (ESI):289.22 [M+H]⁺

### Step 3: Synthesis of tert-butyl ((1S,4S)-4-((2,7-bis(trifluoromethyl))imidazo[1,2-a]pyridin-5-yl)amino)cyclohexyl)carbamate (20A-5)

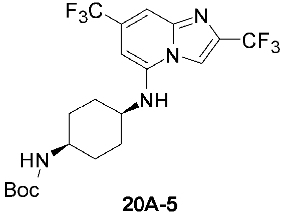

At room temperature, the compound 5-chloro-2,7-bis(trifluoromethyl)imidazo[1,2-*a*]pyridine **(20A-3)** (1.00 g, 3.47 mmol) and a compound 1-*N*-Boc-cis-1,4-cyclohexanediamine (20A-4) (1.48 g, 6.93 mmol) were dissolved in anhydrous DMSO (10 mL), and N,N-diisopropylethylamine (1.79 g, 13.96 mmol) was then added dropwise. The reaction liquid was stirred at 100 °C for 4 h. After the reaction of the starting material was completed as monitored by TLC, 50 mL of water was added for dilution, and the mixture was extracted with ethyl acetate (30 mL × 5). The organic phase was collected, dried over anhydrous sodium sulfate, and concentrated under reduced pressure. The residue was separated and purified by silica gel column chromatography (petroleum ether:ethyl acetate (V/V) = 100:20) to obtain a compound tert-butyl ((1S,4S)-4-((2,7-bis(trifluoromethyl))imidazo[1,2-*a*]pyridin-5-yl)amino)cyclohexyl)carbamate **(20A-5)** (1.60 g, yield: 73.52%).
LC-MS, M/Z (ESI):467.01 [M+H]⁺

### Step 4: Synthesis of (1S,4S)-N¹-(2,7-bis(trifluoromethyl))imidazo[1,2-a]pyridin-5-yl)cyclohexane-1,4-diamine hydrochloride

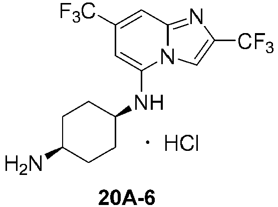

At room temperature, the compound tert-butyl ((1S,4S)-4-((2,7-bis(trifluoromethyl))imidazo[1,2-*a*]pyridin-5-yl)amino)cyclohexyl)carbamate **(20A-5)** (1.60 g, 3.43 mmol) was dissolved in dichloromethane (20 mL), and a solution of hydrogen chloride in 1,4-dioxane (4 mol/L, 4.0 mL, 16.0 mmol) was then slowly added dropwise. The reaction liquid was successively stirred at room temperature for 1 h. After the reaction was completed as monitored by TLC, the solvent was removed by distillation under reduced pressure, ethyl acetate (40 mL) was added, and the mixture was stirred at room temperature for 2 h. The suspension was filtered and dried under vacuum to obtain a compound (1S,4S)-*N*¹-(2,7-bis(trifluoromethyl))imidazo[1,2-a]pyridin-5-yl)cyclohexane-1,4-diamine hydrochloride **(20A-6)** (1.25 g, yield: 90.47%).
LC-MS, M/Z (ESI):367.30 [M+H]⁺

### Step 5: Synthesis of N-((1S,4S)-4-((2,7-bis(trifluoromethyl))imidazo[1,2-a](pyridin-5-yl)amino)cyclohexyl)-3-chloro-1-methyl-1H-pyrazole-4-carboxamide

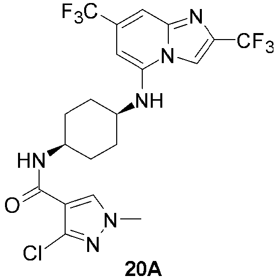

At room temperature, a compound 3-chloro-1-methyl-1*H*-pyrazole-4-carboxylic acid (200 mg, 1.25 mmol) was dissolved in anhydrous DMF (5 mL), and the mixture was stirred at 0 °C for 10 min. O-(7-azabenzotriazol-1-yl)-N,N,N',N'-tetramethyluronium hexafluorophosphate (HATU) (947.28 mg, 2.49 mmol) and N,N-diisopropylethylamine (397.85 mg, 4.98 mmol) were then added, and the reaction liquid was stirred at 0 °C for 30 min. Subsequently, the compound (1S,4S)*-N*¹-(2,7-bis(trifluoromethyl))imidazo[1,2-a]pyridin-5-yl)cyclohexane-1,4-diamine hydrochloride **(20A-6)** (501.71 mg, 1.25 mmol) was added, and the reaction liquid was warmed to room temperature and successively stirred for 12 h. After the reaction of the starting material was completed as monitored by TLC, stirring was stopped. The reaction liquid was added with water (50 mL) for dilution, and extracted with ethyl acetate (30 mL × 5). The organic phase was collected, dried over anhydrous sodium sulfate, and concentrated by distillation under reduced pressure. The resulting residue was separated and purified by column chromatography (dichloromethane:methanol (V/V) = 100: 5) to obtain a compound *N*-((1S,4S)-4-((2,7-bis(trifluoromethyl))imidazo[1,2-*a*](pyridin-5-yl)amino)cyclohexyl)-3-chloro-1-methyl-1*H-*pyrazole-4-carboxamide **(compound 20A)** (220 mg, 34.72%).
LC-MS, M/Z (ESI):509.20 [M+H]⁺
¹H NMR (400 MHz, CDCl₃) δ 7.92 (s, 1H), 7.86 (t, 1H), 7.46 (d, 1H), 6.68 (d, 1H), 6.13 (d, 1H), 4.36 (d, 1H), 4.21 (dd, 1H), 3.88 (s, 3H), 3.78 - 3.72 (m, 1H), 2.13 - 1.79 (m, 8H).

### Example 2:: Preparation of 1-methyl-N-((1R,3S)-3-((6-methyl-2-(trifluoromethyl)-1,5-naphthyridin-4-yl)amino)cyclohexyl)-1H-pyrazole-4-carboxamide (compound 21A)

The synthetic route of target compound **21A** was as follows:

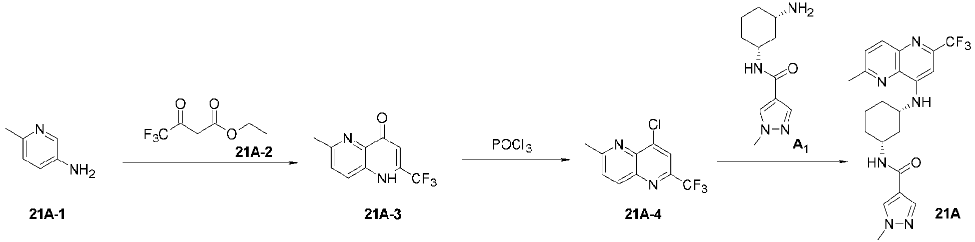

### Step 1: Synthesis of 6-methyl-2-(trifluoromethyl)-1,5-naphthyridin-4(1H)-one

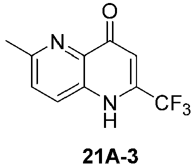

To a 100 mL single-neck flask were sequentially added 5-amino-2-methylpyridine (2 g, 18.49 mmol), diphenyl ether-biphenyl cocrystal (20 mL), and ethyl 4,4,4-trifluoroacetoacetate (4.43 g, 24.04 mmol), and the mixture was warmed to 250 °C and stirred for 7 h. After the reaction was completed, the reaction liquid was filtered, the filtrate was concentrated under reduced pressure, and the crude product was purified by silica gel column chromatography (petroleum ether/ethyl acetate (V/V) = 1:0 to 1:4) to obtain a compound 6-methyl-2-(trifluoromethyl)-1,5-naphthyridin-4(1*H*)-one **(21A-3)** (0.3 g, yield: 7.11%).
LC-MS,M/Z(ESI):229.10[M+H]⁺

### Step 2: Synthesis of 4-chloro-6-methyl-2-(trifluoromethyl)-1,5-naphthyridine

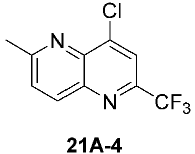

To a 100 mL double-neck flask were sequentially added 6-methyl-2-(trifluoromethyl)-1,5-naphthyridin-4(1*H*)-one (0.2 g, 0.88 mmol) and dichloroethane (5 mL), and the reaction liquid was cooled to 0 °C under nitrogen atmosphere. Subsequently, phosphorus oxychloride (1.34 g, 8.77 mmol) was slowly added, and after 10 min, the reaction liquid was heated to 80 °C and stirred for 5 h. After the reaction was completed, ice water was added to quench the reaction, and the organic phase was separated and concentrated to obtain a crude product. The crude product was purified by silica gel column chromatography (petroleum ether/ethyl acetate (V/V) = 1:0 to 20:1) to obtain a compound 4-chloro-6-methyl-2-(trifluoromethyl)-1,5-naphthyridine **(21A-4)** (120 mg, yield: 55.51%).
LC-MS,M/Z(ESI):247.15[M+H]⁺

### Step 3: Synthesis of 1-methyl-N-((1R,3S)-3-((6-methyl-2-(trifluoromethyl)-1,5-naphthyridin-4-yl)amino)cyclohexyl)-1H-pyrazole-4-carboxamide

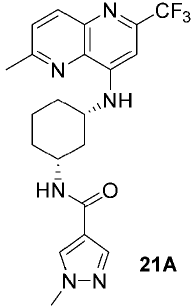

To a 100 mL single-neck flask were sequentially added 4-chloro-6-methyl-2-(trifluoromethyl)-1,5-naphthyridine (80 mg, 0.32 mmol), *N*-((1*R*,3*S*)-3-aminocyclohexyl)-1-methyl-1*H*-pyrazole-4-carboxamide (intermediate A1) (108.16 mg, 0.49 mmol), BrettPhosPdG3 (14.70 mg, 0.16 mmol), and sodium tert-butoxide (77.94 mg, 0.81 mmol), and 1,4-dioxane (8 mL) was then added. The reaction liquid was heated to 100 °C and stirred for 16 h. After the reaction was completed, the reaction liquid was cooled to room temperature and filtered, and the filtrate was concentrated to obtain a crude product. The crude product was purified by reverse-phase preparative liquid chromatography (column: YMC-Triart Prep C18 (30 mm × 40 cm, 7 µm); solvent: A = water + 0.1% aqueous ammonia, B = acetonitrile; gradient: 20%-70%) to obtain 1-methyl-*N*-((1*R*,3*S*)-3-((6-methyl-2-(trifluoromethyl)-1,5-naphthyridin-4-yl)amino)cyclohexyl)-1*H*-pyrazole-4-carboxamide **(compound 21A)** (14 mg, yield: 9.98%).
LC-MS,M/Z(ESI):433.40[M+H]⁺
¹H NMR (400 MHz, DMSO-*d*₆) δ 8.16 (d, 1H), 8.09 (s, 1H), 7.85 (d, 1H), 7.81 (s, 1H), 7.66 (d, 1H), 7.24 (d, 1H), 7.05 (s, 1H), 3.99 - 3.91 (m, 1H), 3.87-3.83 (m, 1H), 3.81 (s, 3H), 2.69 (s, 3H), 2.14 (d, 1H), 1.94 (d, 1H), 1.86-1.76 (m, 2H), 1.53-1.38 (m, 3H), 1.29-1.22 (m, 1H).

### Example 3: Preparation of N-((1R,3S)-3-((2-chloro-6-(trifluoromethyl)thieno[2,3-b]pyridin-4-yl)amino)cyclohexyl)-1-methyl-1H-pyrazole-4-carboxamide (compound 22A)

The synthetic route of **compound 22A** was as follows:

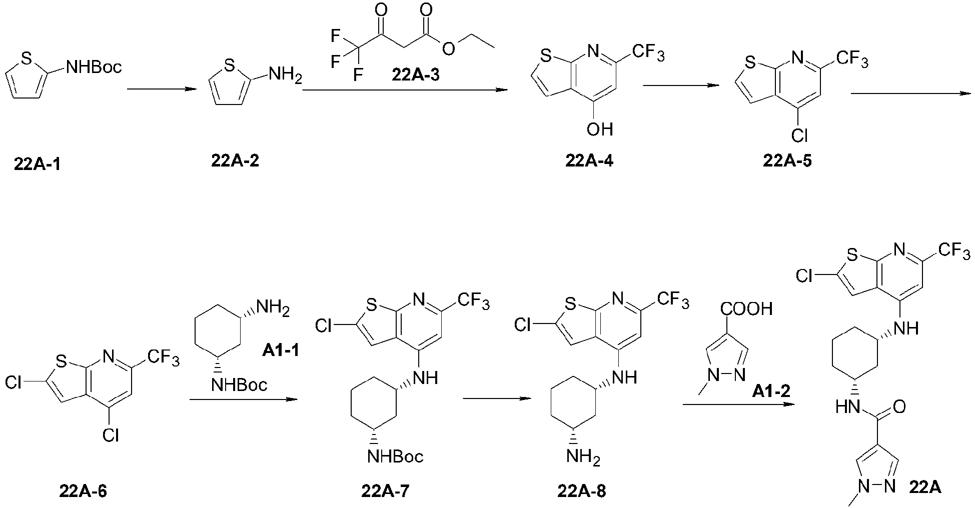

### Step 1: Synthesis of thiophen-2-amine

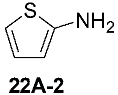

Tert-butyl thiophen-2-ylcarbamate (2.00 g, 10.0 mmol) was dissolved in a solution of hydrogen chloride in 1,4-dioxane (20 mL, 4 mol/L), and the reaction liquid was stirred at 25 °C under nitrogen atmosphere for 1 h. After the reaction was completed, the reaction liquid was concentrated under reduced pressure, and dichloromethane (10 mL) was then added for dissolution. The reaction liquid was concentrated under reduced pressure again, and this process was repeated three times to obtain a crude product thiophen-2-amine **(22A-2)** (1.35 g, yield: 98.4%). The crude product was directly used in the next step.
LC-MS, M/Z (ESI): 100.2 [M+H]⁺.

### Step 2: Synthesis of 6-(trifluoromethyl)thieno[3,2-b]pyridin-4-ol

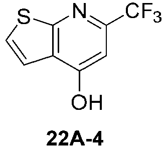

Thiophen-2-amine (4.80 g, 35.0 mmol) was dissolved in polyphosphoric acid (50 mL) and ethyl 4,4,4-trifluoroacetoacetate (6.45 g, 35.0 mmol) was added under nitrogen atmosphere. The reaction liquid was stirred at 140 °C under N ₂ atmosphere for 18 h. The reaction mixture was cooled to room temperature, added with saturated sodium bicarbonate solution (500 mL) for dilution, and then extracted with ethyl acetate (500 mL × 3). The organic layers were combined, and the organic phase was washed with saturated saline (500 mL), dried over anhydrous sodium sulfate, and concentrated under reduced pressure to obtain a crude product. The crude product was separated and purified by silica gel column chromatography (petroleum ether:ethyl acetate (V/V) = 100:0 to 80:20, gradient elution) to obtain a compound 6-(trifluoromethyl)thieno[3,2-*b*]pyridin-4-ol **(22A-4)** (750 mg, yield: 9.3%).
¹H NMR (400 MHz, CDCl₃): δ 7.55 (d, *J =* 6.0 Hz, 1H), 7.46 (d, *J =* 6.0Hz, 1H), 7.09 (s, 1H).
LC-MS, M/Z (ESI): 220.2 [M+H]⁺.

### Step 3: Synthesis of 4-chloro-6-(trifluoromethyl)thieno[2,3-b]pyridine

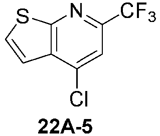

6-(trifluoromethyl)thieno[3,2-*b*]pyridin-4-ol (683 mg, 3.12 mmol) was dissolved in toluene (7 mL), and N,N-dimethylformamide (0.1 mL) and thionyl chloride (1.85 g, 15.6 mmol) were sequentially added. The reaction liquid was stirred at 25 °C for 4 h. The production of the target product was detected by LC-MS. The reaction system was added with water (20 mL) for dilution and extracted with ethyl acetate (10 mL × 3). The organic phase was washed with saturated saline (10 mL), then dried over anhydrous sodium sulfate, and concentrated under reduced pressure to obtain a crude product. The crude product was separated and purified by silica gel column chromatography (petroleum ether:ethyl acetate (V/V) = 100:0 to 80:20, gradient elution) to obtain a compound 4-chloro-6-(trifluoromethyl)thieno[2,3-b]pyridine **(22A-5)** (490 mg, yield: 62.91%).
LC-MS, M/Z (ESI): 238.0 [M+H]⁺.

### Step 4: Synthesis of 2,4-dichloro-6-(trifluoromethyl)thieno[2,3-b]pyridine

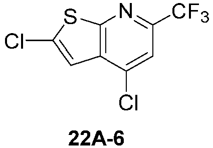

2,2,6,6-tetramethylpiperidine (0.46 mL, 2.73 mmol) was dissolved in tetrahydrofuran (10 mL), and the mixture was purged with nitrogen. n-butyllithium (2.18 mL, 5.45 mmol, 2.5 mol/L n-hexane solution) was added dropwise at - 78 °C. The reaction liquid was stirred at -78 °C for 1 h. A solution of 4-chloro-6-(trifluoromethyl)thieno[2,3-b]pyridine (648 mg, 2.73 mmol) in tetrahydrofuran (2 mL) was slowly added dropwise into the reaction system, and the reaction liquid was stirred at -78 °C for 1 h. Subsequently, a solution of hexachloroethane (775 mg, 3.27 mmol) in tetrahydrofuran (1 mL) was slowly added dropwise into the reaction system, and the resulting reaction liquid was stirred at -78 °C for 1 h, then gradually warmed to 25 °C and successively stirred for 14 h. When the starting material disappeared as detected by LC-MS, water (50 mL) was added to the reaction mixture to quench the reaction, and the mixture was extracted with ethyl acetate (50 mL × 3). The organic layers were combined, dried over anhydrous sodium sulfate, filtered, and concentrated to obtain a crude product. The crude product was separated and purified by silica gel column chromatography (petroleum ether:ethyl acetate (V/V) = 100:0 to 80:20, gradient elution) to obtain a compound 2,4-dichloro-6-(trifluoromethyl)thieno[2,3-*b*]pyridine **(22A-6)** (377 mg, yield: 48.4%).
¹H NMR (400 MHz, CDCl₃): δ 7.67 (s, 1H), 7.35 (s, 1H).
LC-MS, M/Z (ESI): 272.0 [M+H]⁺.

### Step 5: Synthesis of tert-butyl ((1R,3S))-3-((2-chloro-6-trifluoromethyl))thieno[2,3-b]pyridin-4-yl)amino)cyclohexyl)carbamate

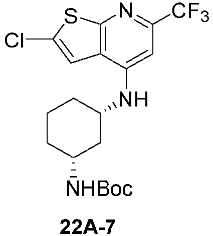

2,4-dichloro-6-(trifluoromethyl)thieno[2,3-*b*]pyridine (250 mg, 0.92 mmol) and tert-butyl (((1*R*,3*S*)-3-aminocyclohexyl)amino)carboxylate (207 mg, 0.97 mmol) were dissolved in N-methylpyrrolidone (5 mL), and N,N-diisopropylethylamine (166 mg, 1.29 mmol) was added. The reaction liquid was stirred at 130 °C for 2 h. The reaction liquid was cooled to room temperature, water (20 mL) was added to the reaction liquid, and the reaction liquid was extracted with ethyl acetate (10 mL × 3). The organic layers were combined, washed with saturated saline (20 mL), dried over anhydrous sodium sulfate, and concentrated to obtain a crude product. The crude product was separated and purified by silica gel column chromatography (petroleum ether:ethyl acetate (V/V) = 100:0 to 80:20, gradient elution) to obtain a compound tert-butyl ((1*R*,3*S*))-3-((2-chloro-6-trifluoromethyl))thieno[2,3-*b*]pyridin-4-yl)amino)cyclohexyl)carbamate **(22A-7)** (270 mg, yield: 64.00%).
LC-MS, M/Z (ESI): 450.2 [M+H]⁺.

### Step 6: Synthesis of (1R,3S)-N¹-((2-chloro-6-(trifluoromethyl)thieno[2,3-b]pyridin-4-yl)cyclohexane-1,3-diamine

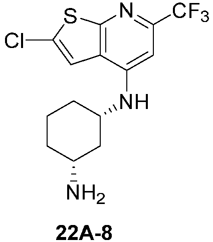

Tert-butyl ((*1*R,3*S*))-3-((2-chloro-6-trifluoromethyl))thieno[2,3-*b*]pyridin-4-yl)amino)cyclohexyl)carbamate (150 mg, 0.33 mmol) was dissolved in anhydrous dichloromethane (5 mL), and trifluoroacetic acid (2 mL) was added. The reaction liquid was stirred at 25 °C for 1 h. The production of the target product was detected by LC-MS. The reaction liquid was concentrated under reduced pressure to obtain a residue, and the residue was dissolved with dichloromethane (10 mL). The resulting liquid was further concentrated under reduced pressure to obtain a crude product compound (1*R*,3*S*)-N¹-((2-chloro-6-(trifluoromethyl)thieno[2,3-b]pyridin-4-yl)cyclohexane-1,3-diamine (**22A-8**) (110 mg, yield: 92.4%). The crude product was directly used in the next step.
LC-MS, M/Z (ESI): 350.2 [M+H]⁺.

### Step 7: Synthesis of N-((1R,3S)-3-((2-chloro-6-(trifluoromethyl)thieno[2,3-b]pyridin-4-yl)amino)cyclohexyl)-1-methyl-1H-pyrazole-4-carboxamide

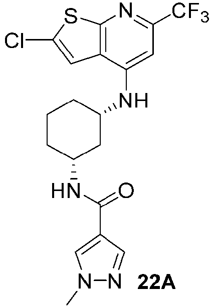

(1*R*,3*S*)-N¹-((2-chloro-6-(trifluoromethyl)thieno[2,3-b]pyridin-4-yl)cyclohexane-1,3-diamine (100 mg, 0.29 mmol) and 1-methylpyrazole-4-carboxylic acid (43.3 mg, 0.34 mmol) were dissolved in anhydrous dimethylsulfoxide (2 mL), and N,N-diisopropylethylamine (185 mg, 1.43 mmol), 1-(3-dimethylaminopropyl)-3-ethylcarbodiimide hydrochloride (164 mg, 0.86 mmol), and 1-hydroxybenzotriazole (116 mg, 0.86 mmol) were sequentially added. The reaction liquid was stirred at 25 °C under nitrogen atmosphere for 18 h. The production of the target product was detected by LC-MS. The reaction liquid was dissolved in saturated saline (20 mL), and then extracted with ethyl acetate (10 mL × 3). The organic layers were combined, dried over anhydrous sodium sulfate, and concentrated to obtain a crude product. The crude product was separated and purified by a reverse-phase silica gel column C18 (water:acetonitrile (V/V) = 100:0 to 70:30, gradient elution) to obtain *N*-((1*R*,3*S*)-3-((2-chloro-6-(trifluoromethyl)thieno[2,3-*b*]pyridin-4-yl)amino)cyclohexyl)-1-methyl-1*H*-pyrazole-4-carboxamide **(compound 22A)** (88.3 mg, yield: 66.6%).

LC-MS, M/Z (ESI): 458.2 [M+H]⁺.

¹H NMR (400 MHz, DMSO-*d₆*): δ 8.10 (s, 1H), 7.89 (s, 1H), 7.87 (d, 1H), 7.82 (s, 1H), 7.23 (d, 1H), 6.98 (s, 1H), 4.02 - 3.90 (m, 1H), 3.83 (s, 4H), 2.13 - 2.07 (m, 1H), 1.96 - 1.90 (m, 1H), 1.87 - 1.76 (m, 2H), 1.55 - 1.46 (m, 1H), 1.41 - 1.31 (m, 1H), 1.25 - 1.21 (m, 2H).

### Example 4: Preparation of N-((1R,3S)-3-((2-chloro-5-(trifluoromethyl)thieno[3,2-b]pyridin-7-yl)amino)cyclohexyl)-1-methyl-1H-pyrazole-4-carboxamide (compound 23A) trifluoroacetate

The synthetic route of **compound 23A** trifluoroacetate was as follows:

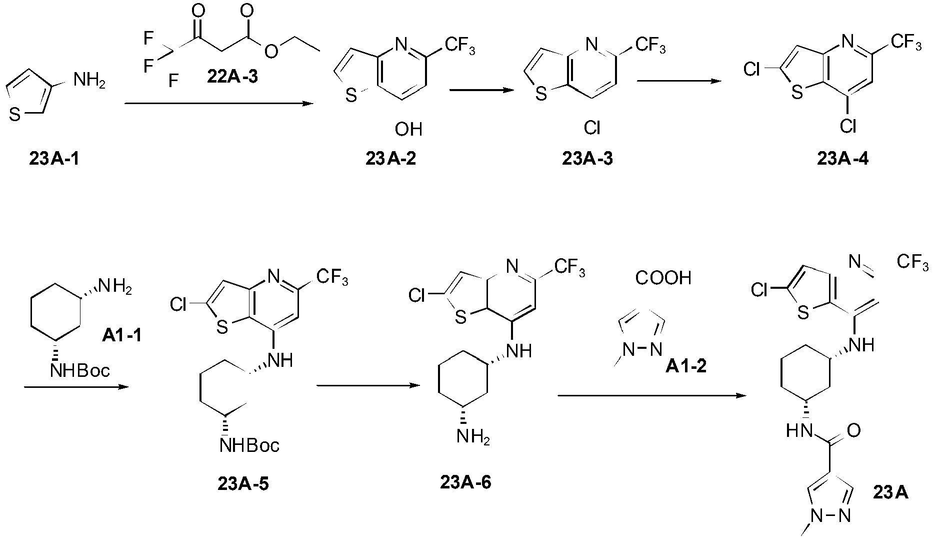

### Step 1: Synthesis of 5-(trifluoromethyl)thieno[3,2-b]pyridin-7-ol

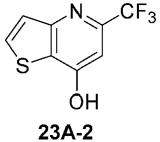

To a solution of 3-aminothiophene (1.00 g, 5.43 mmol) in polyphosphoric acid (10 mL) was added ethyl trifluoroacetoacetate (0.54 g, 5.43 mmol), and the reaction liquid was stirred at 140 °C under nitrogen atmosphere for 16 h. The reaction mixture was cooled to room temperature, then diluted with saturated aqueous sodium bicarbonate solution (100 mL), and extracted with ethyl acetate (100 mL × 3). The organic layers were combined, and the organic phase was washed with saturated saline (50 mL), dried over anhydrous sodium sulfate, and concentrated to obtain a crude product. The crude product was separated and purified by silica gel column chromatography (petroleum ether:ethyl acetate (V/V) = 100:1 to 10:1, gradient elution) to obtain a compound 5-(trifluoromethyl)thieno[3,2-*b*]pyridin-7-ol (720 mg, yield: 55.3%).

¹H NMR (400 MHz, DMSO-*d₆*): δ 8.17 (d, *J =* 5.2 Hz, 1H), 7.57 (d, *J =* 5.6Hz, 1H), 7.02 (s, 1H), 3.87 (s, 1H).

LC-MS, M/Z (ESI): 220.2 [M+H]⁺.

### Step 2: Synthesis of 7-chloro-5-(trifluoromethyl)thieno[3,2-b]pyridine

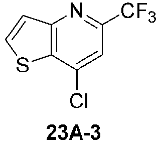

5-(trifluoromethyl)thieno[3,2-*b*]pyridin-7-ol (360 mg, 1.64 mmol) was dissolved in phosphorus oxychloride (3 mL), and the reaction liquid was stirred at 60 °C for 2 h. The reaction liquid was concentrated under reduced pressure to remove phosphorus oxychloride, and water (10 mL) was added to the residue. The resulting mixture was extracted with ethyl acetate (10 mL × 3), and the organic layers were combined, dried over anhydrous sodium sulfate, and concentrated to obtain a crude product. The crude product was separated and purified by silica gel column chromatography (petroleum ether:ethyl acetate (V/V) = 100:1 to 5:1, gradient elution) to obtain 7-chloro-5-(trifluoromethyl)thieno[3,2-*b*]pyridine (292 mg, yield: 71.1%).

LC-MS, M/Z (ESI): 238.2 [M+H]⁺.

### Step 3: Synthesis of 2,7-dichloro-5-(trifluoromethyl)thieno[3,2-b]pyridine

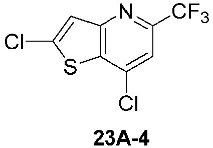

2,2,6,6-tetramethylpiperidine (256 mg, 1.81 mmol) was dissolved in tetrahydrofuran (5 mL), and the mixture was purged with nitrogen. n-butyllithium (0.66 mL, 1.65 mmol, 2.5 M) was added dropwise at -78 °C. The reaction liquid was allowed to react at -78 °C for 1 h, a solution of 7-chloro-5-(trifluoromethyl)thieno(3,2-*b*)pyridine (200 mg, 0.82 mmol) in tetrahydrofuran (1 mL) was added dropwise, and the resulting mixture was stirred at -78 °C for 1 h. Subsequently, a solution of hexachloroethane (234 mg, 0.99 mmol) in tetrahydrofuran (1 mL) was slowly added dropwise, and the resulting reaction liquid was stirred at -78 °C for 1 h, and then gradually warmed to 25 °C and stirred for 14 h. Water (10 mL) was added to the reaction mixture to quench the reaction, and the mixture was extracted with ethyl acetate (10 mL × 3). The organic layers were combined, dried over anhydrous sodium sulfate, and concentrated to obtain a crude product. The crude product was separated and purified by silica gel column chromatography (petroleum ether:ethyl acetate (V/V) = 100:1 to 10:1, gradient elution) to obtain 2,7-dichloro-5-(trifluoromethyl)thieno[3,2-*b*]pyridine (130 mg, yield: 52.1%).

LC-MS, M/Z (ESI): 272.0 [M+H]⁺.

¹H NMR (400 MHz, DMSO-*d₆*): δ 8.21 (s, 1H), 8.04 (s, 1H).

### Step 4: Synthesis of tert-butyl (1R,3S)-3-((2-chloro-5-(trifluoromethyl)thieno[3,2-b]pyridin-7-yl)amino)cyclohexyl)carbamate

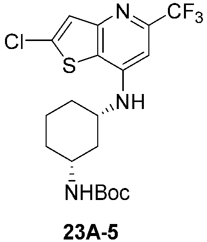

2,7-dichloro-5-(trifluoromethyl)thieno[3,2-*b*]pyridine (100 mg, 0.37 mmol) and tert-butyl (((1*R*,3*S*)-3-aminocyclohexyl)amino)carboxylate (82.7 mg, 0.39 mmol) were dissolved in N-methylpyrrolidone (2 mL), and N,N-diisopropylethylamine (0.06 mL, 0.37 mmol) was added. The reaction liquid was stirred at 130 °C for 3 h. Water (30 mL) was added to the reaction liquid, and the reaction liquid was extracted with ethyl acetate (10 mL × 3). The organic layers were combined, washed with saturated saline (10 mL × 3), dried over anhydrous sodium sulfate, and concentrated to obtain a crude product. The crude product was separated and purified by silica gel column chromatography (petroleum ether:ethyl acetate (V/V) = 100:1 to 5:1, gradient elution) to obtain tert-butyl (1*R*,3*S*)-3-((2-chloro-5-(trifluoromethyl)thieno[3,2-*b*]pyridin-7-yl)amino)cyclohexyl)carbamate (100 mg, yield: 54.4%).

LC-MS, M/Z (ESI): 450.2 [M+H]⁺.

### Step 5: Synthesis of (1S,3R)-N¹-(2-chloro-5-(trifluoromethyl)thieno[3,2-b]pyridin-7-yl)cyclohexane-1,3-diamine

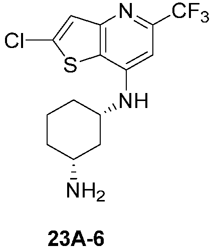

Tert-butyl (1*R*,3*S*)-3-((2-chloro-5-(trifluoromethyl)thieno[3,2-b]pyridin-7-yl)amino)cyclohexyl)carbamate (200 mg, 0.45 mmol) was dissolved in anhydrous dichloromethane (4 mL), and trifluoroacetic acid (2 mL) was added. The reaction liquid was stirred at 25 °C for 2 h. The reaction liquid was concentrated under reduced pressure to obtain a crude product, and the crude product was separated and purified by silica gel column chromatography (dichloromethane:methanol (V/V) = 10:1 to 1:1, gradient elution) to obtain (1*S,*3*R*)*-N* ¹-(2-chloro-5-(trifluoromethyl)thieno[3,2-*b*]pyridin-7-yl)cyclohexane-1,3-diamine (20 mg, yield: 11.6%).

LC-MS, M/Z (ESI): 350.2 [M+H]⁺.

### Step 6: Synthesis of N-((1R,3S)-3-((2-chloro-5-(trifluoromethyl)thieno[3,2-b]pyridin-7-yl)amino)cyclohexyl)-1-methyl-1H-pyrazole-4-carboxamide

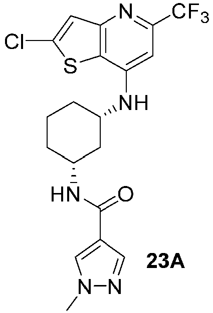

(1*S*,3*R*)-*N*¹-(2-chloro-5-(trifluoromethyl)thieno[3,2-*b*]pyridin-7-yl)cyclohexane-1,3-diamine (10.0 mg, 0.03 mmol) and 1-methylpyrazole-4-carboxylic acid (4.33 mg, 0.03 mmol) were dissolved in anhydrous dimethylsulfoxide (1 mL), and N,N-diisopropylethylamine (2 drops), 1-(3-dimethylaminopropyl)-3-ethylcarbodiimide hydrochloride (16.7 mg, 0.09 mmol), and 1-hydroxybenzotriazole (11.76 mg, 0.09 mmol) were sequentially added. The reaction liquid was stirred at 25 °C under nitrogen atmosphere for 18 h. The reaction liquid was added to saturated saline (10 mL), and then extracted with ethyl acetate (10 mL × 3). The organic layers were combined, dried over anhydrous sodium sulfate, and concentrated to obtain a crude product. The crude product was purified by preparative high performance liquid chromatography (Xtimate C18, 21.2 × 250 mm, 5 µm; 0.1% TFA-ACN; 40-70; 20 mL/min) to obtain a compound *N*-((1*R*,3*S*)-3-((2-chloro-5-(trifluoromethyl)thieno[3,2-*b*]pyridin-7-yl)amino)cyclohexyl)-1-methyl-1*H-*pyrazole-4-carboxamide trifluoroacetate (3.50 mg, yield: 21.4%).

LC-MS, M/Z (ESI): 458.0 [M+H]⁺.

¹H NMR (400 MHz, MeOD): δ 8.03 (s, 1H), 7.88 (s, 1H), 7.37 (s, 1H), 7.04 (s, 1H), 4.10 - 3.98 (m, 1H), 3.90 (s, 3H), 3.89 - 3.77 (m, 1H), 2.40 - 2.29 (m, 1H), 2.12 - 2.05 (m, 1H), 2.01 - 1.91 (m, 2H), 1.66 - 1.57 (m, 1H), 1.51 - 1.35 (m, 3H).

### Example 5: Preparation of 1-methyl-N-((1R,3S)-3-((1-methyl-6-oxo-5-(trifluoromethyl)-1,6-dihydropyridazin-3-yl)amino)cyclohexyl)-1H-pyrazole-4-carboxamide (compound 26A)

The synthetic route of **compound 26A** was as follows:

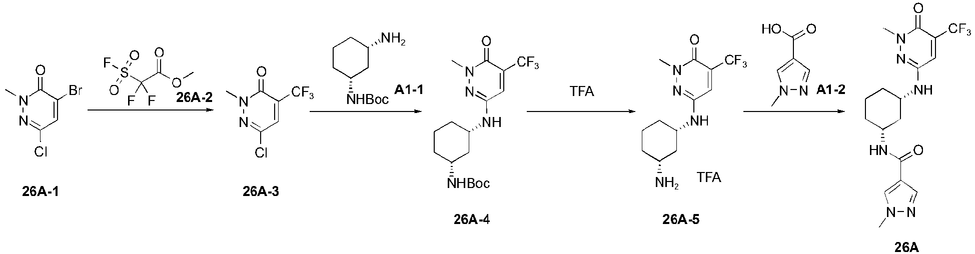

### Step 1: Synthesis of 6-chloro-2-methyl-4-(trifluoromethyl)pyridazin-3(2H)-one

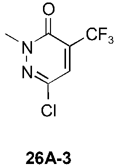

4-bromo-6-chloro-2-methylpyridazin-3(2H)-one (500 mg, 2.24 mmol) was dissolved in a solvent N-methylpyrrolidone (5 mL), and methyl fluorosulfonyldifluoroacetate (12.9 g, 6.71 mmol) and copper(I) iodide (85.3 g, 0.448 mmol) were added. The reaction liquid was stirred at 80 °C for 18 h. LC-MS analysis showed that the reaction was completed. Water (30 mL) was added to the reaction liquid, and the reaction liquid was extracted with ethyl acetate (50 mL × 3). The organic layers were combined and washed with saturated saline (100 mL), then dried over anhydrous sodium sulfate, filtered, and concentrated. The residue was separated and purified by silica gel column chromatography (dichloromethane:ethyl acetate (V/V) = 100:0 to 95:5) to obtain a compound 6-chloro-2-methyl-4-(trifluoromethyl)pyridazine-3(2H)-one (300 mg, yield: 63.1%).

LC-MS, M/Z (ESI):213.0 [M+H]⁺.

### Step 2: Synthesis of tert-butyl ((1R,3S)-3-((1-methyl-6-oxo-5-(trifluoromethyl)-1,6-dihydropyridazin-3-yl)amino)cyclohexyl)carbamate

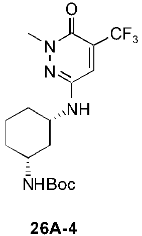

6-chloro-2-methyl-4-(trifluoromethyl)pyridazin-3(2H)-one (500 mg, 2.35 mmol) was dissolved in toluene (10 mL), and tert-butyl (1R,3S)-3-aminocyclohexyl)carbamate (504 mg, 2.35 mmol), methanesulfonato(2-dicyclohexylphosphino-2',4',6'-triisopropyl-1,1'-biphenyl)(2'-amino-1,1'-biphenyl-2-yl)palladium(II) (200 mg, 0.235 mmol), and cesium carbonate (1.53 g, 4.71 mmol) were added. The reaction liquid was stirred at 80 °C for 14 h. LC-MS analysis showed that the reaction was completed. Water (50 mL) was added to the reaction system, and the reaction liquid was extracted with ethyl acetate (50 mL × 3). The organic layers were combined and washed with saturated saline (30 mL × 3), then dried over anhydrous sodium sulfate, filtered, and concentrated. The residue was separated and purified by silica gel column chromatography (petroleum ether:ethyl acetate (V/V) = 100:0 to 90: 10) to obtain tert-butyl ((1R,3S)-3-((1-methyl-6-oxo-5-(trifluoromethyl)-1,6-dihydropyridazin-3-yl)amino)cyclohexyl)carbamate (600 mg, yield: 65.3%).

LC-MS, M/Z (ESI): 391.4 [M+H]⁺.

### Step 3: Synthesis of 6-(((1S,3R)-3-aminocyclohexyl)amino)-2-methyl-4-(trifluoromethyl)pyridazin-3(2H)-one trifluoroacetate

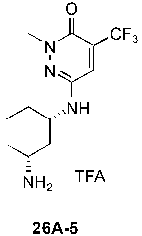

Tert-butyl ((1R,3 S)-3-((1-methyl-6-oxo-5-(trifluoromethyl)-1,6-dihydropyridazin-3-yl)amino)cyclohexyl)carbamate (600 mg, 1.54 mmol) was dissolved in a solvent dichloromethane (5 mL), and trifluoroacetic acid (2 mL) was added. The reaction liquid was allowed to react at 25 °C for 2 h. LC-MS analysis showed that the reaction was completed. The reaction liquid was concentrated under reduced pressure to obtain a crude product of compound 6-(((1S,3R)-3-aminocyclohexyl)amino)-2-methyl-4-(trifluoromethyl)pyridazin-3(2H)-one trifluoroacetate (600 mg, yield: 80.7%).

LC-MS, M/Z (ESI): 291.4 [M+H]⁺.

### Step 4: 1-methyl-N-((1R,3S)-3-((1-methyl-6-oxo-5-(trifluoromethyl)-1,6-dihydropyridazin-3-yl)amino)cyclohexyl)-1H-pyrazole-4-carboxamide

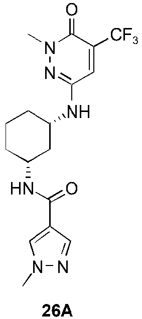

6-(((1S,3R)-3-aminocyclohexyl)amino)-2-methyl-4- (trifluoromethyl)pyridazin-3(2H)-one trifluoroacetate (300 mg, 0.620 mmol) and 1-methylpyrazole-4-carboxylic acid (78.2 mg, 0.620 mmol) were dissolved in dimethylsulfoxide (5 mL), and 1-ethyl-(3-dimethylaminopropyl)carbodiimide hydrochloride (178 mg, 0.930 mmol), 1-hydroxybenzotriazole (126 mg, 0.930 mmol), and N,N-diisopropylethylamine (401 mg, 3.10 mmol) were added. The reaction liquid was stirred at 25 °C under N₂ atmosphere for 2 h. LC-MS analysis showed that the reaction was completed. The reaction liquid was concentrated under reduced pressure, and the residue was purified by preparative high performance liquid chromatography (Xtimate C18 21.2 × 250 mm 5 µm; 0.1% NH₃.H₂O-ACN; 40-52; 20 mL/min) to obtain 1-methyl-N-((1R,3S)-3-((1-methyl-6-oxo-5-(trifluoromethyl)-1,6-dihydropyridazin-3-yl)amino)cyclohexyl)-1H-pyrazole-4-carboxamide (160 mg, yield: 64.9%).

LC-MS, M/Z (ESI): 399.4 [M+H]⁺.

¹H NMR (400 MHz, DMSO-*d₆*): δ 8.09 (s, 1H), 7.84 (d, 1H), 7.81 (d, 1H), 7.41 (s, 1H), 6.59 (d, 1H), 3.83 (s, 3H), 3.82 - 3.74 (m, 1H), 3.53 - 3.46 (m, 4H), 2.20 - 2.14 (m, 1H), 2.03 - 1.96 (m, 1H), 1.83 - 1.73 (m, 2H), 1.43 - 1.31 (m, 1H), 1.25 - 1.11 (m, 2H), 1.08 - 0.98 (m, 1H).

### Example 6: Preparation of N-((1S,4S)-4-((1-methyl-6-oxo-5-(trifluoromethyl)-1,6-dihydropyridazin-3-yl)amino)cyclohexyl)-1-(2,2,2-trifluoroethyl)-1H-pyrazole-4-carboxamide (compound 27A)

The synthetic route of **compound 27A** was as follows:

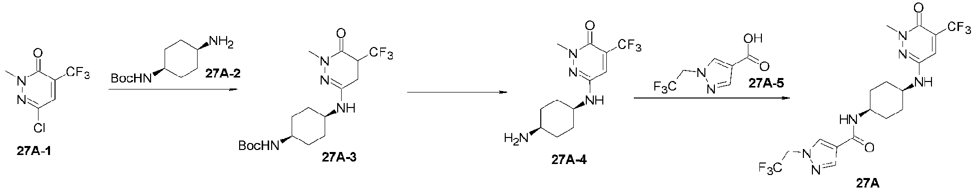

### Step 1: Synthesis of tert-butyl (1S,4S)-4-((1-methyl-6-oxo-5-(trifluoromethyl)-1,6-dihydropyridazin-3-yl)amino)cyclohexyl)carbamate

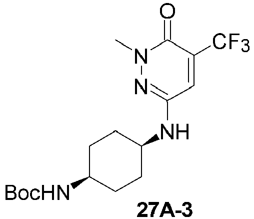

To a solution of 6-chloro-2-methyl-4-(trifluoromethyl)pyridazin-3(2H)-one (1.00 g, 4.70 mmol) in 1,4-dioxane (15 mL) were added tert-butyl cis-4-aminocyclohexyl)carbamate (1.00 g, 21.2 mmol), cesium carbonate (3.06 g, 9.41 mmol), and methanesulfonato(2-dicyclohexylphosphino-2',4',6'-triisopropyl-1,1'-biphenyl)(2'-amino-1,1'-biphenyl-2-yl)palladium(II) (200 mg, 0.240 mmol). The reaction liquid was stirred at 80 °C under nitrogen atmosphere for 18 h. LCMS analysis showed that the reaction was completed. The reaction liquid was filtered, the filtrate was added with water (100 mL) for dilution, and the resulting mixture was extracted with ethyl acetate (100 mL × 3). The organic layers were combined and washed with saturated saline (100 mL × 3), dried over anhydrous sodium sulfate, filtered, and concentrated to obtain a residue. The residue was separated and purified by silica gel column chromatography (petroleum ether:ethyl acetate (V/V) = 100:0 to 80:20) to obtain a compound tert-butyl (1S,4S)-4-((1-methyl-6-oxo-5-(trifluoromethyl)-1,6-dihydropyridazin-3-yl)amino)cyclohexyl)carbamate (27A-2) (700 mg, yield: 38.1%).
LC-MS, M/Z (ESI): 391.2 [M+H]⁺

### Step 2: Synthesis of 6-(((1S,4S)-4-aminocyclohexyl)amino)-2-methyl-4-(trifluoromethyl)pyridazin-3(2H)-one (27A-3)

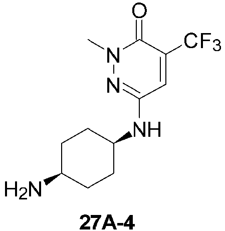

To a solution of tert-butyl cis-4-((1-methyl-6-oxo-5-(trifluoromethyl)-1,6-dihydropyridazin-3-yl)amino)cyclohexyl)carbamate (27A-2) (300 mg, 0.760 mmol) in dichloromethane (10 mL) was added trifluoroacetic acid (3.00 mL), and the reaction liquid was stirred at 25 °C for 1 h. LCMS analysis showed that the reaction was completed. The reaction liquid was poured into saturated aqueous sodium bicarbonate solution (50 mL), and the resulting mixture was extracted with dichloromethane (20 mL × 3). The organic phases were combined, dried over anhydrous sodium sulfate, filtered, and concentrated to obtain a compound 6-(((1S,4S)-4-aminocyclohexyl)amino)-2-methyl-4-(trifluoromethyl)pyridazin-3(2H)-one (27A-3) (300 mg, yield: 80.5%). The compound was directly used in the next step.
LC-MS, M/Z (ESI): 291.2 [M+H]⁺

### Step 3: Synthesis of N-((1S,4S)-4-((1-methyl-6-oxo-5-(trifluoromethyl)-1,6-dihydropyridazin-3-yl)amino)cyclohexyl)-1-(2,2,2-trifluoroethyl)-1H-pyrazole-4-carboxamide (compound 27A)

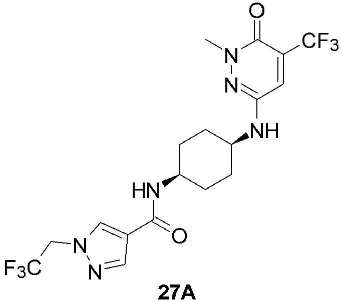

To a solution of 6-(((1S,4S)-4-aminocyclohexyl)amino)-2-methyl-4-(trifluoromethyl)pyridazin-3(2H)-one (27A-3) (250 mg, 0.860 mmol) in dimethylsulfoxide (2.0 mL) were added 1-(2,2,2-trifluoroethyl)-1H-pyrazole-4-carboxylic acid (193 mg, 0.990 mmol), EDCI (237 mg, 1.28 mmol), HOBt (174 mg, 224 mmol), and DIEA (0.64 mL). The reaction liquid was allowed to react at 25 °C for 1 h. LCMS analysis showed that the reaction was completed. The reaction liquid was subjected to suction filtration, and water (50 mL) was then added. The reaction liquid was extracted with ethyl acetate (50 mL × 3) and washed with saturated saline (50 mL × 3). The organic layers were combined, dried over anhydrous sodium sulfate, and concentrated to obtain a crude product. The crude product was purified by preparative high performance liquid chromatography (Xtimate C₁₈ 21.2 × 250 mm 5 µm; 0.1% NH3.H₂O-ACN; 30-62; 40 mL/min) to obtain N-((1S,4S)-4-((1-methyl-6-oxo-5-(trifluoromethyl)-1,6-dihydropyridazin-3-yl)amino)cyclohexyl)-1-(2,2,2-trifluoroethyl)-1H-pyrazole-4-carboxamide (compound 27A) (151 mg, yield: 62.7%).
LC-MS, M/Z (ESI): 467.4 [M+H]⁺
¹H NMR (400 MHz, DMSO-*d₆*): δ 8.31 (s, 1H), 8.00 (s, 1H), 7.94 (d, 1H), 7.56 (s, 1H), 6.59 (d, 1H), 5.18 (q, 2H), 3.85 - 3.74 (m, 1H), 3.67 - 3.60 (m, 1H), 3.50 (s, 3H), 1.91 - 1.80 (m, 2H), 1.69 - 1.56 (m, 6H).

### Example 7: Preparation of 3-chloro-N-((1S,4S)-4-((1-methyl-6-oxo-5-(trifluoromethyl)-1,6-dihydropyridazin-3-yl)amino)cyclohexyl)-1-(2,2,2-trifluoroethyl)-1H-pyrazole-4-carboxamide (compound 28A)

The synthetic route of **compound 28A** was as follows:

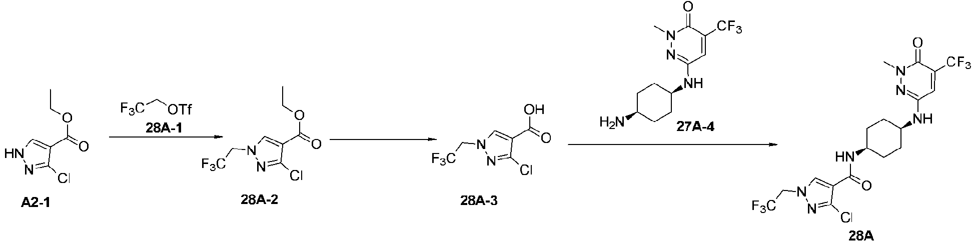

### Step 1: Synthesis of ethyl 3-chloro-1-(2,2,2-trifluoroethyl)-1H-pyrazole-4-carboxylate

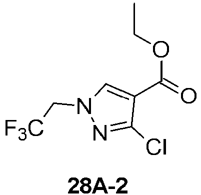

To a solution of ethyl 3-chloro-1H-pyrazole-4-carboxylate (1.00 g, 5.72 mmol) in N,N-dimethylformamide (10 mL) were added 2,2,2-trifluoroethyl trifluoromethanesulfonate (3.90 g, 17.2 mmol) and cesium carbonate (5.60 g, 17.2 mmol), and the reaction liquid was stirred at 25 °C for 18 h. LCMS analysis showed that the reaction was completed. Water (100 mL) was added to the reaction liquid, and the reaction liquid was extracted with ethyl acetate (100 mL × 3). The organic layers were combined and washed with saturated saline (100 mL), dried over anhydrous sodium sulfate, filtered, and concentrated to obtain a crude product. The crude product was separated and purified by silica gel column chromatography (petroleum ether:ethyl acetate (V/V) = 100:0 to 75:25) to obtain a compound ethyl 3-chloro-1-(2,2,2-trifluoroethyl)-1H-pyrazole-4-carboxylate (1.20 g, yield: 81.6%).
LC-MS, M/Z (ESI): 257.4 [M+H]⁺

### Step 2: Synthesis of 3-chloro-1-(2,2,2-trifluoroethyl)-1H-pyrazole-4-carboxylic acid (compound 28A-4)

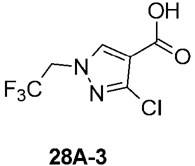

To a solution of ethyl 3-chloro-1-(2,2,2-trifluoroethyl)-1H-pyrazole-4-carboxylate (1.20 g, 4.67 mmol) in tetrahydrofuran (10 mL) were added methanol (3.0 mL), water (2.0 mL), and lithium hydroxide (0.590 g, 14.0 mmol), and the reaction liquid was stirred at 25 °C for 2 h. LCMS analysis showed that the reaction was completed. Water (100 mL) was added to the reaction liquid, the pH was adjusted to 4 with 1 M diluted hydrochloric acid, and the reaction liquid was then extracted with dichloromethane (100 mL × 3). The organic phases were combined, dried over anhydrous sodium sulfate, filtered, and concentrated to obtain a compound 3-chloro-1-(2,2,2-trifluoroethyl)-1H-pyrazole-4-carboxylic acid (600 mg, yield: 56.1%). The compound was directly used in the next step.
LC-MS, M/Z (ESI): 229.0 [M+H]⁺

### Step 3: Synthesis of 3-chloro-N-((1S,4S)-4-((1-methyl-6-oxo-5-(trifluoromethyl)-1,6-dihydropyridazin-3-yl)amino)cyclohexyl)-1-(2,2,2-trifluoroethyl)-1H-pyrazole-4-carboxamide

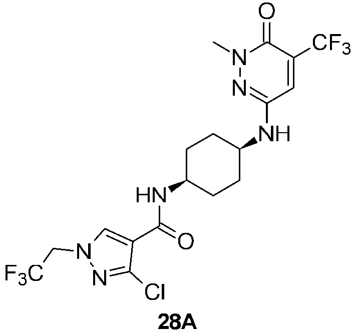

To a solution of 3-chloro-1-(2,2,2-trifluoroethyl)-1H-pyrazole-4-carboxylic acid (100 mg, 0.430 mmol) in dimethylsulfoxide (2.0 mL) were added 6-(((1S,4S)-4-aminocyclohexyl)amino)-2-methyl-4-(trifluoromethyl)pyridazin-3(2H)-one (152 mg, 0.520 mmol), 1-(3-dimethylaminopropyl)-3-ethylcarbodiimide hydrochloride (126 mg, 0.650 mmol), 1-hydroxybenzotriazole (88.7 mg, 0.650 mmol), and N,N-diisopropylethylamine (0.36 mL), and the reaction liquid was stirred at 25 °C for 1 h. LCMS analysis showed that the reaction was completed. The reaction liquid was filtered, the filtrate was added with water (50 mL) for dilution, and the resulting mixture was extracted with ethyl acetate (50 mL × 3). The organic layers were combined and washed with saturated saline (50 mL), dried over anhydrous sodium sulfate, filtered, and concentrated to obtain a crude product. The crude product was purified by preparative liquid chromatography (Xtimate C18 21.2 × 250 mm 5 µm; 0.1% FA.H₂O-ACN; 35-60; 20 mL/min) to obtain a compound 3-chloro-N-((1S,4S)-4-((1-methyl-6-oxo-5-(trifluoromethyl)-1,6-dihydropyridazin-3-yl)amino)cyclohexyl)-1-(2,2,2-trifluoroethyl)-1H-pyrazole-4-carboxamide (16.4 mg, yield: 7.5%).
LC-MS, M/Z (ESI): 501.0 [M+H]⁺
¹H NMR (400 MHz, CD₃OD): δ 8.22 (s, 1H), 7.43 (s, 1H), 4.97 (q, 2H), 4.03 - 3.95 (m, 1H), 3.81 - 3.74 (m, 1H), 3.63 (s, 3H), 1.86 - 1.76 (m, 8H).

### Example 8: Preparation of N-((1S,3S)-3-((1-methyl-6-oxo-5-(trifluoromethyl)-1,6-dihydropyridazin-3-yl)amino)cyclobutyl)-1-(2,2,2-trifluoroethyl)-1H-pyrazole-4-carboxamide (compound 29A)

The synthetic route of **compound 29A** was as follows:

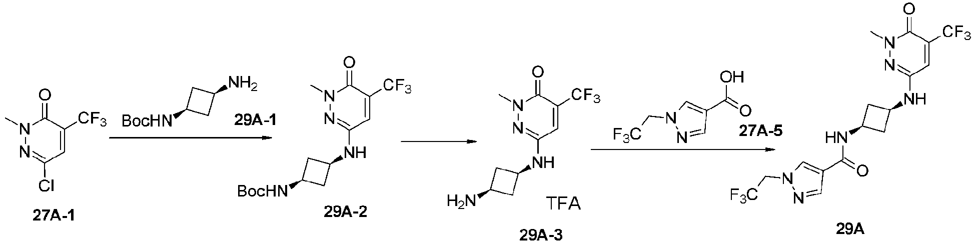

### Step 1: Synthesis of tert-butyl (1S,3S)-3-((1-methyl-6-oxo-5-(trifluoromethyl)-1,6-dihydropyridazin-3-yl)amino)cyclobutyl)carbamate

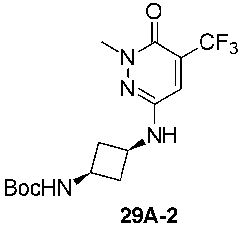

6-chloro-2-methyl-4-(trifluoromethyl)pyridazin-3(2H)-one (100 mg, 0.470 mmol) was dissolved in toluene (2 mL), and tert-butyl ((1S,3S)-3-aminocyclobutyl)carbamate (105 mg, 0.565 mmol), methanesulfonato(2-dicyclohexylphosphino-2',4',6'-triisopropyl-1,1'-biphenyl)(2'-amino-1,1'-biphenyl-2-yl)palladium(II) (40.0 mg, 0.0470 mmol), and cesium carbonate (306 mg, 0.941 mmol) were added. The reaction liquid was stirred at 80 °C for 14 h. LC-MS analysis showed that the reaction was completed. Water (50 mL) was added to the reaction system, and the reaction liquid was extracted with ethyl acetate (50 mL × 3). The organic layers were combined and washed with saturated saline (30 mL), dried over anhydrous sodium sulfate, filtered, and concentrated to obtain a crude product. The crude product was separated and purified by silica gel column chromatography (petroleum ether:ethyl acetate (V/V) = 100:0 to 80:20) to obtain a compound tert-butyl (1S,3S)-3-((1-methyl-6-oxo-5-(trifluoromethyl)-1,6-dihydropyridazin-3-yl)amino)cyclobutyl)carbamate (100 mg, yield: 58.6%).

LC-MS, M/Z (ESI): 363.4 [M+H]⁺.

### Step 2: Synthesis of 6-(((1S,3S)-3-aminocyclobutyl)amino)-2-methyl-4-(trifluoromethyl)pyridazin-3(2H)-one trifluoroacetate

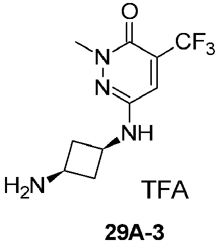

Tert-butyl (1S,3S)-3-((1-methyl-6-oxo-5-(trifluoromethyl)-1,6-dihydropyridazin-3-yl)amino)cyclobutyl)carbamate (100 mg, 0.276 mmol) was dissolved in a solvent dichloromethane (5 mL), and trifluoroacetic acid (2 mL) was added. The reaction liquid was allowed to react at 25 °C for 1.5 h. LC-MS analysis showed that the reaction was completed. The reaction liquid was concentrated under reduced pressure to obtain a crude product of compound 6-(((1S,3S)-3-aminocyclobutyl)amino)-2-methyl-4-(trifluoromethyl)pyridazin-3(2H)-one trifluoroacetate (100 mg, yield: 96.7%), which was directly used in the next step.

LC-MS, M/Z (ESI): 263.4 [M+H]⁺.

### Step 3: Synthesis of N-((1S,3S)-3-((1-methyl-6-oxo-5-(trifluoromethyl)-1,6-dihydropyridazin-3-yl)amino)cyclobutyl)-1-(2,2,2-trifluoroethyl)-1H-pyrazole-4-carboxamide

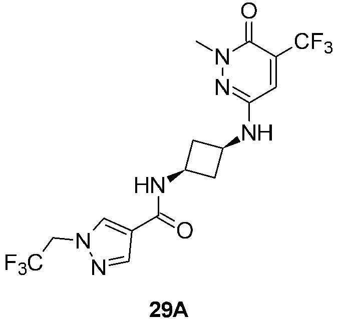

6-(((1S,3S)-3-aminocyclobutyl)amino)-2-methyl-4-(trifluoromethyl)pyridazin-3(2H)-one trifluoroacetate (70.0 mg, 0.240 mmol) and 1-(2,2,2-trifluoroethyl)-1H-pyrazole-4-carboxylic acid (46.6 mg, 0.240 mmol) were dissolved in dimethylsulfoxide (3 mL), and 1-ethyl-(3-dimethylaminopropyl)carbodiimide hydrochloride (92.1 mg, 0.480 mmol), 1-hydroxybenzotriazole (64.9 mg, 0.480 mmol), and N,N-diisopropylethylamine (155 mg, 1.20 mmol) were added. The reaction liquid was stirred at 25 °C under N₂ atmosphere for 2 h. LC-MS analysis showed that the reaction was completed. The reaction liquid was purified by preparative liquid chromatography (Xtimate C18 21.2 × 250 mm 5 µm; 0.05% NH₃.H₂O-ACN; 20-45; 20 mL/min) to obtain a compound N-((1S,3S)-3-((1-methyl-6-oxo-5-(trifluoromethyl)-1,6-dihydropyridazin-3-yl)amino)cyclobutyl)-1-(2,2,2-trifluoroethyl)-1H-pyrazole-4-carboxamide (compound 29A) (74.0 mg, yield: 70.3%).

LC-MS, M/Z (ESI): 439.2 [M+H]⁺.

¹H NMR (400 MHz, DMSO-*d₆*): δ 8.38 (d, 1H), 8.28 (s, 1H), 7.97 (s, 1H), 7.41 (s, 1H), 6.97 (d, 1H), 5.19 (q, 2H), 4.14 - 4.04 (m, 1H), 3.76 - 3.67 (m, 1H), 3.51 (s, 3H), 2.72 - 2.65 (m, 2H), 1.91 - 1.83 (m, 2H).

### Example 9: Preparation of 3-chloro-N-((1S,4S)-4-((6-oxo-1-(2,2,2-trifluoroethyl)-5-(trifluoromethyl)-1,6-dihydropyridazin-3-yl)amino)cyclohexyl)-1-(2,2,2-trifluoroethyl)-1H-pyrazole-4-carboxamide (compound 30A)

The synthetic route of **compound 30A** was as follows:

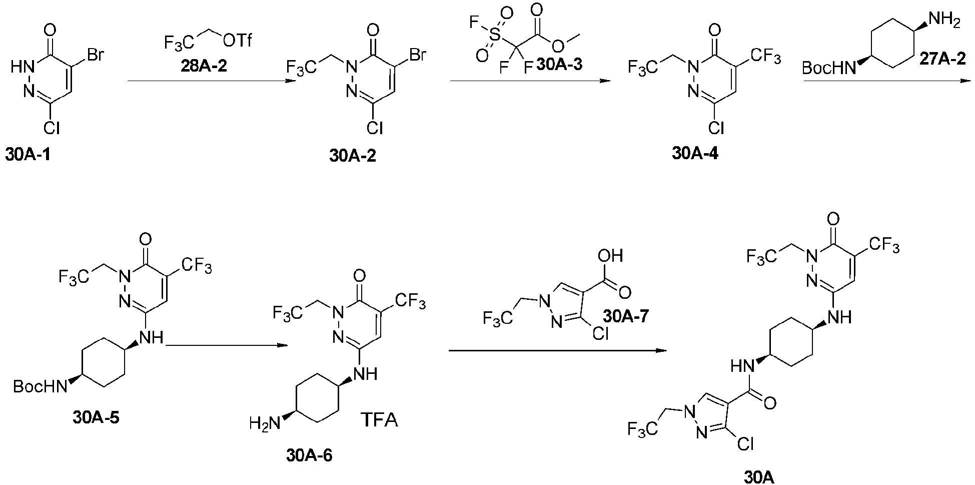

### Step 1: Synthesis of 4-bromo-6-chloro-2-(2,2,2-trifluoroethyl)pyridazin-3(2H)-one

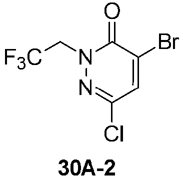

To a solution of 4-bromo-6-chloropyridazin-3(2H)-one (500 mg, 2.39 mmol) in N,N-dimethylformamide (10 mL) were added 2,2,2-trifluoroethyl trifluoromethanesulfonate (831 mg, 3.58 mmol) and potassium carbonate (989 mg, 7.16 mmol), and the resulting mixture was stirred at 25 °C for 18 h. LCMS analysis showed that the reaction was completed. The reaction liquid was added with water (100 mL) for dilution and extracted with ethyl acetate (50 mL × 3). The organic phases were combined, washed with saturated saline (40 mL), and concentrated to obtain a crude product. The crude product was purified by silica gel column chromatography (petroleum ether:ethyl acetate (V/V) = 100:0 to 80:20) to obtain a compound 4-bromo-6-chloro-2-(2,2,2-trifluoroethyl)pyridazin-3(2H)-one (500 mg, yield: 71.9%).

LC-MS, M/Z (ESI): 293.2 [M+H]⁺.

### Step 2: Synthesis of 6-chloro-2-(2,2,2-trifluoroethyl)-4-(trifluoromethyl)pyridazin-3(2H)-one

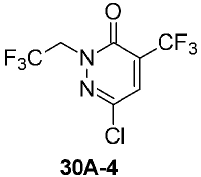

To a solution of 4-bromo-6-chloro-2-(2,2,2-trifluoroethyl)pyridazin-3(2H)-one (2.00 g, 6.862 mmol) in N-methylpyrrolidone (40 mL) were added methyl 2,2-difluoro-2-(fluorosulfonyl)acetate (3.95 g, 20.6 mmol) and copper(I) iodide (261 mg, 1.37 mmol), and the resulting mixture was allowed to react at 25 °C for 14 h. LCMS analysis showed that the reaction was completed. The reaction liquid was added with water (50 mL) for dilution, and then extracted with ethyl acetate (100 mL × 3). The organic layers were combined, washed with saturated saline (100 mL), and concentrated to obtain a crude product. The crude product was purified by silica gel column chromatography (petroleum ether:ethyl acetate (V/V) = 100:0 to 90:10) to obtain a compound 6-chloro-2-(2,2,2-trifluoroethyl)-4-(trifluoromethyl)pyridazin-3(2H)-one (1.00 g, yield: 51.9%).

LC-MS, M/Z (ESI):281.0 [M+H]⁺.

### Step 3: Synthesis of tert-butyl (1S,4S)-4-((6-oxo-1-(2,2,2-trifluoroethyl)-5-(trifluoromethyl)-1,6-dihydropyridazin-3-yl)amino)cyclohexyl)carbamate

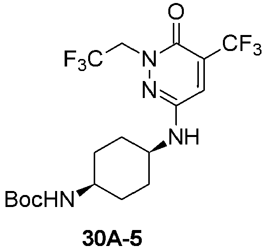

To a solution of tert-butyl ((1S,4S)-4-aminocyclohexyl)carbamate (382 mg, 1.78 mmol) in 1,4-dioxane (10 mL) were added 6-chloro-2-(2,2,2-trifluoroethyl)-4-(trifluoromethyl)pyridazin-3(2H)-one (500 mg, 1.78 mmol), Ruphos Pd G4 (1.55 g, 1.82 mmol), and cesium carbonate (1.74 g, 5.35 mmol), and the resulting mixture was stirred at 80 °C for 18 h. LCMS analysis showed that the reaction was completed. The reaction liquid was added with water (100 mL) for dilution and extracted with ethyl acetate (100 mL × 3). The organic phases were combined, washed with saturated saline (100 mL), and concentrated to obtain a crude product. The crude product was purified by silica gel column chromatography (petroleum ether:ethyl acetate (V/V) = 100:0 to 75:25) to obtain a compound tert-butyl (1S,4S)-4-((6-oxo-1-(2,2,2-trifluoroethyl)-5-(trifluoromethyl)-1,6-dihydropyridazin-3-yl)amino)cyclohexyl)carbamate (200 mg, yield: 24.5%).

LC-MS, M/Z (ESI):459.2 [M+H]⁺.

### Step 4: Synthesis of 6-(((1S,4S)-4-aminocyclohexyl)amino)-2-(2,2,2-trifluoroethyl)-4-(trifluoromethyl)pyridazin-3(2H)-one trifluoroacetate

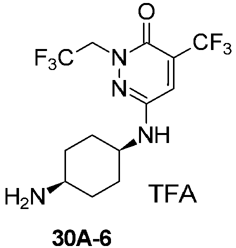

To a solution of tert-butyl (1S,4S)-4-((6-oxo-1-(2,2,2-trifluoroethyl)-5-(trifluoromethyl)-1,6-dihydropyridazin-3-yl)amino)cyclohexyl)carbamate (100 mg, 0.218 mmol) in dichloromethane (5 mL) was added trifluoroacetic acid (2 mL, 0.218 mmol), and the resulting mixture was stirred at 25 °C for 3 h. LCMS analysis showed that the reaction was completed. The reaction liquid was concentrated under reduced pressure to obtain a compound 6-(((1S,4S)-4-aminocyclohexyl)amino)-2-(2,2,2-trifluoroethyl)-4-(trifluoromethyl)pyridazin-3(2H)-one trifluoroacetate (100 mg, crude product), which was directly used in the next step.

LC-MS, M/Z (ESI):359.0 [M+H]⁺.

### Step 5: Synthesis of 3-chloro-N-((1S,4S)-4-((6-oxo-1-(2,2,2-trifluoroethyl)-5-(trifluoromethyl)-1,6-dihydropyridazin-3-yl)amino)cyclohexyl)-1-(2,2,2-trifluoroethyl)-1H-pyrazole-4-carboxamide (compound 30A)

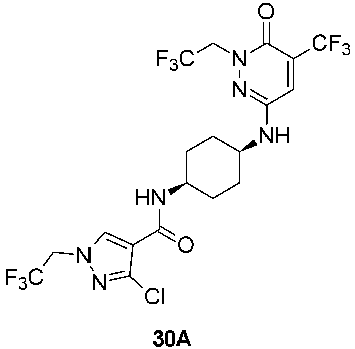

To a solution of 6-(((1S,4S)-4-aminocyclohexyl)amino)-2-(2,2,2-trifluoroethyl)-4-(trifluoromethyl)pyridazin-3(2H)-one trifluoroacetate (80.0 mg, 0.167 mmol) and 3-chloro-1-(2,2,2-trifluoroethyl)-1H-pyrazole-4-carboxylic acid (38.3 mg, 0.167 mmol) in dimethylsulfoxide (2 mL) were added N,N-diisopropylethylamine (0.138 mL, 0.835 mmol), 1-(3-dimethylaminopropyl)-3-ethylcarbodiimide hydrochloride (48.2 mg, 0.251 mmol), and 1-hydroxybenzotriazole (33.9 mg, 0.251 mmol), and the resulting mixture was stirred at 25 °C for 14 h. LCMS analysis showed that the reaction was completed. The reaction liquid was purified by preparative liquid chromatography (Xtimate C18 21.2 × 250 mm 5 µm; 0.05% NH₃.H₂O-ACN; 20-45; 20 mL/min) to obtain a compound 3-chloro-N-((1S,4S)-4-((6-oxo-1-(2,2,2-trifluoroethyl)-5-(trifluoromethyl)-1,6-dihydropyridazin-3-yl)amino)cyclohexyl)-1-(2,2,2-trifluoroethyl)-1H-pyrazole-4-carboxamide (compound 30A) (37.0 mg, yield: 38.8%).

LC-MS, M/Z (ESI): 569.2 [M+H]⁺.

¹H NMR (400 MHz, DMSO-*d₆*): δ 8.37 (s, 1H), 7.87 (d, 1H), 7.65 (s, 1H), 6.82 (d, 1H), 5.20 (q, 2H), 4.75 (q, 2H), 3.85 - 3.74 (m, 1H), 3.64 - 3.55 (m, 1H), 1.85 - 1.75 (m, 2H), 1.72 - 1.60 (m, 6H).

### Example 10: Preparation of 3-chloro-1-(2,2-difluoroethyl)-N-((1S,4S)-4-((1-methyl-6-oxo-5-(trifluoromethyl)-1,6-dihydropyridazin-3-yl)amino)cyclohexyl)-1H-pyrazole-4-carboxamide (compound 34A)

The synthetic route of **compound 34A** was as follows:

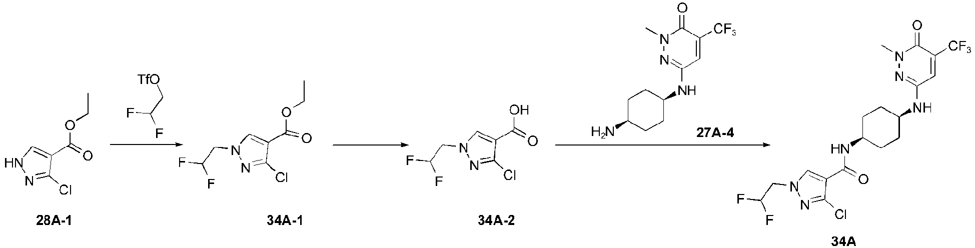

### Step 1: Synthesis of ethyl 3-chloro-1-(2,2-difluoroethyl)-1H-pyrazole-4-carboxylate

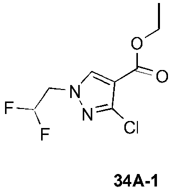

To a solution of ethyl 3-chloro-1H-pyrazole-4-carboxylate (1.00 g, 5.30 mmol) in N,N-dimethylformamide (10 mL) were added 2,2-difluoroethyl trifluoromethanesulfonate (2.45 g, 11.5 mmol) and cesium carbonate (3.73 g, 11.5 mmol), and the resulting mixture was stirred at 25 °C for 18 h. LCMS analysis showed that the reaction was completed. The reaction liquid was added with water (100 mL) for dilution, and then extracted with ethyl acetate (100 mL × 3). The organic layers were combined and concentrated under reduced pressure to obtain a crude product. The crude product was purified by silica gel column chromatography (petroleum ether:ethyl acetate (V/V) = 100:0 to 80:20) to obtain a compound ethyl 3-chloro-1-(2,2-difluoroethyl)-1H-pyrazole-4-carboxylate (500 mg, yield: 36.6%).

LC-MS, M/Z (ESI): 239.0 [M+H]⁺

### Step 2: Synthesis of 3-chloro-1-(2,2-difluoroethyl)-1H-pyrazole-4-carboxylic acid

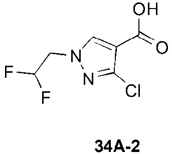

To a mixed solution of ethyl 3-chloro-1-(2,2-difluoroethyl)-1H-pyrazole-4-carboxylate (500 mg, 2.09 mmol) in tetrahydrofuran (5 mL), methanol (2 mL), and water (1 mL) was added lithium hydroxide (264 mg, 6.29 mmol), and the resulting mixture was stirred at 25 °C for 2 h. LCMS analysis showed that the reaction was completed. The reaction liquid was diluted with water (100 mL), and the pH was then adjusted to 4 using 1 M hydrochloric acid. The resulting mixture was extracted with dichloromethane (100 mL × 3). The organic layers were combined, dried over anhydrous sodium sulfate, filtered, and concentrated to obtain a compound 3-chloro-1-(2,2-difluoroethyl)-1H-pyrazole-4-carboxylic acid (420 mg, yield: 95.2%), which was directly used in the next step.

LC-MS, M/Z (ESI): 211.0 [M+H]⁺

### Step 3: Synthesis of 3-chloro-1-(2,2-difluoroethyl)-N-(cis-4-((1-methyl-6-oxo-5-(trifluoromethyl)-1,6-dihydropyridazin-3-yl)amino)cyclohexyl)-1H-pyrazole-4-carboxamide

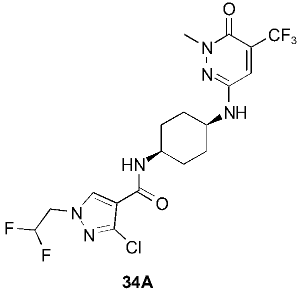

To a solution of 3-chloro-1-(2,2-difluoroethyl)-1H-pyrazole-4-carboxylic acid (150 mg, 0.712 mmol) in dimethylsulfoxide (2 mL) were added 6-(((1S,4S)-4-aminocyclohexyl)amino)-2-methyl-4-(trifluoromethyl)pyridazin-3(2H)-one (207 mg, 0.712 mmol), 1-(3-dimethylaminopropyl)-3-ethylcarbodiimide hydrochloride (205 mg, 1.069 mmol), 1-hydroxybenzotriazole (144 mg, 1.07 mmol), and N,N-diisopropylethylamine (0.588 mL, 3.56 mmol), and the reaction liquid was allowed to react at 25 °C for 1 h. LCMS analysis showed that the reaction was completed. The reaction liquid was diluted with saturated saline (50 mL), and then extracted with ethyl acetate (50 mL × 3). The organic phases were combined and concentrated to obtain a crude product. The crude product was purified by preparative liquid chromatography (Xtimate C18 21.2 × 550 mm 5 µm; 0.1% FA.H₂O-ACN; 30-55; 20 mL/min) to obtain a compound 3-chloro-1-(2,2-difluoroethyl)-N-((1S,4S)-4-((1-methyl-6-oxo-5-(trifluoromethyl)-1,6-dihydropyridazin-3-yl)amino)cyclohexyl)-1H-pyrazole-4-carboxamide (59.8 mg, yield: 17.2%).
LC-MS, M/Z (ESI): 483.4 [M+H]⁺
¹H NMR (400 MHz, CD3OD): δ 8.15 (s, 1H), 7.42 (s, 1H), 6.20 (tt, 1H), 4.60 - 4.58(m, 2H), 4.01 - 3.94 (m, 1H), 3.80 - 3.74 (m, 1H), 3.62 (s, 3H), 1.87 - 1.74 (m, 8H).

### Example 11: Preparation of 3-chloro-N-((1S,4S)-4-((1-methyl-6-oxo-5-(trifluoromethyl)-1,6-dihydropyridin-3-yl)amino)cyclohexyl)-1-(2,2,2-trifluoroethyl)-1H-pyrazole-4-carboxamide (compound 39A)

The synthetic route of **compound 39A** was as follows:

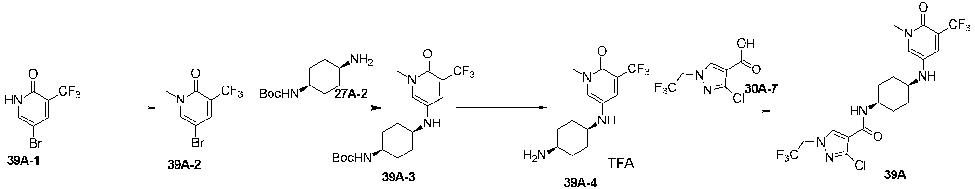

### Step 1: Synthesis of 5-bromo-1-methyl-3-(trifluoromethyl)pyridin-2(1H)-one

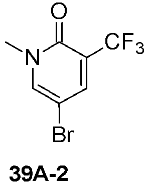

To a solution of 5-bromo-3-(trifluoromethyl)pyridin-2(1H)-one (5.00 g, 20.7 mmol) in N,N-dimethylformamide (50 mL) were added potassium carbonate (14.3 g, 103 mmol) and iodomethane (3.52 g, 24.8 mmol), and the resulting mixture was stirred at 25 °C for 2 h. LCMS analysis showed that the reaction was completed. The reaction liquid was added with water (50 mL) for dilution and extracted with ethyl acetate (50 mL × 3). The organic phases were combined, washed with saturated saline (40 mL), and concentrated under reduced pressure to obtain a crude product. The crude product was purified by silica gel column chromatography (petroleum ether:ethyl acetate (V/V) = 100:0 to 90:10) to obtain a compound 5-bromo-1-methyl-3-(trifluoromethyl)pyridin-2(1H)-one (4.00 g, yield: 75.6%).

LC-MS, M/Z (ESI): 256.0 [M+H]⁺.

### Step 2: Synthesis of tert-butyl (1S,4S)-4-((1-methyl-6-oxo-5-(trifluoromethyl)-1,6-dihydropyridin-3-yl)amino)cyclohexyl)carbamate

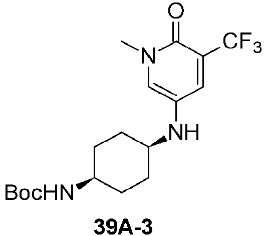

To a solution of 5-bromo-1-methyl-3-(trifluoromethyl)pyridin-2(1H)-one (200 mg, 0.781 mmol) and tert-butyl ((1S,4S)-4-aminocyclohexyl)carbamate (251 mg, 1.17 mmol) in N,N-dimethylformamide (10 mL) were added sodium tert-butoxide (225 mg, 2.34 mmol) and methanesulfonato(2-di-tert-butylphosphino-2',4',6'-triisopropyl-1,1'-biphenyl)(2'-amino-1,1'-biphenyl-2-yl)palladium(II) (62.1 mg, 0.078 mmol), and the resulting mixture was stirred at 120 °C for 18 h. LCMS analysis showed that the reaction was completed. The reaction liquid was added with water (10 mL) for dilution, and then extracted with ethyl acetate (10 mL × 3). The organic layers were combined, washed with saturated saline (10 mL) and concentrated under reduced pressure to obtain a crude product. The crude product was purified by silica gel column chromatography (dichloromethane:methanol (V/V) = 100:0 to 95:5) to obtain a compound tert-butyl (1S,4S)-4-((1-methyl-6-oxo-5-(trifluoromethyl)-1,6-dihydropyridin-3-yl)amino)cyclohexyl)carbamate (50.0 mg, yield: 16.4%).

LC-MS, M/Z (ESI): 390.2 [M+H]⁺.

### Step 3: Synthesis of 5-(((1S,4S)-4-aminocyclohexyl)amino)-1-methyl-3-(trifluoromethyl)pyridin-2(1H)-one trifluoroacetate

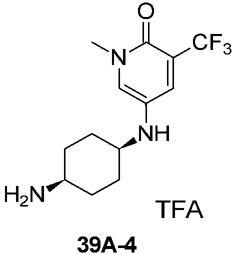

To a solution of tert-butyl (1S,4S)-4-((1-methyl-6-oxo-5-(trifluoromethyl)-1,6-dihydropyridin-3-yl)amino)cyclohexyl)carbamate (20.0 mg, 0.051 mmol) in dichloromethane (2 mL) was added trifluoroacetic acid (0.5 mL, 0.051 mmol), and the resulting mixture was stirred at 25 °C for 1 h. LCMS analysis showed that the reaction was completed. The reaction liquid was concentrated under reduced pressure to obtain a compound 5-(((1S,4S)-4-aminocyclohexyl)amino)-1-methyl-3-(trifluoromethyl)pyridin-2(1H)-one trifluoroacetate (20.0 mg, crude product), which was directly used in the next step.

LC-MS, M/Z (ESI):290.2 [M+H]⁺.

### Step 4: Synthesis of 3-chloro-N-((1S,4S)-4-((1-methyl-6-oxo-5-(trifluoromethyl)-1,6-dihydropyridin-3-yl)amino)cyclohexyl)-1-(2,2,2-trifluoroethyl)-1H-pyrazole-4-carboxamide (compound 39A)

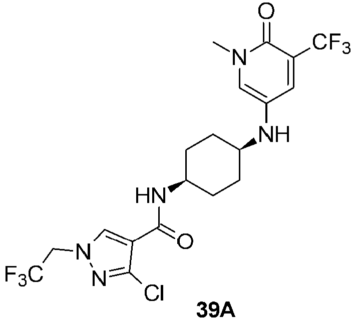

To a solution of 5-(((1S,4S)-4-aminocyclohexyl)amino)-1-methyl-3-(trifluoromethyl)pyridin-2(1H)-one trifluoroacetate (20.0 mg, 0.069 mmol) and 3-chloro-1-(2,2,2-trifluoroethyl)-1H-pyrazole-4-carboxylic acid (15.8 mg, 0.069 mmol) in dimethylsulfoxide (3 mL) were added 1-(3-dimethylaminopropyl)-3-ethylcarbodiimide hydrochloride (26.5 mg, 0.138 mmol), 1-hydroxybenzotriazole (18.7 mg, 0.138 mmol), and N,N-diisopropylethylamine (0.057 mL, 0.346 mmol), and the resulting mixture was allowed to react at 25 °C for 18 h. LCMS analysis showed that the reaction was completed. The reaction liquid was concentrated under reduced pressure, and the residue was purified by preparative liquid chromatography (Xtimate C18 21.2 × 250 mm 5 µm; 0.05% NH₃.H₂O-ACN; 15-35; 25 mL/min) to obtain a compound 3-chloro-N-((1S,4S)-4-((1-methyl-6-oxo-5-(trifluoromethyl)-1,6-dihydropyridin-3-yl)amino)cyclohexyl)-1-(2,2,2-trifluoroethyl)-1H-pyrazole-4-carboxamide (compound 39A) (7.60 mg, yield: 21.4%).

LC-MS, M/Z (ESI): 500.2 [M+H]⁺.

¹H NMR (400 MHz, DMSO-*d₆*): δ 8.39 (s, 1H), 7.81 (d, 1H), 7.68 (d, 1H), 7.15 (d, 1H), 5.20 (q, 2H), 4.93 (d, 1H), 3.93 - 3.70 (m, 1H), 3.44 (s, 3H), 3.20 - 3.13 (m, 1H), 1.75 - 1.56 (m, 8H).

### Example 12: Preparation of 3-chloro-N-((1S,4S)-4-((1-methyl-6-oxo-5-(trifluoromethyl)-1,6-dihydropyridazin-3-yl)amino)cyclohexyl)-1-(1-(methylsulfonyl)ethyl)-1H-pyrazole-4-carboxamide (compound 42A)

The synthetic route of **compound 42A** was as follows:

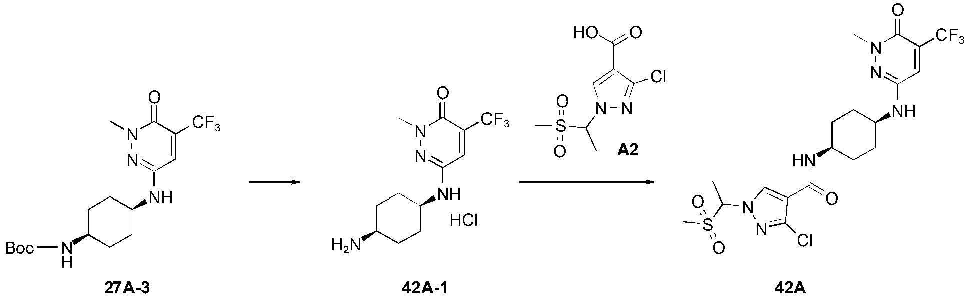

### Step 1: Synthesis of 6-(((1S,4S)-4-aminocyclohexyl)amino)-2-methyl-4-(trifluoromethyl)pyridazin-3(2H)-one hydrochloride

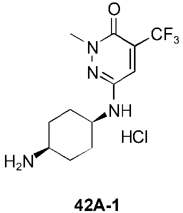

At room temperature, tert-butyl (1S,4S)-4-((1-methyl-6-oxo-5-(trifluoromethyl)-1,6-dihydropyridazin-3-yl)amino)cyclohexyl)carbamate (100 mg, 0.26 mmol) was dissolved in dichloromethane (2 mL), and the mixture was stirred in an ice bath at 0 °C for 10 min. Subsequently, a solution of hydrogen chloride in 1,4-dioxane (0.13 mL, 0.13 mmol) was slowly added dropwise. After the dropwise addition was completed, the reaction liquid was naturally warmed to room temperature, and successively stirred at room temperature for 1 h. After the reaction of the starting material was completed as monitored by TLC, stirring was stopped. The reaction liquid was concentrated under reduced pressure to obtain a compound 6-(((1S,4S)-4-aminocyclohexyl)amino)-2-methyl-4-(trifluoromethyl)pyridazin-3(2H)-one hydrochloride (100 mg, crude product), which was directly used in the next step.
LC-MS,M/Z(ESI):291.1 [M+H]⁺

### Step 2: Synthesis of 3-chloro-N-((1S,4S)-4-((1-methyl-6-oxo-5-(trifluoromethyl)-1,6-dihydropyridazin-3-yl)amino)cyclohexyl)-1(1-(methylsulfonyl)ethyl)-1H-pyrazole-4-carboxamide

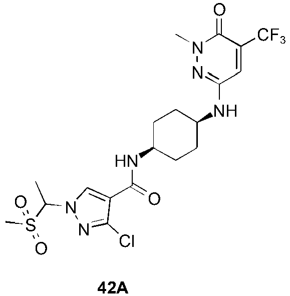

At room temperature, a compound 3-chloro-1-(1-(methylsulfonyl)ethyl)-1H-pyrazole-4-carboxylic acid (32.00 mg, 0.13 mmol) was dissolved in anhydrous N,N-dimethylformamide (1 mL), and N,N-diisopropylethylamine (65.47 mg, 0.50 mmol) and O-(7-azabenzotriazol-1-yl)-N,N,N',N'-tetramethyluronium hexafluorophosphate (96.98 mg, 0.25 mmol) were then added. The mixture was stirred at room temperature for 30 min, and 6-(((1S,4S)-4-aminocyclohexyl)amino)-2-methyl-4-(trifluoromethyl)pyridazin-3(2H)-one hydrochloride (50.00 mg, 0.15 mmol) was then added. The resulting mixture was successively stirred at room temperature for 4 h. After the reaction of the starting material was completed as monitored by TLC, stirring was stopped. The reaction liquid was cooled to room temperature, then added with 10 mL of water for dilution, and extracted with ethyl acetate (5 mL × 3). The organic phase was collected, dried over anhydrous sodium sulfate, and concentrated under reduced pressure. The residue was separated and purified by silica gel column chromatography (petroleum ether:ethyl acetate (V/V) = 100:20) to obtain a compound 3-chloro-N-((1S,4S)-4-((1-methyl-6-oxo-5-(trifluoromethyl)-1,6-dihydropyridazin-3-yl)amino)cyclohexyl)-1-(1-(methylsulfonyl)ethyl)-1H-pyrazole-4-carboxamide.
LC-MS,M/Z(ESI):525.0 [M+H]⁺
¹H NMR (400 MHz, DMSO-d6): δ 8.52 (s, 1H), 7.87 (d, 1H), 7.54 (s, 1H), 6.62 (d, 1H), 5.92(q, 1H), 3.85 - 3.76 (m, 1H), 3.62 (s, 1H), 3.50 (s, 3H), 2.99 (s, 3H), 1.83 (d, J = 7.1 Hz, 3H), 1.75 (s, 2H), 1.65 (d, 6H).

### Example 13: Preparation of 3-chloro-1-(2-hydroxy-2-methylpropyl)-N-((1S,4S)-4-((1-methyl-6-oxo-5-(trifluoromethyl)-1,6-dihydropyridazin-3-yl)amino)cyclohexyl)-1H-pyrazole-4-carboxamide (compound 48A)

The synthetic route of **compound 48A** was as follows:

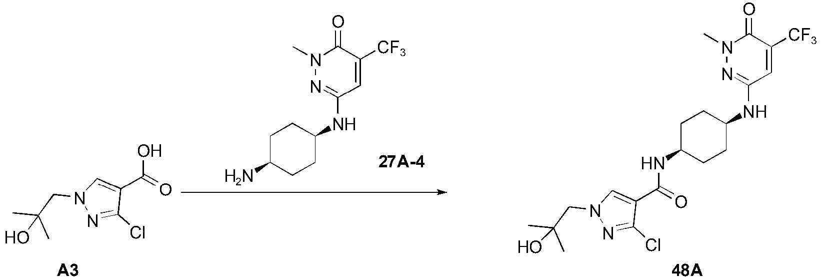

To a solution of 3-chloro-1-(2-hydroxy-2-methylpropyl)-1H-pyrazole-4-carboxylic acid (200 mg, 0.915 mmol) in dimethylsulfoxide (2 mL) were added 6-(((1S,4S)-4-aminocyclohexyl)amino)-2-methyl-4-(trifluoromethyl)pyridazin-3(2H)-one (266 mg, 0.915 mmol), 1-(3-dimethylaminopropyl)-3-ethylcarbodiimide hydrochloride (263 mg, 1.37 mmol), 1-hydroxybenzotriazole (185 mg, 1.37 mmol), and N,N-diisopropylethylamine (0.756 mL, 4.58 mmol), and the reaction liquid was allowed to react at 25 °C for 1 h. LCMS analysis showed that the reaction was completed. The reaction liquid was diluted with saturated saline (50 mL), and then extracted with ethyl acetate (50 mL × 3). The organic phases were combined and concentrated to obtain a crude product. The crude product was purified by preparative liquid chromatography (Xtimate C18 21.2 × 250 mm 5 µm; 0.1% FA.H₂O-ACN; 25-55; 20 mL/min) to obtain a compound 3-chloro-1-(2-hydroxy-2-methylpropyl)-N-((1S,4S)-4-((1-methyl-6-oxo-5-(trifluoromethyl)-1,6-dihydropyridazin-3-yl)amino)cyclohexyl)-1H-pyrazole-4-carboxamide (115 mg, yield: 25.6%).
LC-MS, M/Z (ESI): 491.2 [M+H]⁺
¹H NMR (400 MHz, CD₃OD): δ 8.08 (s, 1H), 7.43 (s, 1H), 4.06 (s, 2H), 4.02 - 3.97 (m, 1H), 3.80 - 3.75 (m, 1H), 3.63 (s, 3H), 1.86 - 1.76 (m, 8H), 1.19 - 1.17 (m, 6H).

### Example 14: Preparation of 3-chloro-1-methyl-N-((1S,4S)-4-((1-methyl-6-oxo-5-(trifluoromethyl)-1,6-dihydropyridazin-3-yl)amino)cyclohexyl)-1H-pyrazole-4-carboxamide (compound 49A)

The synthetic route of **compound 49A** was as follows:

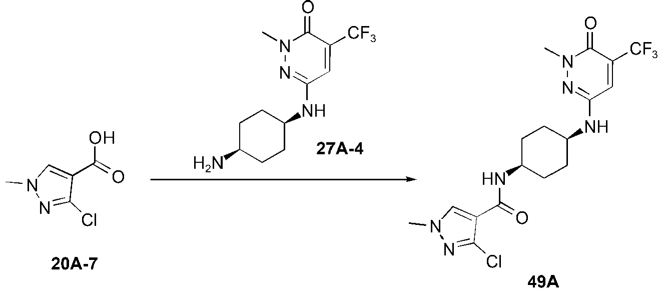

To a solution of 6-(((1S,4S)-4-aminocyclohexyl)amino)-2-methyl-4-(trifluoromethyl)pyridazin-3(2H)-one (200 mg, 0.689 mmol) in dimethylsulfoxide (2 mL) were added 3-chloro-1-methyl-1H-pyrazole-4-carboxylic acid (111 mg, 0.689 mmol), 1-(3-dimethylaminopropyl)-3-ethylcarbodiimide hydrochloride (198 mg, 1.03 mmol), 1-hydroxybenzotriazole (140 mg, 1.03 mmol), and N,N-diisopropylethylamine (0.569 mL, 3.45 mmol), and the resulting mixture was stirred at 25 °C for 1 h. LCMS analysis showed that the reaction was completed. The reaction liquid was diluted with saturated saline (50 mL), and then extracted with ethyl acetate (50 mL × 3). The organic phases were combined and concentrated to obtain a crude product. The crude product was purified by preparative liquid chromatography (Xtimate C18 30 × 150 mm 5 µm; 0.1% FA.H₂O-ACN; 28-48; 25 mL/min) to obtain a compound 3-chloro-1-methyl-N-((1S,4S)-4-((1-methyl-6-oxo-5-(trifluoromethyl)-1,6-dihydropyridazin-3-yl)amino)cyclohexyl)-1H-pyrazole-4-carboxamide (16.7 mg, yield: 2.6%).
LC-MS, M/Z (ESI): 433.2 [M+H]⁺
¹H NMR (400 MHz, CD₃OD): δ 8.04 (s, 1H), 7.43 (s, 1H), 4.02 - 3.95 (m, 1H), 3.87 (s, 3H), 3.81 - 3.74 (m, 1H), 3.63 (s, 3H), 1.87 - 1.73 (m, 8H).

### Example 15: Preparation of 4-fluoro-N-((1S,4S)-4-((1-methyl-6-oxo-5-(trifluoromethyl)-1,6-dihydropyridazin-3-yl)amino)cyclohexyl)benzamide (compound 50A)

The synthetic route of **compound 50A** was as follows:

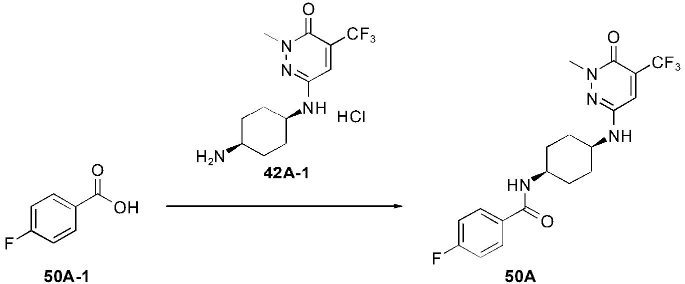

At room temperature, a compound p-fluorobenzoic acid (18.00 mg, 0.13 mmol) was dissolved in anhydrous N,N-dimethylformamide (1 mL), and N,N-diisopropylethylamine (65.47 mg, 0.50 mmol) and O-(7-azabenzotriazol-1-yl)-N,N,N',N'-tetramethyluronium hexafluorophosphate (96.98 mg, 0.25 mmol) were then added. The reaction liquid was stirred at room temperature for 30 min, and 6-(((1S,4S)-4-aminocyclohexyl)amino)-2-methyl-4-(trifluoromethyl)pyridazin-3(2H)-one hydrochloride (50.00 mg, 0.15 mmol) was then added. The reaction liquid was successively stirred at room temperature for 4 h. After the reaction of the starting material was completed as monitored by TLC, stirring was stopped. The reaction liquid was cooled to room temperature, then added with 10 mL of water for dilution, and extracted with ethyl acetate (5 mL × 3). The organic phase was collected, dried over anhydrous sodium sulfate, and concentrated by distillation under reduced pressure. The residue was separated and purified by silica gel column chromatography (petroleum ether:ethyl acetate (V/V) = 100:15) to obtain a compound 4-fluoro-N-((1S,4S)-4-((1-methyl-6-oxo-5-(trifluoromethyl)-1,6-dihydropyridazin-3-yl)amino)cyclohexyl)benzamide.
LC-MS,M/Z(ESI):413.0 [M+H]⁺
¹H NMR (400 MHz, DMSO-d6): δ 8.26 (d, 1H), 7.98 - 7.87 (m, 2H), 7.57 (s, 1H), 7.33-.23 (m, 2H), 6.62 (d, 1H), 3.86-.80 (m, 1H), 3.64 (s, 1H), 3.51 (s, 3H), 1.87 (dd, 2H), 1.73-1.61 (m, 6H).

### Example 16: Preparation of 3-chloro-N-((1S,4S)-4-((1-cyclopropyl-6-oxo-5-(trifluoromethyl)-1,6-dihydropyridazin-3-yl)amino)cyclohexyl)-1-(2,2,2-trifluoroethyl)-1H-pyrazole-4-carboxamide (compound 31A)

The synthetic route of **compound 31A** was as follows:

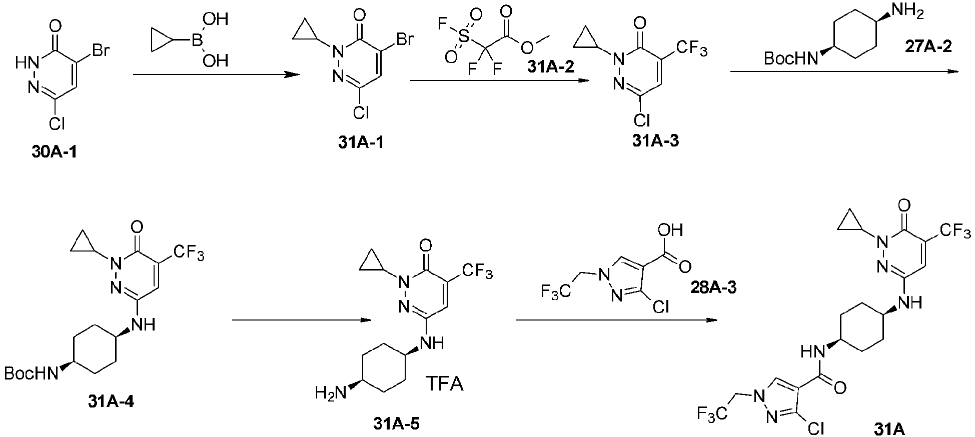

### Step 1: Synthesis of 4-bromo-6-chloro-2-cyclopropylpyridazin-3(2H)-one

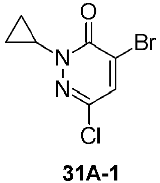

To a solution of 4-bromo-6-chloropyridazin-3(2H)-one (7.50 g, 35.8 mmol) in dioxane (60 mL) were added pyridine (23.1 mL, 286 mmol), triethylamine (24.9 mL, 179 mmol), cyclopropylboronic acid (9.23 g, 107 mmol), and copper(II) acetate (6.50 g, 35.8 mmol), and the resulting reaction liquid was stirred at 80 °C under nitrogen atmosphere for 30 min. LCMS analysis showed that the reaction was completed. The reaction liquid was cooled to room temperature, added with water (50 mL) for dilution, and then extracted with ethyl acetate (50 mL × 3). The organic phases were combined and concentrated to obtain a crude product. The crude product was purified by silica gel column chromatography (petroleum ether:ethyl acetate (V/V) = 100:0 to 80:20) to obtain a compound 4-bromo-6-chloro-2-cyclopropylpyridazin-3(2H)-one (1.40 g, yield: 15.7%).
LC-MS, M/Z (ESI):249.0 [M+H]⁺

### Step 2: Synthesis of 6-chloro-2-cyclopropyl-4-(trifluoromethyl)pyridazin-3(2H)-one

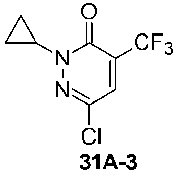

To a solution of 4-bromo-6-chloro-2-cyclopropylpyridazin-3(2H)-one (1.40 g, 5.61 mmol) in N-methylpyrrolidone (15 mL) were added copper(I) iodide (534 mg, 2.81 mmol) and methyl fluorosulfonyldifluoroacetate (5.39 g, 28.1 mmol), and the resulting reaction liquid was stirred at 100 °C under nitrogen atmosphere for 14 h. LCMS analysis showed that the reaction was completed. The reaction liquid was cooled to room temperature, added with water (50 mL) for dilution, and then extracted with ethyl acetate (50 mL × 3). The organic phases were combined and concentrated under reduced pressure to obtain a crude product. The crude product was purified by silica gel column chromatography (petroleum ether:ethyl acetate (V/V) = 100:0 to 90:10) to obtain a compound 6-chloro-2-cyclopropyl-4-(trifluoromethyl)pyridazin-3(2H)-one (1.20 g, yield: 89.6%).
LC-MS, M/Z (ESI): 239.0 [M+H]⁺

### Step 3: Synthesis of tert-butyl (1S,4S)-4-((1-cyclopropyl-6-oxo-5-(trifluoromethyl)-1,6-dihydropyridazin-3-yl)amino)cyclohexyl)carbamate

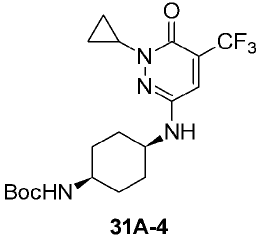

To a solution of 6-chloro-2-cyclopropyl-4-(trifluoromethyl)pyridazin-3(2H)-one (1.20 g, 5.03 mmol) in 1,4-dioxane (15 mL) were added tert-butyl ((1S,4S)-4-aminocyclohexyl)carbamate (2.16 g, 10.1 mmol), methanesulfonato(2-dicyclohexylphosphino-2',4',6'-triisopropyl-1,1'-biphenyl)(2'-amino-1,1'-biphenyl-2-yl)palladium(II) (428 mg, 0.503 mmol), and cesium carbonate (4.92 g, 15.1 mmol), and the resulting reaction liquid was stirred at 80 °C under nitrogen atmosphere for 18 h. LCMS analysis showed that the reaction was completed. The reaction liquid was cooled to room temperature, added with water (100 mL) for dilution, and then extracted with ethyl acetate (50 mL × 3). The organic layers were combined and concentrated under reduced pressure to obtain a crude product. The crude product was purified by silica gel column chromatography (petroleum ether:ethyl acetate (V/V) = 100:0 to 80:20) to obtain a compound tert-butyl (1S,4S)-4-((1-cyclopropyl-6-oxo-5-(trifluoromethyl)-1,6-dihydropyridazin-3-yl)amino)cyclohexyl)carbamate (1.50 g, yield: 71.6%).
LC-MS, M/Z (ESI): 417.4 [M+H]⁺

### Step 4: Synthesis of 6-(((1S,4S)-4-aminocyclohexyl)amino)-2-cyclopropyl-4-(trifluoromethyl)pyridazin-3(2H)-one trifluoroacetate

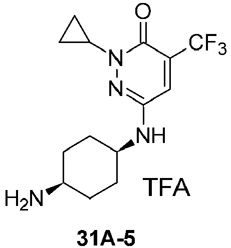

To a solution of tert-butyl (1S,4S)-4-((1-cyclopropyl-6-oxo-5-(trifluoromethyl)-1,6-dihydropyridazin-3-yl)amino)cyclohexyl)carbamate (500 mg, 1.20 mmol) in dichloromethane (5 mL) was added trifluoroacetic acid (2 mL), and the resulting mixture was stirred at 25 °C for 14 h. LCMS analysis showed that the reaction was completed. The reaction liquid was directly concentrated under reduced pressure to obtain a compound 6-(((1S,4S)-4-aminocyclohexyl)amino)-2-cyclopropyl-4-(trifluoromethyl)pyridazin-3(2H)-one trifluoroacetate (500 mg, crude product), which was directly used in the next step.
LC-MS, M/Z (ESI): 317.2 [M+H]⁺

### Step 5: Synthesis of 3-chloro-N-((1S,4S)-4-((1-cyclopropyl-6-oxo-5-(trifluoromethyl)-1,6-dihydropyridazin-3-yl)amino)cyclohexyl)-1-(2,2,2-trifluoroethyl)-1H-pyrazole-4-carboxamide

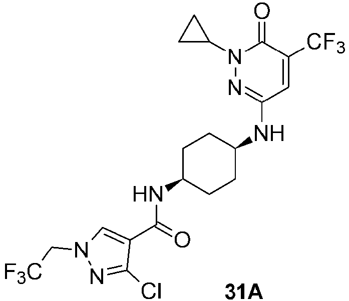

To a solution of 6-(((1S,4S)-4-aminocyclohexyl)amino)-2-cyclopropyl-4-(trifluoromethyl)pyridazin-3(2H)-one trifluoroacetate (500 mg, 1.11 mmol) and 3-chloro-1-(2,2,2-trifluoroethyl)-1H-pyrazole-4-carboxylic acid (253 mg, 1.11 mmol) in dimethylsulfoxide (10 mL) were added N,N-diisopropylethylamine (0.914 mL, 5.53 mmol), 1-(3-dimethylaminopropyl)-3-ethylcarbodiimide hydrochloride (318 mg, 1.66 mmol), and 1-hydroxybenzotriazole (224 mg, 1.66 mmol), and the resulting mixture was stirred at 25 °C for 14 h. LCMS analysis showed that the reaction was completed. The reaction liquid was purified by preparative liquid chromatography (Xtimate C18 21.2 × 550 mm 5 µm; 0.1% FA.H₂O-ACN; 30-60; 20 mL/min) to obtain a compound 3-chloro-N-((1S,4S)-4-((1-cyclopropyl-6-oxo-5-(trifluoromethyl)-1,6-dihydropyridazin-3-yl)amino)cyclohexyl)-1-(2,2,2-trifluoroethyl)-1H-pyrazole-4-carboxamide (350 mg, yield: 60.0%).
LC-MS, M/Z (ESI): 527.2 [M+H]⁺
¹H NMR (400 MHz, CD3OD): δ 8.21 (s, 1H), 7.38 (s, 1H), 4.96 (q, 2H), 4.13 - 4.04 (m, 1H), 4.01 - 3.93 (m, 1H), 3.74 - 3.67 (m, 1H), 1.84 - 1.71 (m, 8H), 1.14 - 1.08 (m, 2H), 0.97 - 0.90 (m, 2H).

### Example 17: Preparation of 3-fluoro-N-((1S,4S)-4-((1-methyl-6-oxo-5-(trifluoromethyl)-1,6-dihydropyridazin-3-yl)amino)cyclohexyl)-1-(2,2,2-trifluoroethyl)-1H-pyrazole-4-carboxamide (compound 45A)

The synthetic route of **compound 45A** was as follows:

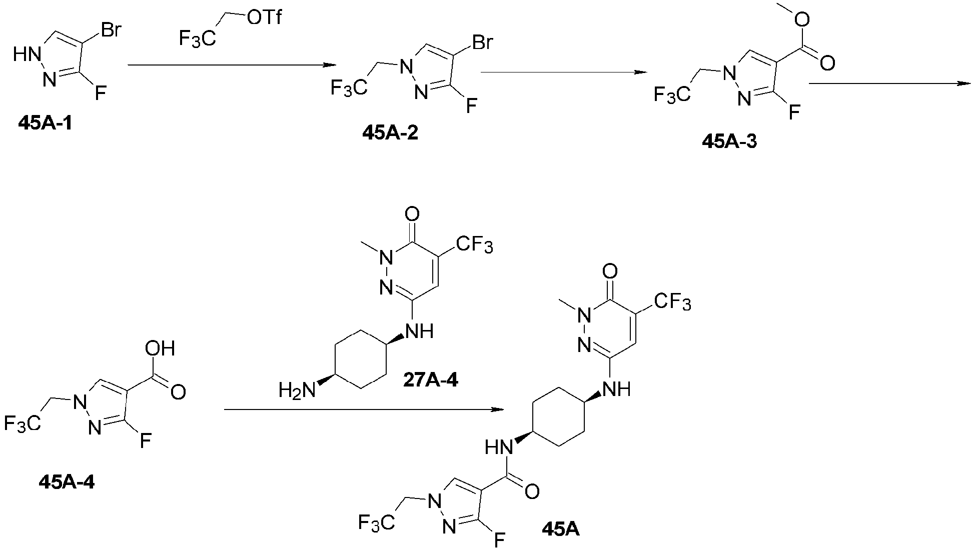

### Step 1: Synthesis of 4-bromo-3-fluoro-1-(2,2,2-trifluoroethyl)-1H-pyrazole

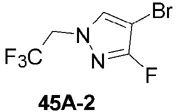

To a solution of 4-bromo-3-fluoro-1H-pyrazole (0.80 g, 4.85 mmol) in N,N-dimethylformamide (5 mL) were added 2,2,2-trifluoroethyl trifluoromethanesulfonate (2.25 g, 9.70 mmol) and cesium carbonate (3.16 g, 9.70 mmol), and the resulting mixture was stirred at 25 °C for 14 h. LCMS analysis showed that the reaction was completed. The reaction liquid was diluted with water (50 mL), and then extracted with ethyl acetate (30 mL × 3). The organic layers were combined and concentrated under reduced pressure to obtain a crude product. The crude product was subjected to silica gel column chromatography (PE:EA (V/V) = 100:0 to 90:10) to obtain a compound 4-bromo-3-fluoro-1-(2,2,2-trifluoroethyl)-1H-pyrazole (0.70 g, yield: 58.4%).

¹H NMR (400 MHz, DMSO-*d₆*): δ 8.11 (s, 1H), 5.06 (q, 2H).

### Step 2: Synthesis of methyl 3-fluoro-1-(2,2,2-trifluoroethyl)-1H-pyrazole-4-carboxylate

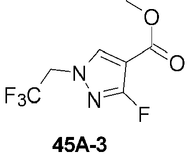

To a solution of 4-bromo-3-fluoro-1-(2,2,2-trifluoroethyl)-1H-pyrazole (300 mg, 1.22 mmol) in methanol (10 mL) were added (1,1'-bis(diphenylphosphino)ferrocene)dichloropalladium(II) (88.9 mg, 0.121 mmol) and triethylamine (0.506 mL, 3.64 mmol), and the resulting mixture was stirred at 80 °C under CO atmosphere for 18 h. LCMS analysis showed that the reaction was completed. The reaction liquid was cooled to room temperature, added with water (20 mL) for dilution, and then extracted with dichloromethane (10 mL×3). The organic phases were combined and concentrated under reduced pressure to obtain a crude product. The crude product was subjected to silica gel column chromatography (PE:EA (V/V) = 100:0 to 80:20) to obtain a compound methyl 3-fluoro-1-(2,2,2-trifluoroethyl)-1H-pyrazole-4-carboxylate (170 mg, yield: 61.9%).
LC-MS, M/Z (ESI): 227.0 [M+H]⁺

### Step 3: Synthesis of 3-fluoro-1-(2,2,2-trifluoroethyl)-1H-pyrazole-4-carboxylic acid

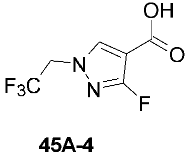

To a solution of methyl 3-fluoro-1-(2,2,2-trifluoroethyl)-1H-pyrazole-4-carboxylate (40.0 mg, 0.177 mmol) in a mixed solvent of tetrahydrofuran (2 mL) and water (2 mL) was added lithium hydroxide (37.1 mg, 0.884 mmol), and the resulting mixture was stirred at 25 °C for 14 h. LCMS analysis showed that the reaction was completed. The pH of the reaction liquid was adjusted to 3 with 1 M hydrochloric acid, and the reaction liquid was then extracted with dichloromethane (10 mL × 3). The organic layers were combined, dried over anhydrous sodium sulfate, filtered, and concentrated to obtain a compound 3-fluoro-1-(2,2,2-trifluoroethyl)-1H-pyrazole-4-carboxylic acid (30.0 mg, yield: 79.9%). The crude product was directly used in the next step without purification.
LC-MS, M/Z (ESI): 213.0 [M+H]⁺

### Step 4: Synthesis of 3-fluoro-N-((1S,4S)-4-((1-methyl-6-oxo-5-(trifluoromethyl)-1,6-dihydropyridazin-3-yl)amino)cyclohexyl)-1-(2,2,2-trifluoroethyl)-1H-pyrazole-4-carboxamide

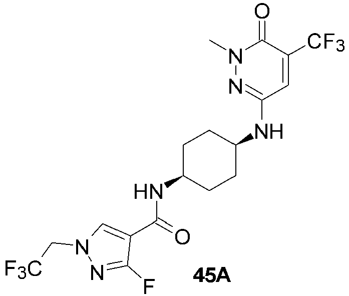

To a solution of 6-(((1S,4S)-4-aminocyclohexyl)amino)-2-methyl-4-(trifluoromethyl)pyridazin-3(2H)-one (41.1 mg, 0.141 mmol) and 3-fluoro-1-(2,2,2-trifluoroethyl)-1H-pyrazole-4-carboxylic acid (30.0 mg, 0.141 mmol) in dimethylsulfoxide (0.117 mL, 0.707 mmol) were added 1-(3-dimethylaminopropyl)-3-ethylcarbodiimide hydrochloride (54.2 mg, 0.283 mmol), 1-hydroxybenzotriazole (38.2 mg, 0.283 mmol), and N,N-diisopropylethylamine (91.4 mg, 0.707 mmol), and the resulting reaction liquid was stirred at 25 °C for 14 h. LCMS analysis showed that the reaction was completed. The reaction liquid was directly purified by preparative liquid chromatography (Xtimate C18 21.2 × 550 mm 5 µm; 0.1% FA.H₂O-ACN; 30-60; 20 mL/min) to obtain compound 3-chloro-1-(2,2-difluoroethyl)-N-((1S,4S)-4-((1-methyl-6-oxo-5-(trifluoromethyl)-1,6-dihydropyridazin-3-yl)amino)cyclohexyl)-1H-pyrazole-4-carboxamide (30.1 mg, yield: 32.1%).
LC-MS, M/Z (ESI): 485.4 [M+H]⁺
¹H NMR (400 MHz, CD₃OD): δ 8.10 - 8.07 (m, 1H), 7.43 (s, 1H), 4.91 - 4.87 (m, 2H), 3.97 - 3.90 (m, 1H), 3.80 - 3.75 (m, 1H), 3.62 (s, 3H), 1.91 - 1.84 (m, 2H), 1.82 - 1.68 (m, 6H).

### Example 18: Preparation of 3-chloro-N-((1S,4S)-4-((5-(difluoromethyl)-1-methyl-6-oxo-1,6-dihydropyridazin-3-yl)amino)cyclohexyl)-1-(2,2,2-trifluoroethyl)-1H-pyrazole-4-carboxamide (compound 51A)

The synthetic route of **compound 51A** was as follows:

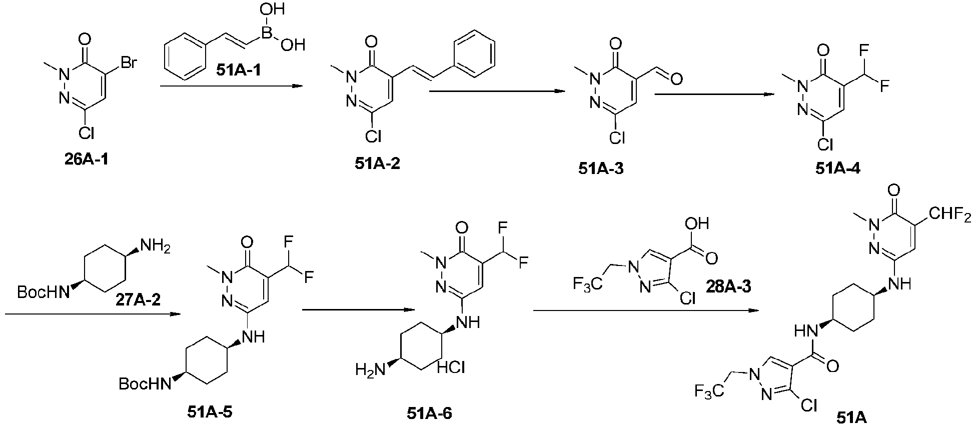

### Step 1: Synthesis of (E)-6-chloro-2-methyl-4-styrylpyridazin-3(2H)-one

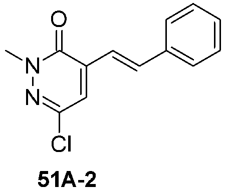

To a solution of 4-bromo-6-chloro-2-methylpyridazin-3(2H)-one (1.21 g, 5.41 mmol) in 1,4-dioxane (15 mL) were added (E)-styrylboronic acid (1.00 g, 6.76 mmol), potassium phosphate (1.72 g, 8.11 mmol), and bis(triphenylphosphine)palladium(II) dichloride (0.38 g, 0.541 mmol), and the resulting mixture was stirred at 85 °C under nitrogen atmosphere for 18 h. LCMS analysis showed that the reaction was completed. The reaction liquid was cooled to room temperature, diluted with water (100 mL), and then extracted with ethyl acetate (100 mL × 3). The organic phases were combined, dried over anhydrous sodium sulfate, filtered, and concentrated to obtain a crude product. The crude product was subjected to silica gel column chromatography (PE:EA (V/V) = 100:0 to 85:15) to obtain a compound (E)-6-chloro-2-methyl-4-styrylpyridazin-3(2H)-one (1.20 g, yield: 90.2%).
LC-MS, M/Z (ESI): 247.0 [M+H]⁺

### Step 2: Synthesis of 6-chloro-2-methyl-3-oxo-2,3-dihydropyridazin-4-carbaldehyde

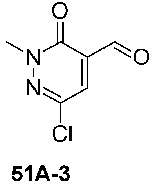

At 0 °C, to a solution of (E)-6-chloro-2-methyl-4-styrylpyridazin-3(2H)-one (800 mg, 3.24 mmol) in a mixed solvent of acetonitrile (3 mL), ethyl acetate (2 mL), and water (3 mL) were added ruthenium(III) chloride (84.8 mg, 0.324 mmol) and sodium periodate (2.08 mg, 9.73 mmol), and the resulting mixture was stirred at 25 °C for 18 h. LCMS analysis showed that the reaction was completed. The resulting mixture was diluted with water (50 mL), and then extracted with ethyl acetate (20 mL × 3). The organic phases were combined, dried over anhydrous sodium sulfate, filtered, and concentrated to obtain a compound 6-chloro-2-methyl-3-oxo-2,3-dihydropyridazin-4-carbaldehyde (500 mg, crude product), which was directly used in the next step.
LC-MS, M/Z (ESI): 173.4 [M+H]⁺

### Step 3: Synthesis of 6-chloro-4-(difluoromethyl)-2-methylpyridazin-3(2H)-one

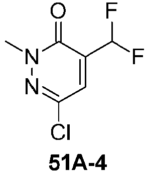

At 0 °C, to a solution of 6-chloro-2-methyl-3-oxo-2,3-dihydropyridazin-4-carbaldehyde (500 mg, 1.74 mmol) in dichloromethane (5 mL) was added diethylaminosulfur trifluoride (841 mg, 5.22 mmol), and the resulting mixture was stirred at 20 °C for 18 h. LCMS analysis showed that the reaction was completed. The reaction liquid was quenched with saturated aqueous sodium carbonate solution (30 mL), and then extracted with dichloromethane (20 mL × 3). The organic phases were combined, dried over anhydrous sodium sulfate, filtered, and concentrated to obtain a crude product. The crude product was subjected to silica gel column chromatography (petroleum ether:ethyl acetate (V/V) = 100:0-90:10) to obtain a compound 6-chloro-4-(difluoromethyl)-2-methylpyridazin-3(2H)-one (160 mg, yield: 33.1%).
LC-MS, M/Z (ESI): 195.0 [M+H]⁺

### Step 4: Synthesis of tert-butyl (18S,4S)-4-((5-(difluoromethyl)-1-methyl-6-oxo-1,6-dihydropyridazin-3-yl)amino)cyclohexyl)carbamate

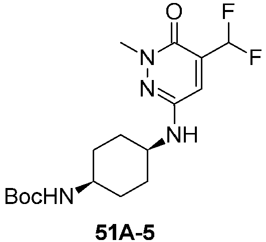

To a solution of 6-chloro-4-(difluoromethyl)-2-methylpyridazin-3(2H)-one (150 mg, 0.771 mmol) in 1,4-dioxane (2 mL) were added tert-butyl ((1S,4S)-4-aminocyclohexyl)carbamate (330 mg, 1.54 mmol), methanesulfonato(2-dicyclohexylphosphino-2',4',6'-triisopropyl-1,1'-biphenyl)(2'-amino-1,1'-biphenyl-2-yl)palladium(II) (65.6 mg, 0.077 mmol), and cesium carbonate (754 mg, 2.31 mmol), and the resulting mixture was stirred at 25 °C under nitrogen atmosphere for 14 h. LCMS analysis showed that the reaction was completed. The reaction liquid was diluted with water (30 mL), and then extracted with ethyl acetate (50 mL × 3). The organic phases were combined, dried over anhydrous sodium sulfate, filtered, and concentrated to obtain a crude product. The crude product was purified by silica gel column chromatography (petroleum ether:ethyl acetate (V/V) = 100:0 to 75:35) to obtain a compound tert-butyl (1S,4S)-4-((5-(difluoromethyl)-1-methyl-6-oxo-1,6-dihydropyridazin-3-yl)amino)cyclohexyl) carbamate (100 mg, yield: 34.8%).LC-MS, M/Z (ESI): 373.2 [M+H]⁺
LC-MS, M/Z (ESI): 373.2 [M+H]+

### Step 5: Synthesis of 6-(((1S,4S)-4-aminocyclohexyl)amino)-4-(difluoromethyl)-2-methylpyridazin-3(2H)-one

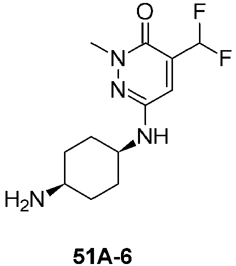

Hydrochloric acid (2 mL, 4 M dioxane solution) was added to tert-butyl (1S,4S)-4-((5-(difluoromethyl)-1-methyl-6-oxo-1,6-dihydropyridazin-3-yl)amino)cyclohexyl)carbamate (30.0 mg, 0.073 mmol), and the resulting mixture was stirred at 25 °C for 1 h. LCMS analysis showed that the reaction was completed. The reaction liquid was directly concentrated to obtain 6-(((1S,4S)-4-aminocyclohexyl)amino)-4-(difluoromethyl)-2-methylpyridazin-3(2H)-one hydrochloride (30.0 mg, crude product), which was directly used in the next step.
LC-MS, M/Z (ESI): 273.4 [M+H]⁺

### Step 6: Synthesis of 3-chloro-N-((1S,4S)-4-((5-(difluoromethyl)-1-methyl-6-oxo-1,6-dihydropyridazin-3-yl)amino)cyclohexyl)-1-(2,2,2-trifluoroethyl)-1H-pyrazole-4-carboxamide

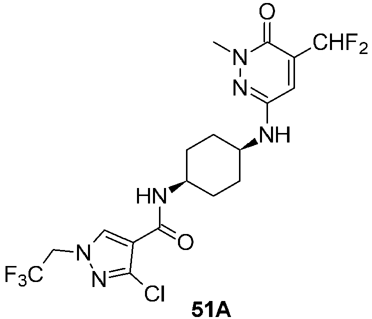

To a solution of 6-(((1S,4S)-4-aminocyclohexyl)amino)-4-(difluoromethyl)-2-methylpyridazin-3(2H)-one (60.0 mg, 0.220 mmol) and 3-chloro-1-(2,2,2-trifluoroethyl)-1H-pyrazole-4-carboxylic acid (50.4 mg, 0.220 mmol) in dimethylsulfoxide (3 mL) were added N,N-diisopropylethylamine (142 mg, 1.10 mmol), 1-(3-dimethylaminopropyl)-3-ethylcarbodiimide hydrochloride (84.6 mg, 0.440 mmol), and 1-hydroxybenzotriazole (59.5 mg, 0.440 mmol), and the resulting mixture was stirred at 25 °C for 18 h. LCMS analysis showed that the reaction was completed. The reaction liquid was added with water (5 mL) for dilution, and then extracted with ethyl acetate (5 mL × 3). The organic phases were combined, washed with saturated saline (10 mL), dried over anhydrous sodium sulfate, filtered, and concentrated to obtain a crude product. The crude product was purified by preparative liquid chromatography (Prime C18, 30 × 150 mm, 5 µm; 0.05% NH₃.H₂O-ACN; 35-56; 25 mL/min) to obtain a compound 3-chloro-N-((1S,4S)-4-((5-(difluoromethyl)-1-methyl-6-oxo-1,6-dihydropyridazin-3-yl)amino)cyclohexyl)-1-(2,2,2-trifluoroethyl)-1H-pyrazole-4-carboxamide (12.0 mg, yield: 11.3%).
LC-MS, M/Z (ESI): 483.2 [M+H]⁺
¹H NMR (400 MHz, CD₃OD): δ 8.22 (s, 1H), 7.27 (s, 1H), 6.76 (t, 1H), 4.97 (q, 2H), 4.02 - 3.92 (m, 1H), 3.82 - 3.73 (m, 1H), 3.62 (s, 3H), 1.86 - 1.73 (m, 8H).

### Example 19: Preparation of 3-chloro-N-((1S,4S)-4-((1-ethyl-6-oxo-5-(trifluoromethyl)-1,6-dihydropyridazin-3-yl)amino)cyclohexyl)-1-(2,2,2-trifluoroethyl)-1H-pyrazole-4-carboxamide (compound 52A)

The synthetic route of **compound 52A** was as follows:

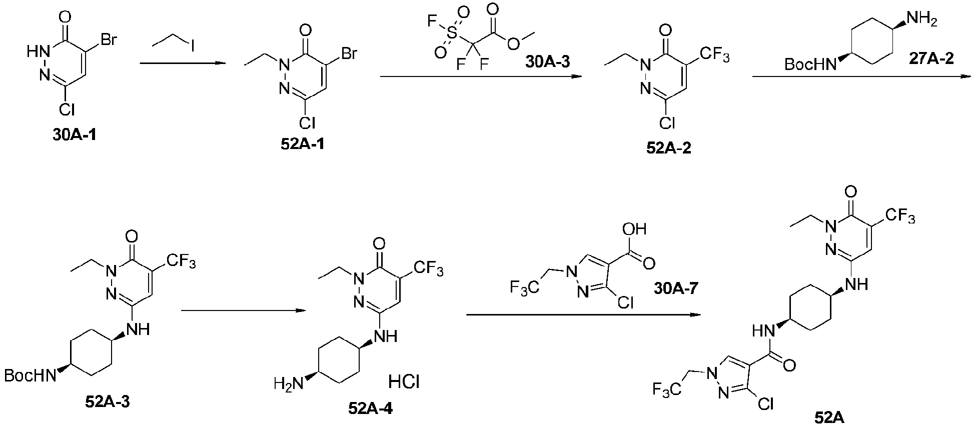

### Step 1: Synthesis of 4-bromo-6-chloro-2-ethylpyridazin-3(2H)-one

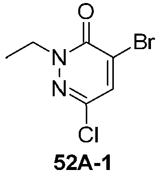

At 0 °C, to a solution of 4-bromo-6-chloropyridazin-3(2H)-one (2.00 g, 9.55 mmol) in N,N-dimethylformamide (10 mL) was added sodium hydride (955 mg, 23.9 mmol, 60% Wt), and after the mixture was stirred at 0 °C for 30 min, iodoethane (2.98 g, 19.1 mmol) was added. The resulting mixture was stirred at 25 °C for 1 h. LCMS analysis showed that the reaction was completed. The reaction liquid was added with water (50 mL) for dilution, and then extracted with ethyl acetate (20 mL × 3). The organic phases were combined and concentrated to obtain a crude product. The crude product was purified by silica gel column chromatography (petroleum ether:ethyl acetate (V/V) = 100:0 to 90:0) to obtain a compound 4-bromo-6-chloro-2-ethylpyridazin-3(2H)-one (1.00 g, yield: 44.1%).
LC-MS, M/Z (ESI):237.0 [M+H]⁺

### Step 2: Synthesis of 6-chloro-2-ethyl-4-(trifluoromethyl)pyridazin-3(2H)-one

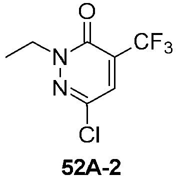

To a solution of 4-bromo-6-chloro-2-ethylpyridazin-3(2H)-one (1.00 g, 4.21 mmol) in N-methylpyrrolidone (10 mL) were added copper(I) iodide (541 mg, 1.26 mmol) and methyl fluorosulfonyldifluoroacetate (2.63 g, 12.6 mmol), and the resulting mixture was stirred at 90 °C under nitrogen atmosphere for 18 h. LCMS analysis showed that the reaction was completed. The reaction liquid was cooled to room temperature, added with water (50 mL) for dilution, and then extracted with ethyl acetate (20 mL × 3). The organic phases were combined and concentrated to obtain a crude product. The crude product was purified by silica gel column chromatography (petroleum ether:ethyl acetate (V/V) = 100:0 to 90:0) to obtain a compound 6-chloro-2-ethyl-4-(trifluoromethyl)pyridazin-3(2H)-one (500 mg, yield: 52.4%).
LC-MS, M/Z (ESI): 227.0 [M+H]⁺

### Step 3: Synthesis of tert-butyl (1S,4S)-4-((1-ethyl-6-oxo-5-(trifluoromethyl)-1,6-dihydropyridazin-3-yl)amino)cyclohexyl)carbamate

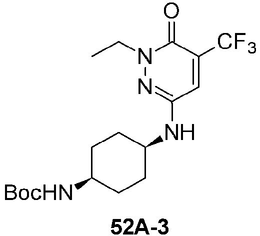

To a solution of 6-chloro-2-ethyl-4-(trifluoromethyl)pyridazin-3(2H)-one (200 mg, 0.883 mmol) and tert-butyl ((1S,4S)-4-aminocyclohexyl)carbamate (378 mg, 1.77 mmol) in 1,4-dioxane (4 mL) were added methanesulfonato(2-dicyclohexylphosphino-2',4',6'-triisopropyl-1,1'-biphenyl)(2'-amino-1,1'-biphenyl-2-yl)palladium(II) (375 mg, 0.441 mmol) and cesium carbonate (719 mg, 2.21 mmol), and the resulting mixture was stirred at 80 °C for 18 h. LCMS analysis showed that the reaction was completed. The reaction liquid was cooled to room temperature, added with water (20 mL) for dilution, and extracted with ethyl acetate (10 mL × 3). The organic phases were combined and concentrated to obtain a crude product. The crude product was purified by silica gel column chromatography (petroleum ether:ethyl acetate (V/V) = 100:0 to 80:20) to obtain a compound tert-butyl (1S,4S)-4-((1-ethyl-6-oxo-5-(trifluoromethyl)-1,6-dihydropyridazin-3-yl)amino)cyclohexyl)carbamate (180 mg, yield: 50.4%).
LC-MS, M/Z (ESI): 405.4 [M+H]⁺

### Step 4: Synthesis of 6-(((1S,4S)-4-aminocyclohexyl)amino)-2-ethyl-4-(trifluoromethyl)pyridazin-3(2H)-one hydrochloride

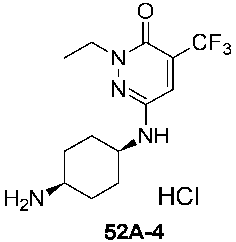

To a solution of tert-butyl (1S,4S)-4-((1-ethyl-6-oxo-5-(trifluoromethyl)-1,6-dihydropyridazin-3-yl)amino)cyclohexyl)carbamate (180 mg, 0.445 mmol) in dichloromethane (2 mL) was added a hydrogen chloride solution (2 mL, 4 M, in 1,4-dioxane), and the resulting mixture was stirred at 25 °C for 2 h. LCMS analysis showed that the reaction was completed. The reaction liquid was directly concentrated by rotary evaporation to obtain a compound 6-(((1S,4S)-4-aminocyclohexyl)amino)-2-ethyl-4-(trifluoromethyl)pyridazin-3(2H)-one (120 mg, yield: 88.6%), which was directly used in the next step.
LC-MS, M/Z (ESI): 305.4 [M+H]⁺

### Step 5: Synthesis of 3-chloro-N-((1S,4S)-4-((1-ethyl-6-oxo-5-(trifluoromethyl)-1,6-dihydropyridazin-3-yl)amino)cyclohexyl)-1-(2,2,2-trifluoroethyl)- 1H-pyrazole-4-carboxamide

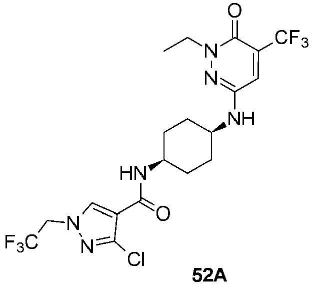

To a solution of 6-(((1S,4S)-4-aminocyclohexyl)amino)-2-ethyl-4-(trifluoromethyl)pyridazin-3(2H)-one (60.0 mg, 0.197 mmol) and 3-chloro-1-(2,2,2-trifluoroethyl)-1H-pyrazole-4-carboxylic acid (54.1 mg, 0.237 mmol) in dimethylsulfoxide (4 mL) were added N,N-diisopropylethylamine (76.5 mg, 0.591 mmol), 1-(3-dimethylaminopropyl)-3-ethylcarbodiimide hydrochloride (56.7 mg, 0.296 mmol), and 1-hydroxybenzotriazole (39.9 mg, 0.296 mmol), and the resulting mixture was stirred at 25 °C for 18 h. LCMS analysis showed that the reaction was completed. The reaction liquid was purified by preparative liquid chromatography (Xtimate C18 21.2 × 550 mm 5 µm; 0.1% FA.H₂O-ACN; 20-60; 20 mL/min) to obtain a compound 3-chloro-N-((1S,4S)-4-((1-ethyl-6-oxo-5-(trifluoromethyl)-1,6-dihydropyridazin-3-yl)amino)cyclohexyl)-1-(2,2,2-trifluoroethyl)-1H-pyrazole-4-carboxamide (45.9 mg, yield: 45.2%).
LC-MS, M/Z (ESI): 515.4 [M+H]⁺
¹H NMR (400 MHz, CD₃OD): δ 8.22 (s, 1H), 7.41 (s, 1H), 4.96 (q, 2H), 4.06 (q, 2H), 4.01 - 3.94 (m, 1H), 3.81 - 3.73 (m, 1H), 1.90 - 1.69 (m, 8H), 1.31 (t, 3H).

### Example 20: Preparation of 4-methoxy-N-((1S,4S)-4-((1-methyl-6-oxo-5-(trifluoromethyl)-1,6-dihydropyridazin-3-yl)amino)cyclohexyl)benzamide (compound 53A)

The synthetic route of **compound 53A** was as follows:

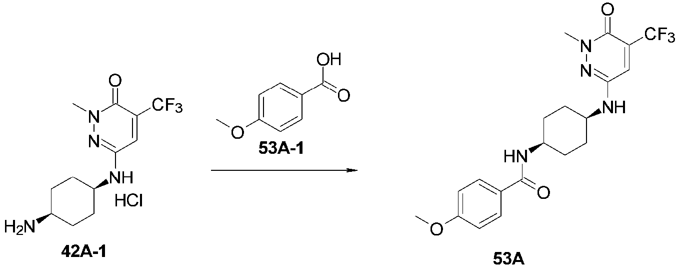

At room temperature, a compound p-methoxybenzoic acid (45.00 mg, 0.30 mmol) was dissolved in anhydrous N,N-dimethylformamide (1 mL), and N,N-diisopropylethylamine (152.90 mg, 1.18 mmol) and O-(7-azabenzotriazol-1-yl)-N,N,N',N'-tetramethyluronium hexafluorophosphate (224.92 mg, 0.59 mmol) were then added. The reaction liquid was stirred at room temperature for 30 min, and 6-(((1S,4S)-4-aminocyclohexyl)amino)-2-methyl-4-(trifluoromethyl)pyridazin-3(2H)-one hydrochloride (103.03 mg, 0.35 mmol) was then added. The reaction liquid was successively stirred at room temperature for 4 h. After the reaction of the starting material was completed as monitored by TLC, stirring was stopped. The reaction liquid was added with water (10 mL) for dilution, and extracted with ethyl acetate (4 mL × 3). The organic phase was collected, dried over anhydrous sodium sulfate, filtered, and concentrated. The residue was separated and purified by silica gel column chromatography (petroleum ether:ethyl acetate (V/V) = 50:50) to obtain a compound 4-methoxy-N-((1S,4S)-4-((1-methyl-6-oxo-5-(trifluoromethyl)-1,6-dihydropyridazin-3-yl)amino)cyclohexyl)benzamide (46.6 mg, yield: 37.10%).
LC-MS,M/Z(ESI):425.1 [M+H]⁺
¹H NMR (400 MHz, CDCl₃) δ 7.76 - 7.68 (m, 2H), 7.08 (s, 1H), 6.97 - 6.89 (m, 2H), 5.99 (d, 1H), 4.17 - 4.08 (m, 2H), 3.85 (s, 3H), 3.79 (s, 1H), 3.67 (s, 3H), 1.97 - 1.62 (m, 8H).

### Example 21: Preparation of N-((1R,3S)-3-((7-chloro-4-oxo-3-(2,2,2-trifluoroethyl)-3,4-dihydrophthalazin-1-yl)amino)cyclohexyl)-1-methyl-1H-pyrazole-4-carboxamide (compound 24A)

The synthetic route of **compound 24A** was as follows:

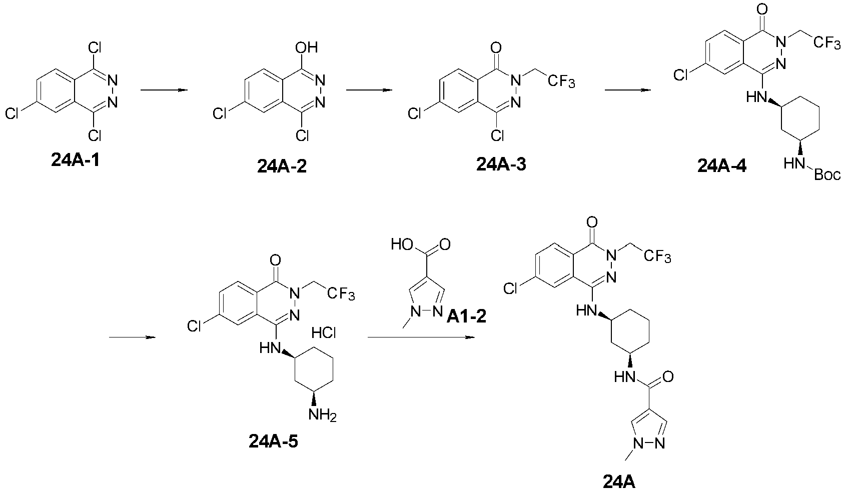

### Step 1: Synthesis of compound 4,6-dichlorophthalazin-1-ol

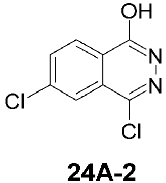

At room temperature, a compound 1,4,6-trichlorophthalazine (5.00 g, 21.42 mmol) was dissolved in acetic acid (50 mL), and the reaction liquid was stirred at 120 °C for 5 h. After the reaction of the starting material was completed as monitored by TLC, stirring was stopped, and the reaction liquid was cooled to room temperature. Subsequently, the reaction liquid was added with water (300 mL) for dilution, and extracted with ethyl acetate (50 mL × 3). The organic phase was collected, dried over anhydrous sodium sulfate, and concentrated by distillation under reduced pressure. The residue was separated and purified by silica gel column chromatography (petroleum ether:ethyl acetate (V/V) = 100:20) to obtain a compound 4,6-dichlorophthalazin-1-ol (4.32 g, yield: 93.81%).
LC-MS,M/Z(ESI):216.2[M+H]⁺

### Step 2: Synthesis of compound 4,6-dichloro-2-(2,2,2-trifluoroethyl)phthalazin-1(2H)-one

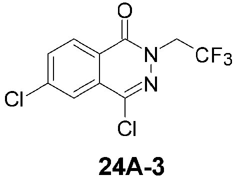

At room temperature, the compound 4,6-dichlorophthalazin-1-ol (3.00 g, 13.95 mmol) and potassium carbonate (1.93 g, 27.90 mmol) were dissolved in anhydrous N,N-dimethylformamide (30 mL), and 2-iodo-1,1,1-trifluoroethane (4.39 g, 20.93 mmol) was then added. After the addition was completed, the reaction liquid was stirred in an oil bath at 80 °C for 12 h. After the reaction of the starting material was completed as monitored by TLC, stirring was stopped, and the reaction liquid was cooled to room temperature. Subsequently, the reaction liquid was added with water (200 mL) for dilution, and extracted with ethyl acetate (40 mL × 3). The organic phase was collected, dried over anhydrous sodium sulfate, and concentrated by distillation under reduced pressure. The residue was separated and purified by silica gel column chromatography (petroleum ether:ethyl acetate (V/V) = 100: 10) to obtain a compound 4,6-dichloro-2-(2,2,2-trifluoroethyl)phthalazin-1(2H)-one (580 mg, yield: 14%).
LC-MS, [M+H]⁺

### Step 3: Synthesis of compound tert-butyl ((1R,3S)-3-((7-chloro-4-oxo-3-(2,2,2-trifluoroethyl)-3,4-dihydrophthalazin-1-yl)amino)cyclohexyl)carbamate

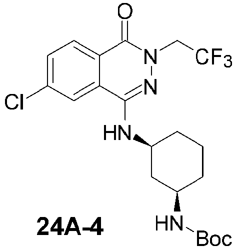

At room temperature, the compound 6-dichloro-2-(2,2,2-trifluoroethyl)phthalazin-1(2H)-one (400.00 mg, 1.35 mmol) and tert-butyl ((1R,3S)-3-aminocyclohexyl)carbamate (346.27 mg, 1.62 mmol) were dissolved in toluene (5 mL), and sodium tert-butoxide (258.80 mg, 2.70 mmol), 2-dicyclohexylphosphino-2',6'-diisopropoxy-1,1'-biphenyl (125.07 mg, 0.27 mmol), and tris(dibenzylidene-BASE acetone)dipalladium (123.30 mg, 0.13 mmol) were added. The reaction liquid was stirred at 80 °C under nitrogen atmosphere for 4 h. After the reaction of the starting material was completed as monitored by TLC, stirring was stopped, and the reaction liquid was cooled to room temperature. Subsequently, the reaction liquid was added with water (20 mL) for dilution, and extracted with ethyl acetate (8 mL × 5). The organic phase was collected, dried over anhydrous sodium sulfate, and concentrated by distillation under reduced pressure. The residue was separated and purified by silica gel column chromatography (petroleum ether:ethyl acetate (V/V) = 100:40) to obtain a compound tert-butyl ((1R,3S)-3-((7-chloro-4-oxo-3-(2,2,2-trifluoroethyl)-3,4-dihydrophthalazin-1-yl)amino)cyclohexyl)carbamate (100.00 mg, yield: 15.64%).
LC-MS,M/Z(ESI):475.9[M+H]⁺

### Step 4: Synthesis of compound 4-(((1S,3R)-3-aminocyclohexyl)amino)-6-chloro-2-(2,2,2-trifluoroethyl)phthalazin-1(2H)-one hydrochloride

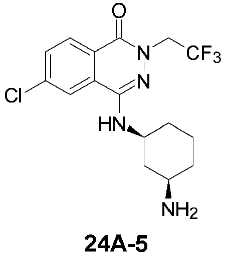

At room temperature, the compound tert-butyl ((1R,3S)-3-((7-chloro-4-oxo-3-(2,2,2-trifluoroethyl)-3,4-dihydrophthalazin-1-yl)amino)cyclohexyl)carbamate (100 mg, 0.21 mmol) was dissolved in dichloromethane (2 mL). The mixture was stirred at 0 °C for 10 min, and a solution of hydrogen chloride in 1,4-dioxane (0.11 mL, 0.11 mmol) was slowly added dropwise. The reaction liquid was naturally warmed to room temperature and stirred for 2 h. After the reaction of the starting material was completed as monitored by TLC, stirring was stopped. The reaction liquid was evaporated under reduced pressure to remove the solvent, so as to obtain a compound 4-(((1S,3R)-3-aminocyclohexyl)amino)-6-chloro-2-(2,2,2-trifluoroethyl)phthalazin-1(2H)-one hydrochloride (80 mg, crude product), which was directly used in the next step.
LC-MS,M/Z(ESI):375.3[M+H]⁺

### Step 5: Synthesis of compound N-((1R,3S)-3-((7-chloro-4-oxo-3-(2,2,2-trifluoroethyl)-3,4-dihydrophthalazin-1-yl)amino)cyclohexyl)-1-methyl-1H-pyrazole-4-carboxamide

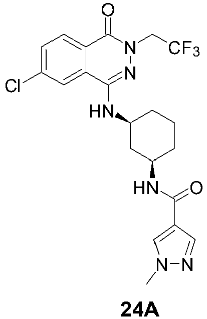

At room temperature, a compound 1-methyl-1H-pyrazole-4-carboxylic acid (20 mg, 0.16 mmol) was dissolved in anhydrous N,N-dimethylformamide (2 mL), and N,N-diisopropylethylamine (81.98 mg, 0.64 mmol) and O-(7-azabenzotriazol-1-yl)-N,N,N',N'-tetramethyluronium hexafluorophosphate (120.60 mg, 0.32 mmol) were then added. The mixture was stirred at room temperature for 30 min, and 4-(((1S,3R)-3-aminocyclohexyl)amino)-6-chloro-2-(2,2,2-trifluoroethyl)phthalazin-1(2H)-one hydrochloride (80 mg, 0.19 mmol) was then added. The mixture was successively stirred at room temperature for 4 h. After the reaction of the starting material was completed as monitored by TLC, stirring was stopped, and the reaction liquid was cooled to room temperature. Subsequently, the reaction liquid was added with water (10 mL) for dilution, and extracted with ethyl acetate (5 mL × 3). The organic phase was collected, dried over anhydrous sodium sulfate, and concentrated by distillation under reduced pressure. The residue was separated and purified by silica gel column chromatography (petroleum ether:ethyl acetate (V/V) = 100:30) to obtain a compound N-((1R,3S)-3-((7-chloro-4-oxo-3-(2,2,2-trifluoroethyl)-3,4-dihydrophthalazin-1-yl)amino)cyclohexyl)-1-methyl-1H-pyrazole-4-carboxamide (23.4 mg, yield: 24.81%).
LC-MS,M/Z(ESI):483.1 [M+H]⁺
¹H NMR (400 MHz, DMSO-d6) δ 8.41 (d, 1H), 8.26 (d, 1H), 8.10 (s, 1H), 7.90 (dd, 1H), 7.86 (d, 1H), 7.82 (s, 1H), 6.73 (d, 1H), 4.87 - 4.70 (m, 2H), 3.83 (s, 3H), 3.77 - 3.67 (m, 2H), 2.23 (d, 1H), 2.08 (d, 1H), 1.87 - 1.76 (m, 2H), 1.42 - 1.25 (m, 4H).

For the preparation methods for the following compounds, reference can be made to the examples described above.

| Structure | Name | LC-MS [M+H]⁺ |
|---|---|---|
| 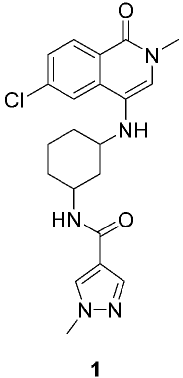 | *N*-(3-((6-chloro-2-methyl-1-oxo-1,2-dihydroisoquinolin-4-yl)amino)cyclohexyl)-1-methyl-1*H*-pyrazole-4-carboxamide | 414.1 |
| 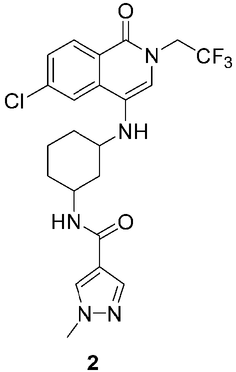 | *N*-(3-((6-chloro-1-oxo-2-(2,2,2-trifluoroethyl)-1,2-dihydroisoquinolin-4-yl)amino)cyclohexyl)-1-methyl-1*H-*pyrazole-4-carboxamide | 482.2 |
| 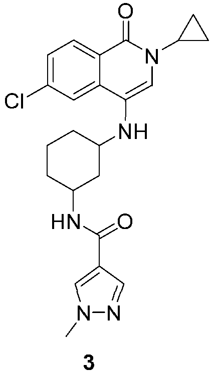 | *N*-(3-((6-chloro-2-cyclopropyl-1-oxo-1,2-dihydroisoquinolin-4-yl)amino)cyclohexyl)-1-methyl-1*H*-pyrazole-4-carboxamide | 440.0 |
| 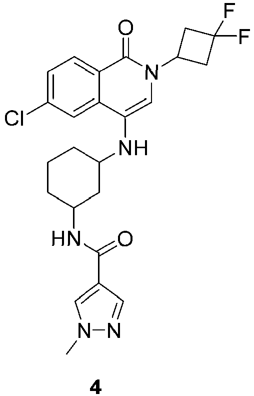 | *N*-(3-((6-chloro-2-(3,3-difluorocyclobutyl)-1-oxo-1,2-dihydroisoquinolin-4-yl)amino)cyclohexyl)-1-methyl-1*H-*pyrazole-4-carboxamide | 490.2 |
| 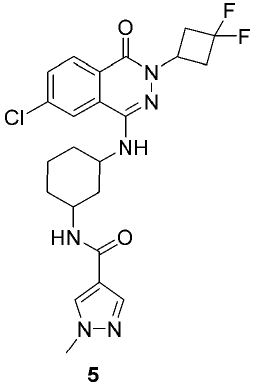 | *N*-(3-((7-chloro-3-(3,3-difluorocyclobutyl)-4-oxo-3,4-dihydrophthalazin-1-yl)amino)cyclohexyl)-1-methyl-1*H-*pyrazole-4-carboxamide | 491.3 |
| 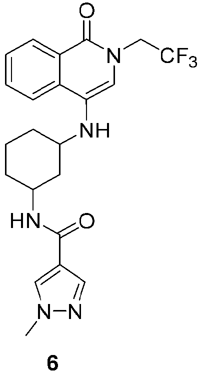 | 1-methyl-*N*-(3-((1-oxo-2-(2,2,2-trifluoroethyl)-1,2-dihydroisoquinolin-4-yl)amino)cyclohexyl)-1*H*-pyrazole-4-carboxamide | 448.2 |
| 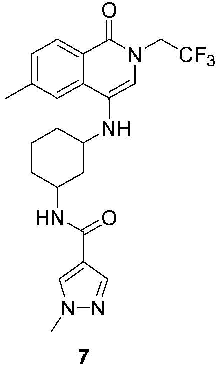 | 1-methyl-*N*-(3-((6-methyl-1-oxo-2-(2,2,2-trifluoroethyl)-1,2-dihydroisoquinolin-4-yl)amino)cyclohexyl)-1*H*-pyrazole-4-carboxamide | 462.2 |
| 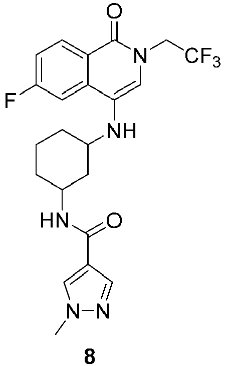 | *N*-(3-((6-fluoro-1-oxo-2-(2,2,2-trifluoroethyl)-1,2-dihydroisoquinolin-4-yl)amino)cyclohexyl)-1-methyl-1*H-*pyrazole-4-carboxamide | 466.1 |
| 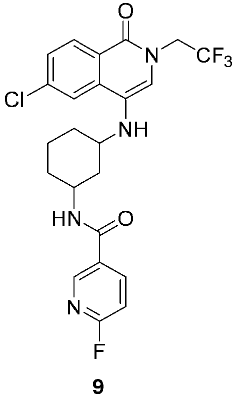 | *N*-(3-((6-chloro-1-oxo-2-(2,2,2-trifluoroethyl)-1,2-dihydroisoquinolin-4-yl)amino)cyclohexyl)-6-fluoronicotinamide | 497.3 |
| 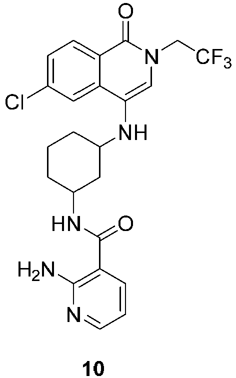 | 2-amino-*N*-(3-((6-chloro-1-oxo-2-(2,2,2-trifluoroethyl)-1,2-dihydroisoquinolin-4-yl)amino)cyclohexyl)nicotinamide | 494.2 |
| 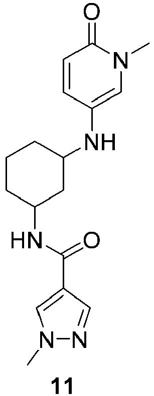 | 1-methyl-*N*-(3-((1-methyl-6-oxo-1,6-dihydropyridin-3-yl)amino)cyclohexyl)- 1*H*-pyrazole-4-carboxamide | 330.1 |
| 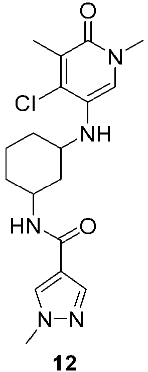 | *N*-(3-((4-chloro-1,5-dimethyl-6-oxo-1,6-dihydropyridin-3-yl)amino)cyclohexyl)-1-methyl-1*H*-pyrazole-4-carboxamide | 378.1 |
| 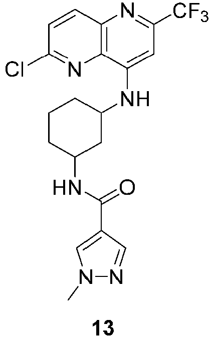 | *N*-(3-((6-chloro-2-(trifluoromethyl)-1,5-naphthyridin-4-yl)amino)cyclohexyl)-1-methyl-1*H*-pyrazole-4-carboxamide | 453.2 |
| 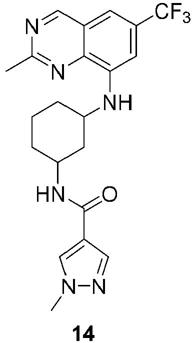 | 1-methyl-*N*-(3-((2-methyl-6-(trifluoromethyl)quinazolin-8-yl)amino)cyclohexyl)-1-methyl-1*H*-pyrazole-4-carboxamide | 433.2 |
| 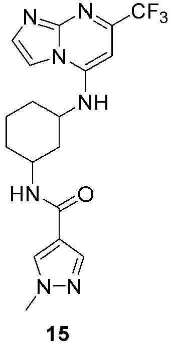 | 1-methyl-*N*-(3-((7-(trifluoromethyl)imidazo[1,2-*a*]pyrimidin-5-yl)amino)cyclohexyl)-1-methyl-1*H*-pyrazole-4-carboxamide | 408.1 |
| 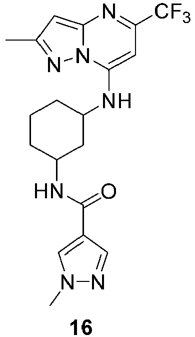 | 1-methyl-*N*-(3-((2-methyl-5-(trifluoromethyl)pyrazolo[ 1,5-*a*]pyrimidin-7-yl)amino)cyclohexyl)-1-methyl-1*H*-pyrazole-4-carboxamide | 422.2 |
| 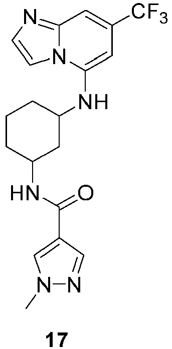 | 1-methyl-*N*-(3-((7-(trifluoromethyl)imidazo[1,2-*a*]pyridin-5-yl)amino)cyclohexyl)-1-methyl-1H-pyrazole-4-carboxamide | 407.1 |
| 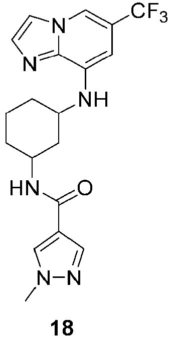 | 1-methyl-*N*-(3-((6-(trifluoromethyl)imidazo[1,2-*a*]pyridin-8-yl)amino)cyclohexyl)-1-methyl-1H-pyrazole-4-carboxamide | 407.2 |
| 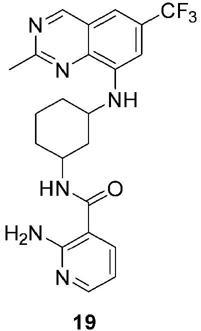 | 2-amino-N-(3-((2-methyl-6-(trifluoromethyl)quinazolin-8-yl)amino)cyclohexyl)nicotinamide | 445.3 |
| 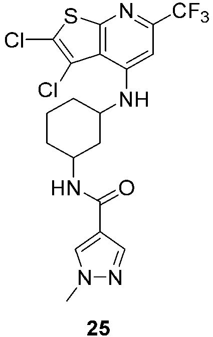 | *N*-(3-((2,3-dichloro-6-(trifluoromethyl)thieno[2,3-*b*]pyridin-4-yl)amino)cyclohexyl)-1-methyl-1*H*-pyrazole-4-carboxamide | 492.1 |
| 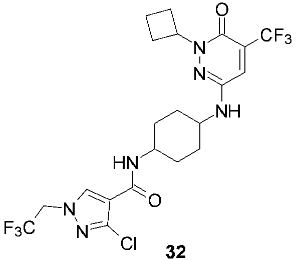 | 3-chloro-N-(4-((1-cyclobutyl-6-oxo-5-(trifluoromethyl)-1,6-dihydropyridazin-3-yl)amino)cyclohexyl)-1-(2,2,2-trifluoroethyl)-1H-pyrazole-4-carboxamide | 541.2 |
| 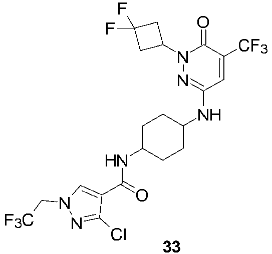 | 3-chloro-N-(4-((1-(3,3-difluorocyclobutyl)-6-oxo-5-(trifluoromethyl)-1,6-dihydropyridazin-3-yl)amino)cyclohexyl)-1-(2,2,2-trifluoroethyl)-1H-pyrazole-4-carboxamide | 577.1 |
| 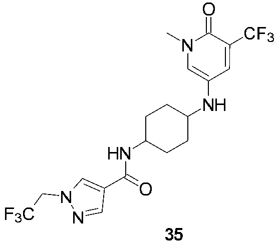 | N-(4-((1-methyl-6-oxo-5-(trifluoromethyl)-1,6-dihydropyridin-3-yl)amino)cyclohexyl)-1-(2,2,2-trifluoroethyl)-1H-pyrazole-4-carboxamide | 466.2 |
| 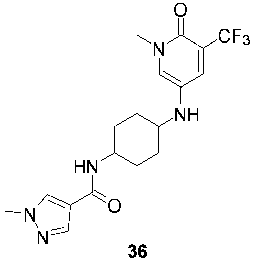 | 1-methyl-N-(4-((1-methyl-6-oxo-5-(trifluoromethyl)-1,6-dihydropyridin-3-yl)amino)cyclohexyl)-1H-pyrazole-4-carboxamide | 398.2 |
| 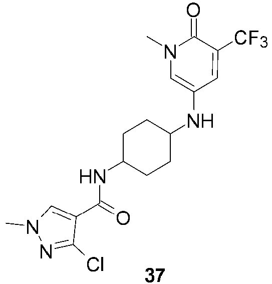 | 3-chloro-1-methyl-N-(4-((1-methyl-6-oxo-5-(trifluoromethyl)-1,6-dihydropyridin-3-yl)amino)cyclohexyl)-1H-pyrazole-4-carboxamide | 433.1 |
| 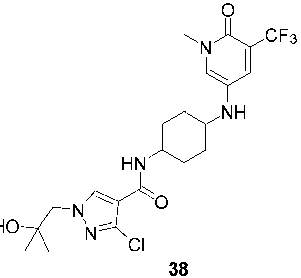 | 3-chloro-1-(2-hydroxy-2-methylpropyl)-N-(4-((1-methyl-6-oxo-5-(trifluoromethyl)-1,6-dihydropyridin-3-yl)amino)cyclohexyl)-1H-pyrazole-4-carboxamide | 490.2 |
| 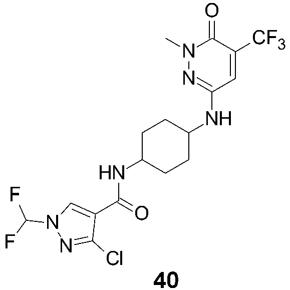 | 3-chloro-1-(difluoromethyl)-N-(4-((1-methyl-6-oxo-5-(trifluoromethyl)-1,6-dihydropyridazin-3-yl)amino)cyclohexyl)-1H-pyrazole-4-carboxamide | 469.1 |
| 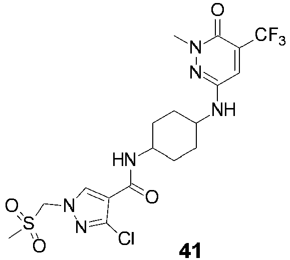 | 3-chloro-N-(4-((1-methyl-6-oxo-5-(trifluoromethyl)-1,6-dihydropyridazin-3-yl)amino)cyclohexyl)-1-((methylsulfonyl)methyl)-1H-pyrazole-4-carboxamide | 511.1 |
| 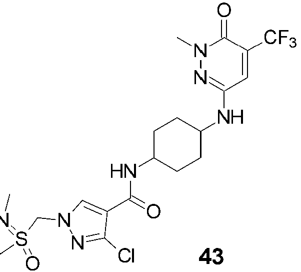 | 3-chloro-1-((N,S-dimethylsulfonimidoyl)methyl)-N-(4-((1-methyl-6-oxo-5-(trifluoromethyl)-1,6-dihydropyridazin-3-yl)amino)cyclohexyl)-1H-pyrazole-4-carboxamide | 524.1 |
| 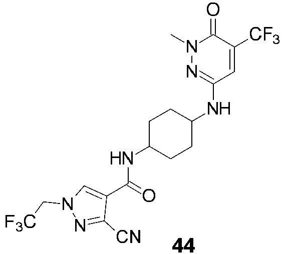 | 3-cyano-N-(4-((1-methyl-6-oxo-5-(trifluoromethyl)-1,6-dihydropyridazin-3-yl)amino)cyclohexyl)-1-(2,2,2-trifluoroethyl)-1H-pyrazole-4-carboxamide | 492.2 |
| 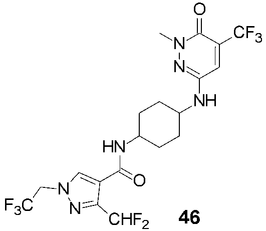 | 3-(difluoromethyl)-N-(4-((1-methyl-6-oxo-5-(trifluoromethyl)-1,6-dihydropyridazin-3-yl)amino)cyclohexyl)-1-(2,2,2-trifluoroethyl)-1H-pyrazole-4-carboxamide | 517.2 |
| 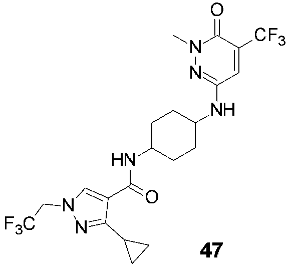 | 3-cyclopropyl-N-(4-((1-methyl-6-oxo-5-(trifluoromethyl)-1,6-dihydropyridazin-3-yl)amino)cyclohexyl)-1-(2,2,2-trifluoroethyl)-1H-pyrazole-4-carboxamide | 507.2 |

### Test Example 1: MRGPRX2 In Vitro Calcium Flux Assay

The antagonistic effect of the compounds on MRGPRX2 was determined in a CHO stably-transfected cell line that highly expresses human MRGPRX2. 18 h before the assay, cells were seeded in a black-wall transparent base plate containing DMEM/F12 (1:1) medium at a specific density and incubated at 37 °C with 5% CO₂ for 18 h. Then, an appropriate amount of dye solution was added to each well, and the cells were incubated for another 30 min in the incubator at 37 °C in the dark. The cells were further incubated for 10 min at room temperature in the dark. Subsequently, the compounds at different final concentrations were added to each well and equilibrated for 20 min. Finally, a certain amount of C48/80 solution was added to the cells, and fluorescence signals were measured using FLIPR. The concentration of the compound was plotted on the X-axis, and the fluorescence signal value was plotted on the Y-axis. The antagonistic effect (IC₅₀ value) of the compound was calculated using GraphPad Prism 8.0 software. A denotes an IC₅₀ value ≤ 100 nM, B denotes 100 nM < IC₅₀ value ≤ 500 nM, and C denotes an IC₅₀ value greater than 500 nM.

The MRGPRX2 calcium flux assay results demonstrate that the compounds of the present disclosure exhibit good antagonistic effect on MRGPRX2.

**Table 1. MRGPRX2 calcium flux assay results**

| Compound | IC₅₀ (nM) |
|---|---|
| Compound 22A | B |
| Compound 23A | B |
| Compound 24A | B |
| Compound 28A | A |
| Compound 30A | A |
| Compound 31A | A |
| Compound 34A | B |
| Compound 42A | B |
| Compound 45A | A |
| Compound 48A | B |
| Compound 50A | A |
| Compound 51A | A |
| Compound 52A | B |
| Compound 53A | A |

### Test Example 2: MRGPRX2 In Vitro IP-1 Assay

The antagonistic effect of the compounds on MRGPRX2 was determined in a CHO stably-transfected cell line that highly expresses human MRGPRX2. The drugs prepared by serial dilution with DMSO were added to a micropore plate, and the stably-transfected cells were treated with trypsin. The cells were collected, counted, and then seeded into the microplate, and the microplate was incubated at 37 °C for 10 min. Cortistatin-14 solution at a specific concentration was added, and the cells were centrifuged and then incubated at 37 °C for 60 min. After the incubation was completed, diluted d2-IP1 and Anti-IP 1-Cryptate were added to each well. The cells were centrifuged and then left to stand at room temperature for 1 h. After the incubation was completed, the fluorescence readings at 665 nm and 620 nm were obtained. The concentration of the compound was plotted on the X-axis, and the fluorescence signal ratio was plotted on the Y-axis. The antagonistic effect (IC₅₀ value) of the compound was calculated using Graphpad software.

**Table 2: MRGPRX2 IP-1 assay results**

| Example | IC₅₀ (nM) |
|---|---|
| Compound 28A | 99 |
| Compound 30A | 52 |
| Compound 31A | 10 |
| Compound 34A | 161 |
| Compound 42A | 224 |
| Compound 45A | 86 |
| Compound 48A | 364 |
| Compound 50A | 75 |
| Compound 51A | 85 |
| Compound 52A | 137 |
| Compound 53A | 70 |

The MRGPRX2 IP-1 assay results demonstrate that the compounds of the present disclosure exhibit good antagonistic effect on MRGPRX2.

### Test Example 3: Hepatotoxicity Assay for Compounds

The hepatotoxicity assay for the compounds was performed using HepG2 (ATCC, HB-8065) cells. Cell viability was determined using a CellTiter-Glo Luminescent Cell Viability Assay kit (Promega, G7573), and the toxicity of the compounds was characterized by the inhibition of viability of HepG2 cells. HepG2 cells in the logarithmic growth phase were collected, the concentration of the cell suspension was adjusted, and then the cells were plated on a 96-well cell culture plate at 5000 cells/well. The cells were incubated in a cell incubator with 5% CO₂ at 37 °C overnight, and the next day, medium change was performed, and compound solutions at different concentrations were added. Meanwhile, a negative control group (cells + DMSO) and a blank control group (medium + DMSO) were set, and the cells were incubated in the cell incubator with 5% CO₂ at 37 °C for 72 h. After the treatment was completed, procedures were performed according to the instructions of the kit, and luminescence signal values in different wells were measured using an EnVision plate reader (2104). The inhibition of the viability of HepG2 cells by the compounds at different concentrations was calculated according to the following formula. The concentration of the compound was plotted on the X-axis, and the inhibition rate was plotted on the Y-axis. The toxic effect (IC₅₀ value) of the compound on HepG2 was calculated using GraphPad Prism 8.0 software. $Inhibition rate \left(%\right) = \left(1-\frac{signal value of each compound group - signal value of blank control group}{signal value of negative control group - signal value of blank control group}\right) \times 100 %$

**Table 3: Assay results of toxicity on HepG2**

| Example | IC₅₀ (µM) |
|---|---|
| Compound 27A | > 100 |
| Compound 28A | > 100 |
| Compound 34A | > 100 |
| Compound 42A | > 100 |
| Compound 48A | > 100 |
| Compound 49A | > 100 |
| Compound 50A | > 100 |
| Compound 52A | > 100 |
| Compound 53A | > 100 |

The hepatotoxicity assay results demonstrate that the compounds of the present disclosure exhibit good safety with minimal hepatotoxicity.

### Test Example 4: Pharmacokinetic Assay in Mice

Pharmacokinetic assay in mice was performed, and the mice used were male ICR mice weighing 20-25 g and fasted overnight. Three mice were used, and oral intragastric administration was performed at a dose of 10 mg/kg. Blood was collected before administration and at 15 min, 30 min and 1 h, 2 h, 4 h, 8 h, and 24 h after administration. The blood samples were centrifuged at 6800 g at 2-8 °C for 6 min, and plasma was collected and stored at -80 °C. The plasma at each time point was taken, and 3-5 times its amount of an acetonitrile solution containing an internal standard was added for mixing. The mixture was vortexed for 1 min and then centrifuged at 13000 rpm at 4 °C for 10 min. The supernatant was taken, and 3 times its amount of water was added for mixing. An appropriate amount of the resulting mixture was taken for LC-MS/MS analysis. The major pharmacokinetic parameters were analyzed using a non-compartmental model in the WinNonlin 7.0 software.

**Table 4. Results of pharmacokinetic assay in mice**

| Compound | Cmax (ng/mL) | AUC₀₋ₜ (h*ng/mL) |
|---|---|---|
| Compound 28A | 4140 | 31492 |
| Compound 30A | 6624 | 45500 |
| Compound 31A | 7407 | 33271 |
| Compound 50A | 4045 | 29680 |
| Compound 53A | 2642 | 21770 |

The results of the pharmacokinetic assay in mice demonstrate that the compounds of the present disclosure exhibit excellent pharmacokinetic properties and good druggability.

### Test Example 5: Pharmacokinetic Assay in Rats

Pharmacokinetic assay in rats was performed, and the rats used were male SD rats weighing 180-240 g and fasted overnight. Three rats were used, and oral intragastric administration was performed at a dose of 10 mg/kg. Blood was collected before administration and at 15 min, 30 min and 1 h, 2 h, 4 h, 8 h, and 24 h after administration. The blood samples were centrifuged at 6800 g at 2-8 °C for 6 min, and plasma was collected and stored at -80 °C. The plasma at each time point was taken, and 3-5 times its amount of an acetonitrile solution containing an internal standard was added for mixing. The mixture was vortexed for 1 min and then centrifuged at 13000 rpm at 4 °C for 10 min. The supernatant was taken, and 3 times its amount of water was added for mixing. An appropriate amount of the resulting mixture was taken for LC-MS/MS analysis. The major pharmacokinetic parameters were analyzed using a non-compartmental model in the WinNonlin 7.0 software.

**Table 5. Results of pharmacokinetic assay in rats**

| Compound | Cmax (ng/mL) | AUC₀₋ₜ (h*ng/mL |
|---|---|---|
| Compound 27A | 3822 | 24330 |
| Compound 28A | 8730 | 76414 |
| Compound 30A | 7670 | 69406 |
| Compound 45A | 4545 | 37434 |
| Compound 53A | 3176 | 36630 |

The results of the pharmacokinetic assay in rats demonstrate that the compounds of the present disclosure exhibit excellent pharmacokinetic properties in rats and good druggability.

### Test Example 6: Assay for Inhibitory Effect on BSEP by Compounds

The assay for the inhibitory effect on bile salt export pump (BSEP) by the compounds was performed using a vesicle expressing human BSEP (GenoMembrane). The compounds at different concentrations were pre-incubated with the vesicle for 5 min, with a negative control (NC) group and a positive control (PC) group set up. The NC group involved pre-incubation of the vesicle with blank buffer at 37 °C for 5 min, and the PC group involved pre-incubation of the vesicle with a positive inhibitor at 37 °C for 5 min. Subsequently, with the addition ofATP or AMP separately, the mixture was incubated with the probe substrate at 37 °C for 5 min. The assay was terminated using pre-cooled Buffer B1 (10 × Buffer B1 (Stopping and Washing Buffer): 100 mM Hepes-Tris, 1000 mM KNO3, 500 mM Sucrose). The test samples were transferred to a 96-well filter plate, subjected to suction filtration by a vacuum pump, and then washed 5 times with 0.2 mL of pre-cooled Buffer B1. 50 µL of 80% methanol was used to dissolve the vesicles on the filter plate. After collection, the mixture was centrifuged at 2000 rpm for 2 min to collect the filtrate, and the process was repeated once. The two filtrates were combined and mixed well to obtain approximately 100 µL of filtrate. Pre-cooled methanol containing an internal standard was added, and the mixture was centrifuged at 12,000 rpm for 5 min. The supernatant was collected for LC-MS/MS quantification analysis to detect the amount of substrate transported. The concentration of the compound was plotted on the X axis, and the relative activity (% of NC) was plotted on the Y axis. The IC₅₀ value and the inhibition rate of the compound on inhibiting bile salt export pump activity were calculated using Prism software.

The transport rate (activity) and relative activity under different conditions were calculated according to the following formulas: $\begin{matrix}Substrate active transport rate \left(pmol/min/mg\right) = \\ \frac{transport amount of substrate driven by ATP - average transport amount of substrate driven by AMP \left(pmol\right)}{incubation time \left(min\right) \times vesicle protein amount \left(mg\right)}\end{matrix}$ $Relative activity \left(% of NC\right) = \frac{transport activity of PC sample or test sample}{transport activity of NC sample} %$

**Table 6. Inhibitory effect of test compounds on BSEP**

| Compound | IC₅₀ (µM) |
|---|---|
| Control compound 1 | 3.8 |
| Compound 24A | 65 |
| Compound 27A | 77 |
| Compound 28A | 79 |
| Compound 34A | > 100 |
| Compound 42A | > 100 |
| Compound 45A | 88 |
| Compound 48A | > 100 |
| Compound 53A | 95 |

The results of the assay for the inhibitory effect on BSEP by the compounds demonstrate that the compounds of the present disclosure exhibit no significant inhibitory effect on the BSEP, without the risk of cholestatic toxicity.

The structure of control compound 1 is as follows:

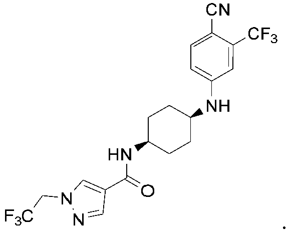

### Test Example 7: Thermodynamic Solubility Experiment

A phosphate-buffered saline (PBS) at pH 7.4 was prepared. The compounds were weighed out accurately and added to the prepared phosphate-buffered saline at pH 7.4 to prepare a solution at a concentration of 4 mg/mL. The prepared solution was shaken at 1000 rpm for 1 h, and then incubated at room temperature overnight. The incubated solution was centrifuged at 12,000 rpm for 10 min to remove undissolved particles, and the supernatant was transferred to a new centrifuge tube. After the supernatant was diluted properly, an acetonitrile solution containing an internal standard was added, and quantification was performed using a calibration curve prepared with the same matrix.

The thermodynamic solubility experiment results demonstrate that the compounds of the present disclosure exhibit good thermodynamic solubility and good druggability.

### Test Example 8: Human Liver Microsome Stability Assay

The human liver microsome stability assay was performed by co-incubating the compounds with human liver microsomes *in vitro.* Test compounds were first prepared as 10 mM stock solutions in DMSO solvent, followed by dilution of the compounds to 0.5 mM using acetonitrile. Human liver microsomes (Corning) were diluted with PBS to form a microsome/buffer solution, and 0.5 mM compound was diluted with this solution to form a working solution with a compound concentration of 1.5 µM and a human liver microsome concentration of 0.75 mg/mL. A deep-well plate was used, and 30 µL of the working solution was added to each well. Subsequently, 15 µL of pre-warmed 6 mM NADPH solution was added to start the reaction, and the plate was incubated at 37 °C. Reactions were terminated by adding 135 µL of acetonitrile to the corresponding wells at 0, 5, 15, 30, and 45 min of incubation. After the reaction was terminated with acetonitrile at the last 45 min time point, the deep-well plate was vortexed for 10 min (600 rpm/min) and then centrifuged for 15 min. The supernatant was collected after centrifugation, and purified water was added at a ratio of 1:1 for LC-MS/MS analysis to obtain the ratio of peak area of compound to peak area of internal standard at each time point. The peak area ratios of the compounds at 5, 15, 30, and 45 min were compared with the peak area ratio thereof at 0 min, and the remaining percentage of compounds at each time point was calculated. T_{1/2} was calculated using Graphpad 5 software.

**Table 8. Human liver microsome stability data for the compounds**

| Compound | Half-life (T_{1/2}, min) |
|---|---|
| Compound 27A | 220 |
| Compound 28A | 482 |
| Compound 30A | 560 |
| Compound 45A | 422 |
| Compound 50A | 355 |
| Compound 53A | 370 |

The results of the human liver microsome stability experiment demonstrate that the compounds of the present disclosure exhibit excellent human liver microsome stability and good druggability.

### Test Example 9: Assay for Inhibition of Cytochrome P450 by Compounds

The inhibition potential of the compounds against the cytochrome P450 (CYP450) isoform CYP3A4 (using two substrates, midazolam and testosterone) was detected. First, the test compounds were prepared as 10 mM stock solutions in DMSO solvent, and the CYP3A4 inhibitor ketoconazole was prepared as 10 mM, 2.5 mM, and 2.5 mM stock solutions in DMSO. The test compounds and ketoconazole were diluted with acetonitrile to a 400-fold final concentration (compound: 10 µM, ketoconazole: 2.5 µM).

A 4-fold final concentration of NADPH cofactor was prepared in potassium phosphate buffer (0.1 M, pH 7.4) (66.7 mg of NADPH in 10 mL of potassium phosphate buffer), and the substrates were prepared. The final concentration of the CYP3A4 substrate midazolam was 320 µM, and the final concentration of the CYP3A4 substrate testosterone was 20 µM.

Human liver microsome solution was prepared on ice using potassium phosphate buffer at a concentration of 0.2 mg/mL. The human liver microsome solution was used to prepare test compound solution and control inhibitor solution at a 2-fold final concentration on ice. 30 µL of the test compound solution and 30 µL of the control inhibitor solution were added to each test well, 15 µL of the substrates was added, and the process was repeated for replicate wells. The 96-well assay plate and NADPH solution were incubated at 37 °C for 5 min. 15 µL of pre-warmed 8 mM NADPH solution was added to the assay plate to start the reaction. The CYP3A4 assay plate was pre-incubated at 37 °C for 5 min. 120 µL of acetonitrile was added to terminate the reaction. After quenching, the plate was shaken on a vortexer (IKA, MTS 2/4) at 600 rpm/min for 10 min, followed by centrifugation for 15 min. The supernatant was collected after centrifugation, and purified water was added at a ratio of 1:1 for LC-MS/MS analysis to obtain the ratio of peak area of compound to peak area of internal standard. The peak area ratio of the compound was compared with the peak area ratio of the control inhibitor, and the inhibition rate was calculated.

The results of the assay for the inhibition of cytochrome P450 by the compounds demonstrate that the compounds of the present disclosure exhibit no significant inhibitory effect on CYP3A4 (using two substrates, midazolam and testosterone) and good druggability.

### Test Example 10: Evans Blue Vascular Permeability Experiment

The Evans blue vascular permeability experiment was performed using C57 mice, with the left hind toe used as the model group and the right hind toe as the negative control group. Intragastric administration was performed using different doses of the drug, and 2 h after administration, 0.4% Evans blue (Sigma-Aldrich, Lot# SHBP1253) solution was injected via the tail vein. 5-10 min later, C48/80 (Sigma-Aldrich, Lot# C2313) solution was injected subcutaneously into the left hind toe, while normal saline was injected into the right hind toe. After 15 min, the mice were sacrificed, and the thickness of the left and right hind toes was measured using a vernier caliper. The feet of the mice were excised, dried, and weighed. The toes were crushed, and Evans blue in the toes was dissolved using a mixture of acetone:normal saline (7:3). Absorbance was measured at 620 nm, and the absorbance per unit volume was calculated. The swelling rate of the mouse ear and the exudate amount per unit mass of Evans blue were calculated.

The results of the Evans blue vascular permeability experiment demonstrate that the compounds of the present disclosure exhibit good efficacy and are capable of significantly inhibiting C48/80-induced vascular leakage, with good druggability.

Although examples of the present disclosure are illustrated and described above, it can be understood that the above examples are exemplary and not to be construed as limiting the present disclosure, and that changes, modifications, substitutions, and alterations can be made to the above examples by those of ordinary skill in the art within the scope of the present disclosure.

## Claims

1. A compound represented by formula IA, or a tautomer, a stereoisomer, a hydrate, a solvate, a pharmaceutically acceptable salt, or a prodrug thereof: 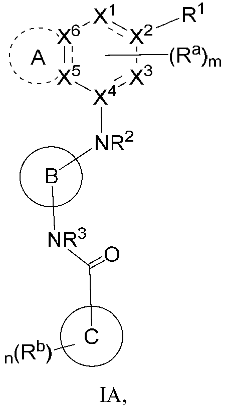 wherein
X¹, X², X³, X⁴, X⁵, and X⁶ each independently represent a ring atom;
X¹, X², X³, X⁵, and X⁶ are each independently N, CH₂, CH, or C;
X⁴ is C;
each of the bonds linked between X¹ and X² and between X⁵ and X⁶ is a single bond or a double bond;
in a group fragment 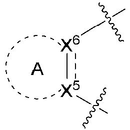 linked by X⁵ and X⁶, A is absent, and X⁵ and X⁶ are each independently substituted with R⁴ or R⁵; or when A is present, A, together with X⁵ and X⁶ ring atoms, forms 6- to 10-membered aryl, 3- to 11-membered heterocycloalkyl, or 5- to 10-membered heteroaryl, and A is further substituted with R^{c}; one or more R^{c} substitutions are present, and when a plurality of R^{c} substituents are present, the substituents are identical or different; heteroatoms in the heterocycloalkyl and heteroaryl are selected from one or more of N, O, and S, and the number of the heteroatoms is 1, 2, or 3;
and when A, together with X⁵ and X⁶ ring atoms, forms 6-membered aryl, R^{a} is oxo;
or, when A, together with X⁵ and X⁶ ring atoms, forms 5-membered heteroaryl, and when R^{c} is not H, R¹ is C₁₋₆ haloalkyl; heteroatoms in the heteroaryl are selected from one or more of N, O, and S, and the number of the heteroatoms is 1, 2, or 3;
ring B is C₃₋₁₂ cycloalkyl;
ring C is 6- to 10-membered aryl or 5- to 10-membered heteroaryl; heteroatoms in the heteroaryl are selected from one or more of N, O, and S, and the number of the heteroatoms is 1, 2, or 3;
R^{a}, R^{c}, R¹, R², R³, R⁴, and R⁵ are each independently H, halogen, cyano, amino, oxo, C₁₋₆ alkyl, C₁₋₆ alkyl substituted with hydroxyl, C₂₋₆ alkenyl, C₂₋₆ alkynyl, C₁₋₆ haloalkyl, C₃₋₈ cycloalkyl, halogenated C₃₋₈ cycloalkyl, -OR^{f}, - C(O)OR^{f}, -OC(O)R^{f}, -N(R^{f})₂, -N(R^{f})C(O)R^{f}, -N(R^{f})S(O)₂R^{f}, or -S(O)₂R^{f}; R^{f} is selected from H, C₁₋₆ alkyl, C₂₋₆ alkenyl, C₂₋₆ alkynyl, C₁₋₆ alkylamino, -(CH₂)rR^{g}, 6- to 10-membered aryl, C₃₋₈ cycloalkyl, 5- to 10-membered heteroaryl, or 5- to 10-membered heterocycloalkyl, or two R^{f} groups, together with an atom attached thereto, form 5- to 11-membered heterocycloalkyl; R^{g} is H, C₁₋₆ alkyl, C₂₋₆ alkenyl, C₂₋₆ alkynyl, C₃₋₈ cycloalkyl, 6- to 10-membered aryl, 5- to 10-membered heteroaryl, or 5- to 10-membered heterocycloalkyl; heteroatoms in the heterocycloalkyl and heteroaryl are selected from one or more of N, O, and S, and the number of the heteroatoms is 1, 2, or 3;
R^{b} is H, halogen, hydroxyl, amino, cyano, carbonyl, oxo, C₁₋₆ alkyl, C₃₋₈ cycloalkyl, C₂₋₆ alkenyl, C₂₋₆ alkynyl, C₁₋₆ haloalkyl, C₃₋₈ halocycloalkyl, C₁₋₆ hydroxyalkyl, C₁₋₆ alkylcarbonyl, C₁₋₆ alkoxy, C₁₋₆ haloalkoxy or 3- to 10-membered heterocycloalkyl, -(CHR^{j})₀₋₃-S(=O)(-NR^{j})-R^{k}, -(CHR^{j})₁₋₃-S(O)₂-R^{k}, -(CHR^{j})₁₋₃-OC(O)-N(R^{k})₂, - (CHR^{j})₀₋₃-(N=)S(=O)(R^{k})₂, or -(CHR^{j})₀₋₃-P(=O)(R^{k})₂; the C₁₋₆ alkyl, C₃₋₈ cycloalkyl, C₂₋₆ alkenyl, C₂₋₆ alkynyl, C₁₋₆ haloalkyl, C₃₋₈ halocycloalkyl, C₁₋₆ hydroxyalkyl, C₁₋₆ alkylcarbonyl, C₁₋₆ alkoxy, and C₁₋₆ haloalkoxy are optionally substituted with one or more R^{d}; R^{d} is selected from the following substituents: halogen, hydroxyl, amino, nitro, cyano, carbonyl, oxo, carboxyl, C₁₋₆ alkyl, C₂₋₆ alkenyl, C₂₋₆ alkynyl, C₁₋₆ haloalkyl, C₁₋₆ hydroxyalkyl, C₁₋₆ alkylcarbonyl, C₁₋₆ alkoxy, and C₁₋₆ haloalkoxy; when a plurality of R^{d} substituents are present, the R^{d} substituents are identical or different; heteroatoms in the heterocycloalkyl are selected from one or more of N, O, and S, and the number of the heteroatoms is 1, 2, or 3;
Rⁱ and R^{k} are each independently H, cyano, amino, C₁₋₆ alkyl, C₁₋₆ alkylamino, -(CH₂)₀₋₃C₃₋₈ cycloalkyl, -COC₁₋₆ alkyl, C₁₋₆ haloalkyl, C₁₋₆ alkyl substituted with hydroxyl, or unsubstituted 5- to 10-membered heterocycloalkyl; m, n, and r are 0, 1, 2, or 3, respectively.

2. The compound represented by formula IA, or the tautomer, the stereoisomer, the hydrate, the solvate, the pharmaceutically acceptable salt, or the prodrug thereof according to claim 1,
wherein R^{b} is H, F, Cl, -CN, methyl, amino, methoxy, oxo, -CH₂CF₃, -CF₃, -CHF₂, -CH₂CHF₂, -CH₂-SO₂-CH₃, - CH(CH₃)SO₂CH₃, -CH₂S(=O)(=NCH₃)-CH₃, 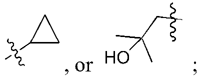
and/or, R^{a}, R^{c}, R¹, R², and R³ are each independently H, F, Cl, methyl, ethyl, amino, oxo, -CHF₂, -CH₂CF₃, -CF₃, 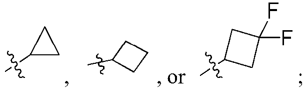
and/or, in the group fragment 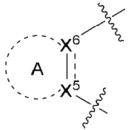 linked by X⁵ and X⁶, when A is absent and X⁵ and X⁶ are each independently substituted with R⁴ or R⁵, a structural fragment 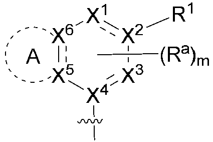 is selected from 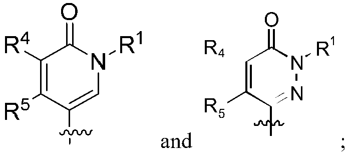
and/or, R⁴ and R⁵ are each independently H, F, Cl, methyl, -CF₃, or -CHF₂;
and/or, in the group fragment 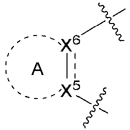 linked by X⁵ and X⁶, when A is absent and X⁵ and X⁶ are each independently substituted with R⁴ or R⁵, a structural fragment 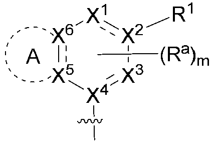 is selected from 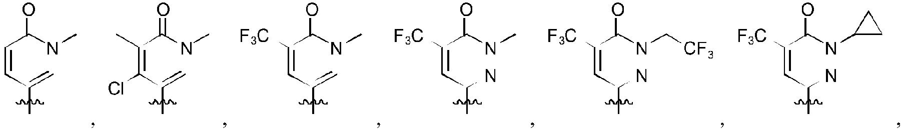 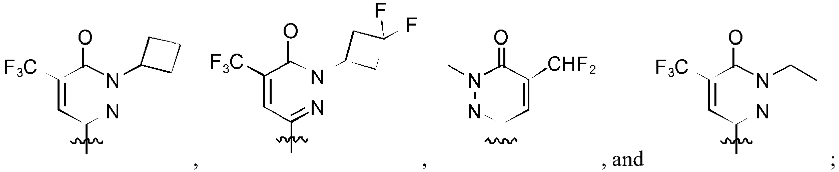
and/or, in the group fragment 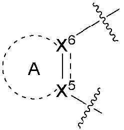 linked by X⁵ and X⁶, when A is present, A, together with X⁵ and X⁶ ring atoms, forms 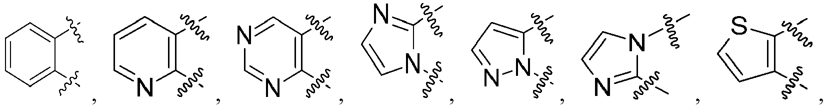 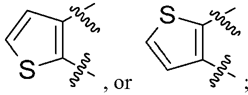 and A is further substituted with R^{c};
and/or, when A is present, the structural fragment 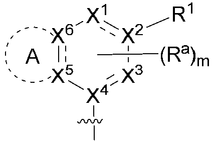 is selected from 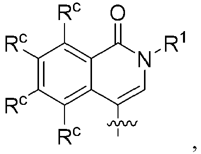 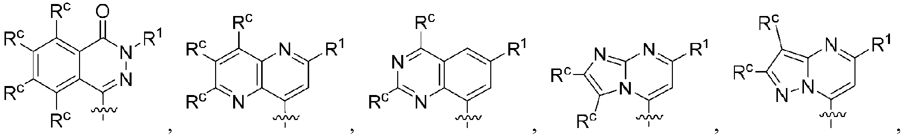 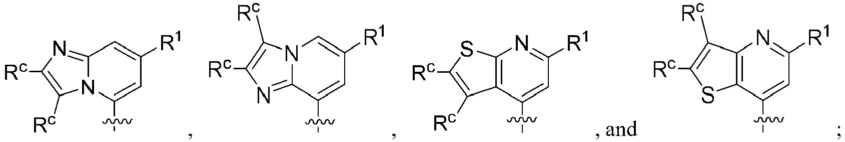
and/or, when A is present, the structural fragment 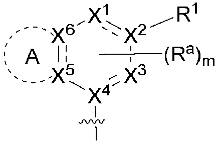 is selected from 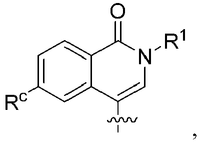 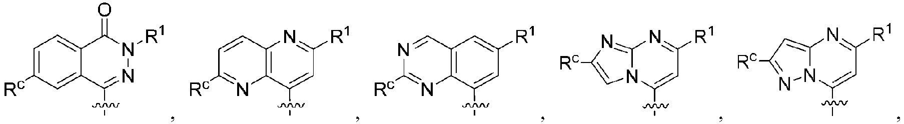 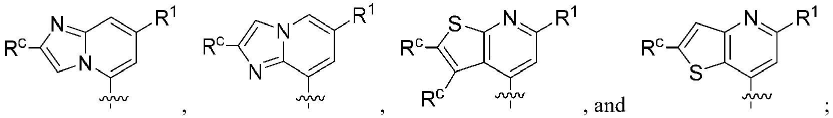
and/or, ring B is cyclopropyl, cyclobutyl, cyclopentyl, cyclohexyl, or cycloheptyl;
and/or, the structural fragment 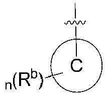 is selected from 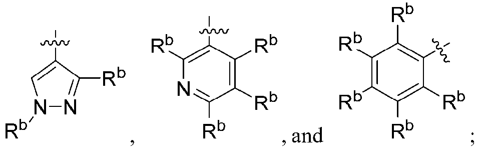
and/or, the structural fragment 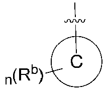 is selected from 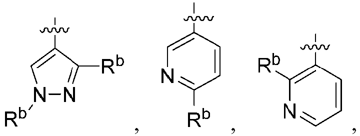 and

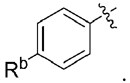

3. The compound represented by formula IA, or the tautomer, the stereoisomer, the hydrate, the solvate, the pharmaceutically acceptable salt, or the prodrug thereof according to claim 1, wherein:
the formula IA may be selected from IA-1 as follows: 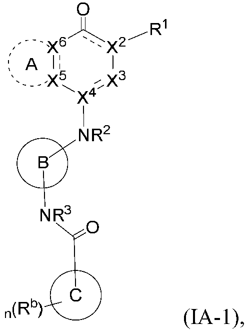
wherein A, ring B, ring C, X², X³, X⁴, X⁵, X⁶, R¹, R², R³, R^{b}, m, and n are as defined in claim 1;
or the formula IA is selected from the structure represented by formula I-1, I-2, or I-3 as follows, 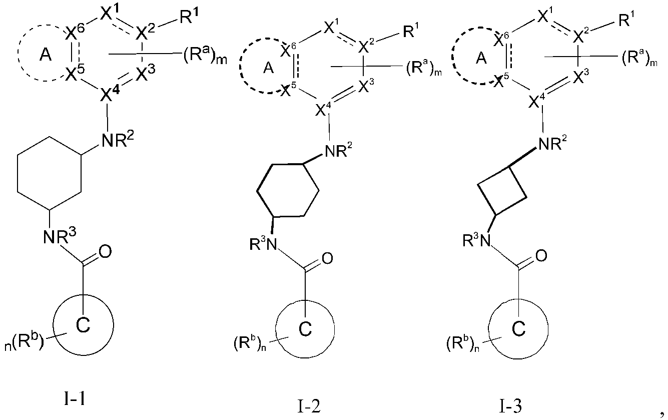
wherein
in a group fragment 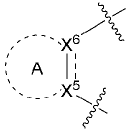 linked by X⁵ and X⁶, A is as defined in claim 1;
ring C is 6- to 10-membered aryl or 5- to 10-membered heteroaryl; heteroatoms in the heteroaryl are selected from one or more of N, O, and S, and the number of the heteroatoms is 1, 2, or 3;
X¹, X², X³, X⁴, X⁵, X⁶, R¹, R², R³, R^{a}, R^{b}, m, and n are as defined in claim 1;
or the formula IA is selected from the structure represented by formula II-1 or II-3 as follows, 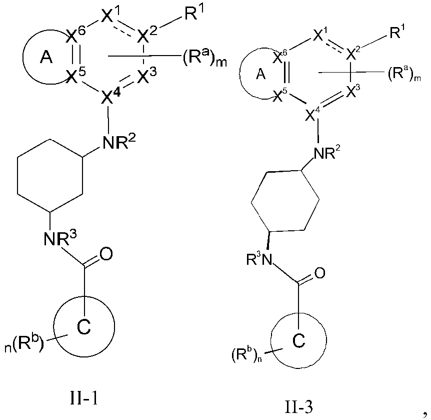
wherein A, together with X⁵ and X⁶ ring atoms, forms 6- to 8-membered aryl, 3- to 8-membered heterocycloalkyl, 5-membered heteroaryl, or 6-membered heteroaryl; the heteroatoms are independently selected from one or more of N, O, and S; A is further substituted with R^{c}; one or more R^{c} substitutions are present, and when a plurality of R^{c} substituents are present, the substituents are identical or different; and when A, together with X⁵ and X⁶ ring atoms, forms 6-membered aryl, R^{a} is oxo; or, when A, together with X⁵ and X⁶ ring atoms, forms 5-membered heteroaryl and R^{c} is not H, R¹ is C₁₋₆ haloalkyl; heteroatoms in the heterocycloalkyl and heteroaryl are selected from one or more of N, O, and S, and the number of the heteroatoms is 1, 2, or 3;
ring C is 6- to 10-membered aryl or 5- to 8-membered heteroaryl; heteroatoms in the heteroaryl are selected from one or more of N, O, and S, and the number of the heteroatoms is 1, 2, or 3;
R^{b} is H, halogen, hydroxyl, amino, cyano, carbonyl, oxo, C₁₋₆ alkyl, C₃₋₈ cycloalkyl, C₂₋₆ alkenyl, C₂₋₆ alkynyl, C₁₋₆ haloalkyl, C₃₋₈ halocycloalkyl, C₁₋₆ hydroxyalkyl, C₁₋₆ alkylcarbonyl, C₁₋₆ alkoxy, C₁₋₆ haloalkoxy or 3- to 8-membered heterocycloalkyl, -(CHR^{j})₀₋₃-S(=O)(-NR^{j})-R^{k}, -(CHR^{j})₁₋₃-S(O)₂-R^{k}, -(CHR^{j})₁₋₃-OC(O)-N(R^{k})₂, - (CHR^{j})₀₋₃-(N=)S(=O)(R^{k})₂, or -(CHR^{j})₀₋₃-P(=O)(R^{k})₂; the C₁₋₆ alkyl, C₃₋₈ cycloalkyl, C₂₋₆ alkenyl, C₂₋₆ alkynyl, C₁₋₆ haloalkyl, C₃₋₈ halocycloalkyl, C₁₋₆ hydroxyalkyl, C₁₋₆ alkylcarbonyl, C₁₋₆ alkoxy, and C₁₋₆ haloalkoxy are optionally substituted with one or more R^{d}; R^{d} is selected from the following substituents: halogen, hydroxyl, amino, nitro, cyano, carbonyl, oxo, carboxyl, C₁₋₆ alkyl, C₂₋₆ alkenyl, C₂₋₆ alkynyl, C₁₋₆ haloalkyl, C₁₋₆ hydroxyalkyl, C₁₋₆ alkylcarbonyl, C₁₋₆ alkoxy, and C₁₋₆ haloalkoxy; heteroatoms in the heterocycloalkyl are selected from one or more of N, O, and S, and the number of the heteroatoms is 1, 2, or 3;
R^{j} and R^{k} are each independently H, cyano, amino, C₁₋₆ alkyl, C₁₋₆ alkylamino, -(CH₂)₀₋₃C₃₋₈ cycloalkyl, -COC₁₋₆ alkyl, C₁₋₆ haloalkyl, C₁₋₆ alkyl substituted with hydroxyl, or unsubstituted 5- to 10-membered heterocycloalkyl; R^{a}, R^{c}, R¹, R², and R³ are each independently H, halogen, cyano, amino, oxo, C₁₋₆ alkyl, C₁₋₆ alkyl substituted with hydroxyl, C₂₋₆ alkenyl, C₂₋₆ alkynyl, C₁₋₆ haloalkyl, C₃₋₈ cycloalkyl, or halogenated C₃₋₈ cycloalkyl;
X¹, X², X³, X⁴, X⁵, X⁶, m, and n are as defined in claim 1;
and/or, R^{b} is H, F, Cl, -CN, methyl, amino, oxo, -CH₂CF₃, -CF₃, -CHF₂, -CH₂CHF₂, -CH₂-SO₂-CH₃, - CH(CH₃)SO₂CH₃, -CH₂S(=O)(=NCH₃)-CH₃, 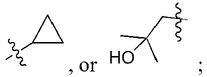
and/or, R^{a}, R^{c}, R¹, R², and R³ are each independently H, F, Cl, methyl, amino, oxo, -CHF₂, -CH₂CF₃, -CF₃,

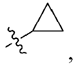

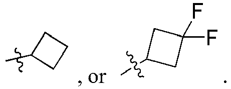

4. The compound represented by formula IA, or the tautomer, the stereoisomer, the hydrate, the solvate, the pharmaceutically acceptable salt, or the prodrug thereof according to claim 1, wherein the compound has a structure represented by formula II-2, II-4, or II-5, 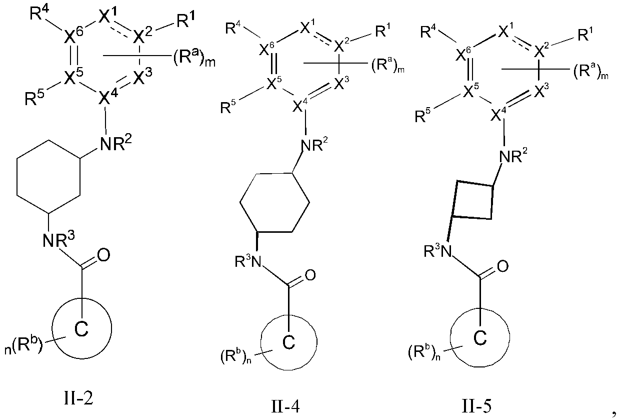 wherein
ring C is 6- to 10-membered aryl or 5- to 8-membered heteroaryl; heteroatoms in the heteroaryl are selected from one or more of N, O, and S, and the number of the heteroatoms is 1, 2, or 3;
R^{b} is H, halogen, hydroxyl, amino, cyano, carbonyl, oxo, C₁₋₆ alkyl, C₃₋₈ cycloalkyl, C₂₋₆ alkenyl, C₂₋₆ alkynyl, C₁₋₆ haloalkyl, C₃₋₈ halocycloalkyl, C₁₋₆ hydroxyalkyl, C₁₋₆ alkylcarbonyl, C₁₋₆ alkoxy, C₁₋₆ haloalkoxy or 3- to 8-membered heterocycloalkyl, -(CHR^{j})₀₋₃-S(=O)(-NR^{j})-R^{k}, -(CHR^{j})₁₋₃-S(O)₂-R^{k}, -(CHR^{j})₁₋₃-OC(O)-N(R^{k})₂, - (CHR^{j})₀₋₃-(N=)S(=O)(R^{k})₂, or -(CHR^{j})₀₋₃-P(=O)(R^{k})₂; the C₁₋₆ alkyl, C₃₋₈ cycloalkyl, C₂₋₆ alkenyl, C₂₋₆ alkynyl, C₁₋₆ haloalkyl, C₃₋₈ halocycloalkyl, C₁₋₆ hydroxyalkyl C₁₋₆ alkylcarbonyl, C₁₋₆ alkoxy, and C₁₋₆ haloalkoxy are optionally substituted with one or more R^{d}; R^{d} is selected from the following substituents: halogen, hydroxyl, amino, nitro, cyano, carbonyl, oxo, carboxyl, C₁₋₆ alkyl, C₂₋₆ alkenyl, C₂₋₆ alkynyl, C₁₋₆ haloalkyl, C₁₋₆ hydroxyalkyl, C₁₋₆ alkylcarbonyl, C₁₋₆ alkoxy, and C₁₋₆ haloalkoxy; heteroatoms in the heterocycloalkyl are selected from one or more of N, O, and S, and the number of the heteroatoms is 1, 2, or 3;
R^{j} and R^{k} are each independently H, cyano, amino, C₁₋₆ alkyl, C₁₋₆ alkylamino, -(CH₂)₀₋₃C₃₋₈ cycloalkyl, -COC₁₋₆ alkyl, C₁₋₆ haloalkyl, C₁₋₆ alkyl substituted with hydroxyl, or unsubstituted 5- to 10-membered heterocycloalkyl; R^{a}, R¹, R², R³, R⁴, and R⁵ are each independently H, halogen, cyano, amino, oxo, C₁₋₆ alkyl, C₂₋₆ alkenyl, C₂₋₆ alkynyl, C₁₋₆ haloalkyl, C₃₋₈ cycloalkyl, or halogenated C₃₋₈ cycloalkyl;
X¹, X², X³, X⁴, X⁵, X⁶, m, and n are as defined in claim 1;
and/or, R^{b} is H, F, Cl, -CN, methyl, methoxy, amino, oxo, -CH₂CF₃, -CF₃, -CHF₂,
-CH₂CHF₂, -CH₂-SO₂-CH₃, -CH(CH₃)SO₂CH₃, -CH₂S(=O)(=NCH₃)-CH₃, 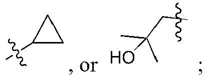
and/or, R^{a}, R¹, R², R³, R⁴, and R⁵ are each independently H, F, Cl, methyl, ethyl, amino, oxo, -CHF₂, -CH₂CF₃, - CF₃,

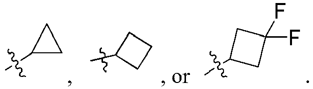

5. The compound represented by formula IA, or the tautomer, the stereoisomer, the hydrate, the solvate, the pharmaceutically acceptable salt, or the prodrug thereof according to any one of claims 1-4, wherein ring C is 6- to 8-membered aryl or 5- to 10-membered heteroaryl; heteroatoms in the heteroaryl are selected from one or more of N, O, and S, and the number of the heteroatoms is 1, 2, or 3;
and/or, ring C is 5- to 6-membered nitrogen-containing heteroaryl, and the number of the nitrogen atoms is 1, 2, or 3;
and/or, ring C is phenyl, pyrazolyl, or pyridinyl.

6. The compound represented by formula IA, or the tautomer, the stereoisomer, the hydrate, the solvate, the pharmaceutically acceptable salt, or the prodrug thereof according to claim 1, wherein the compound comprises: 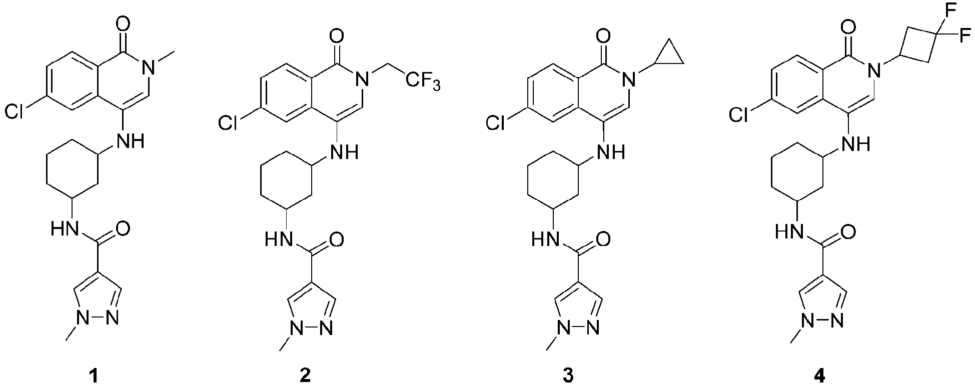 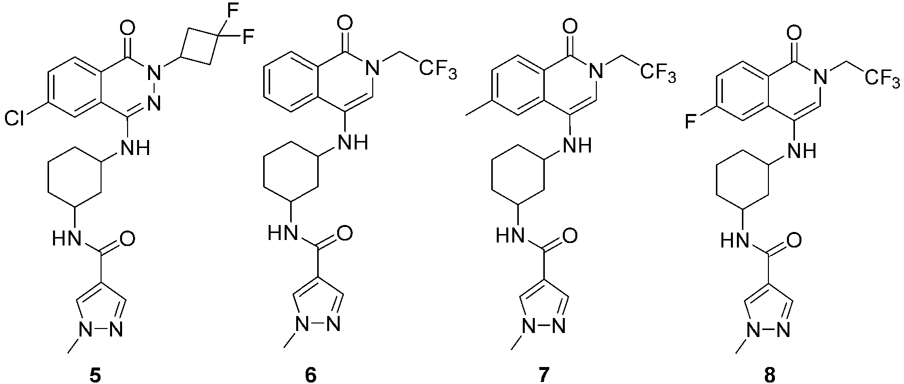 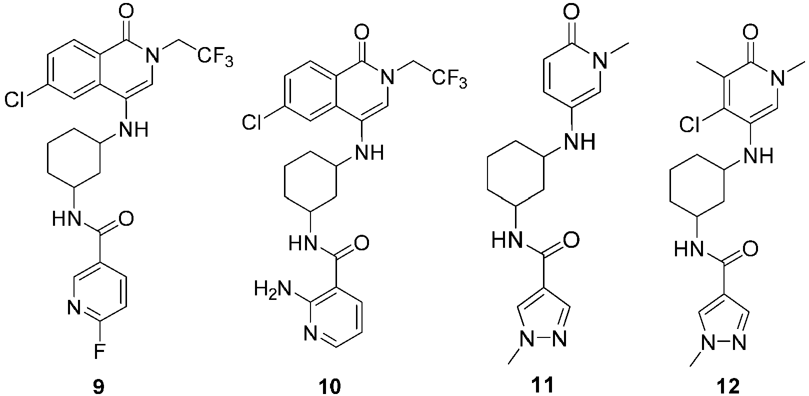 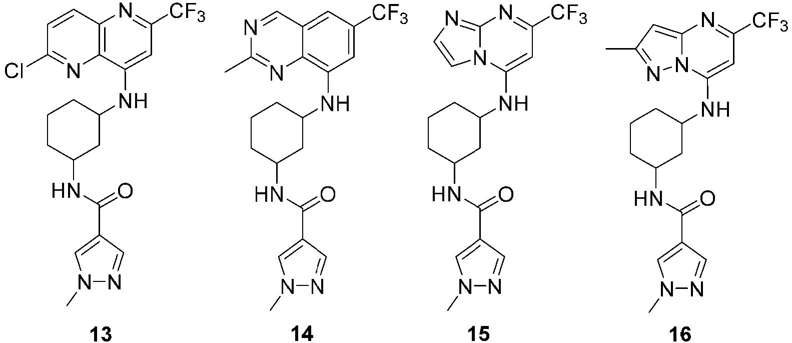 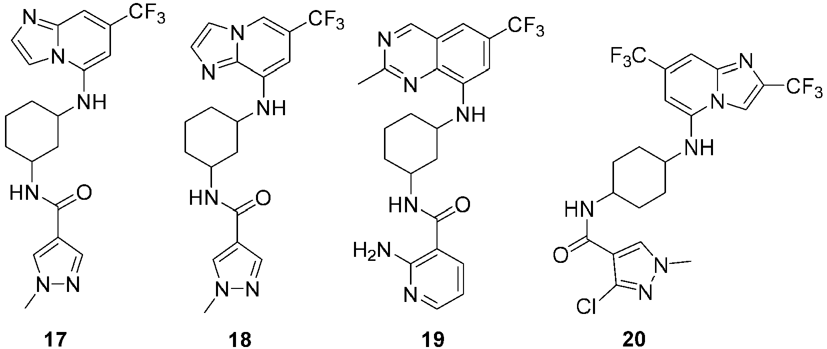 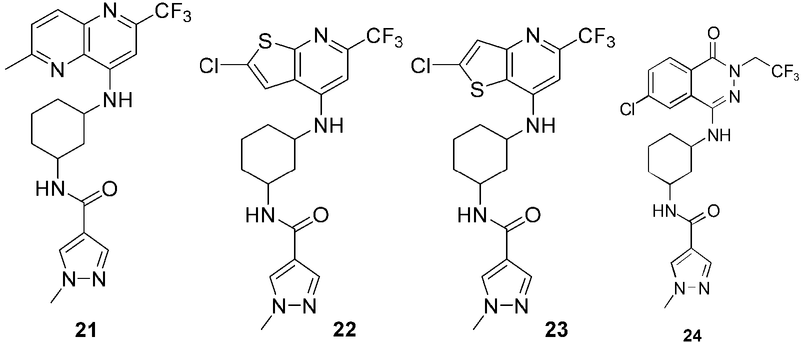 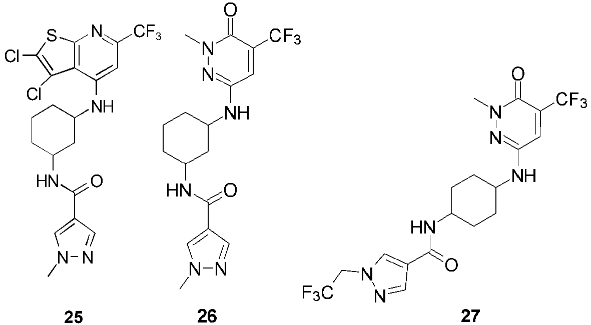 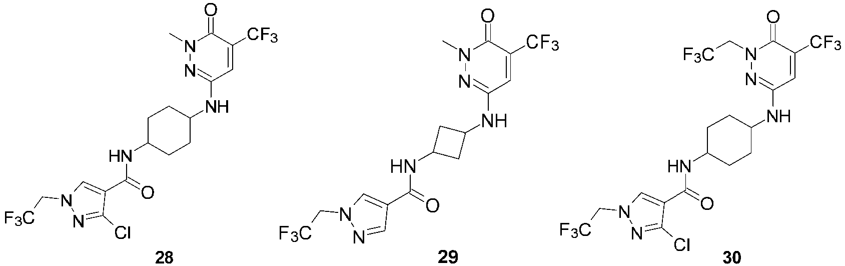 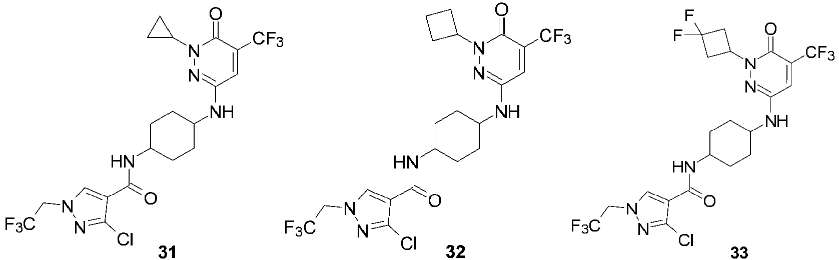 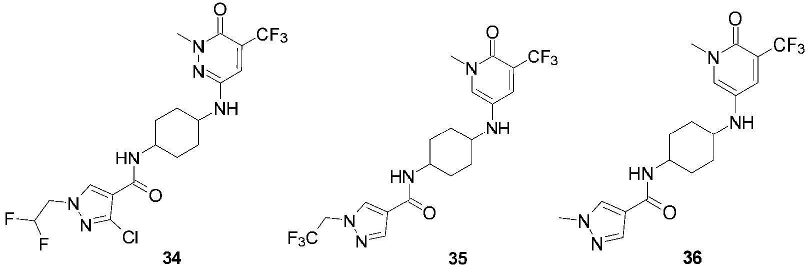 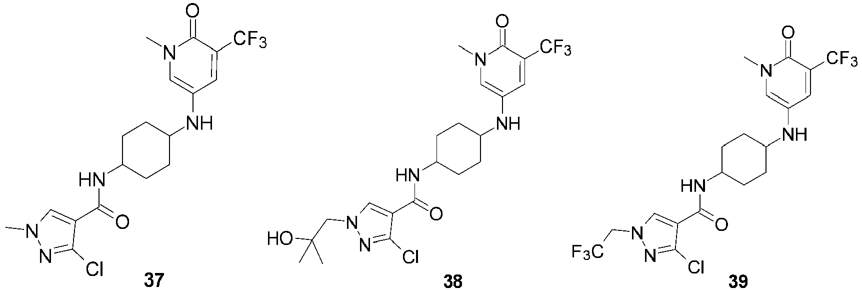 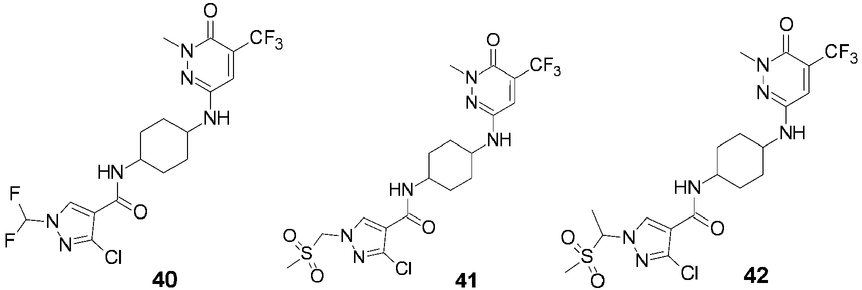 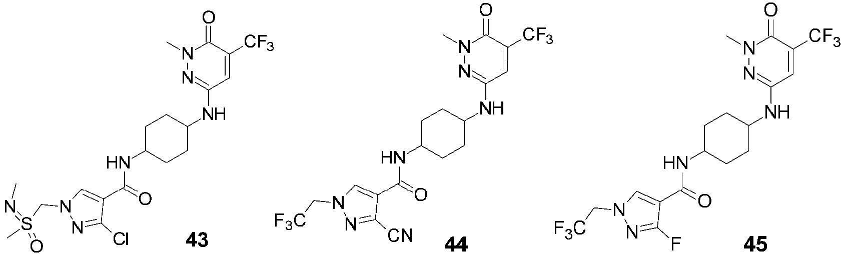 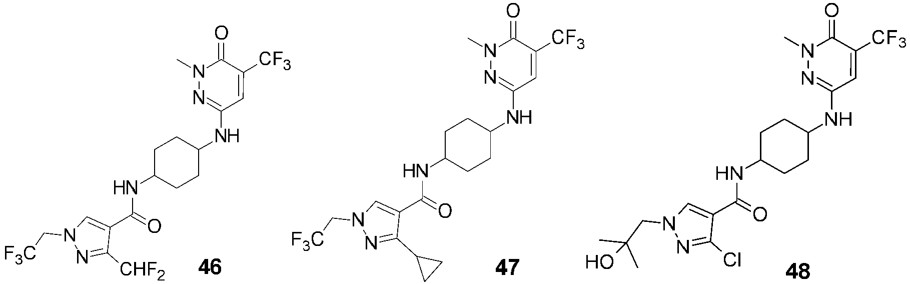 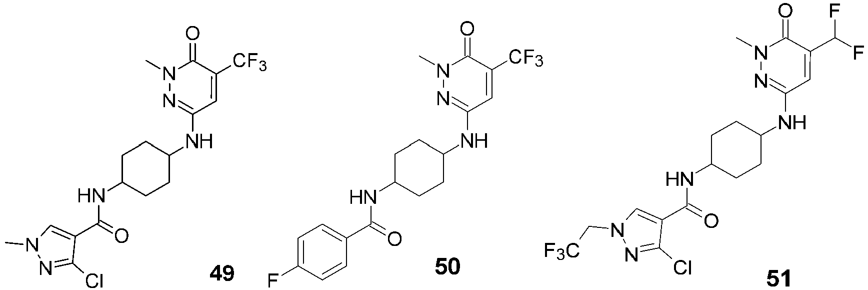 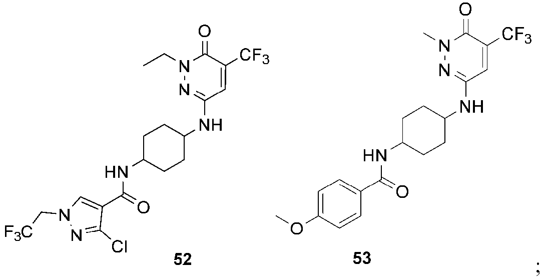 optionally, the pharmaceutically acceptable salt of the compound is a trifluoroacetate.

7. The compound represented by formula IA, or the tautomer, the stereoisomer, the hydrate, the solvate, the pharmaceutically acceptable salt, or the prodrug thereof according to claim 1, wherein the compound comprises: 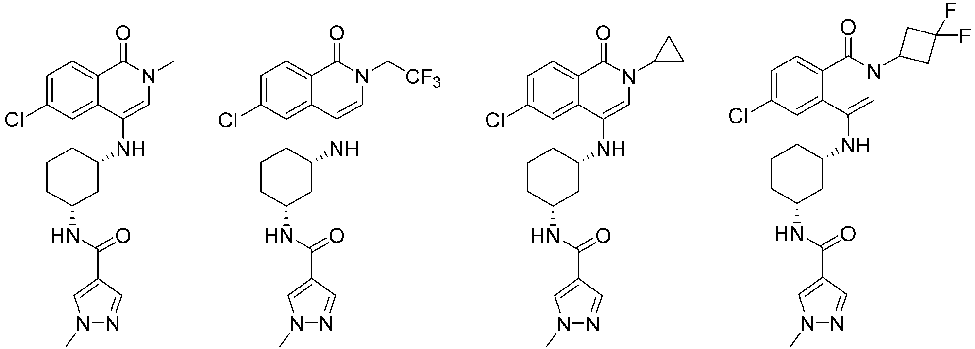 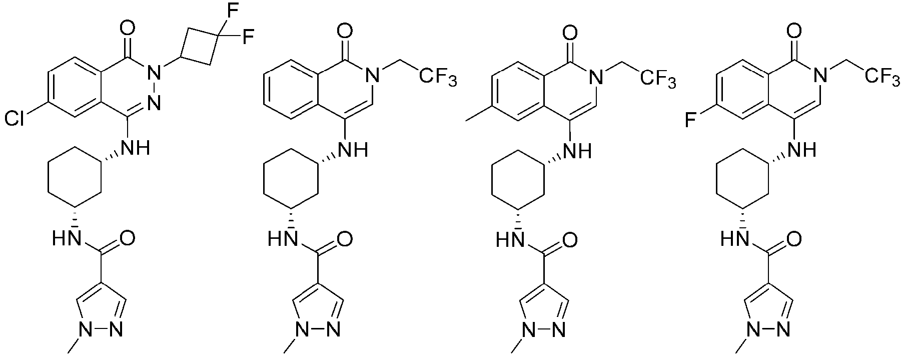 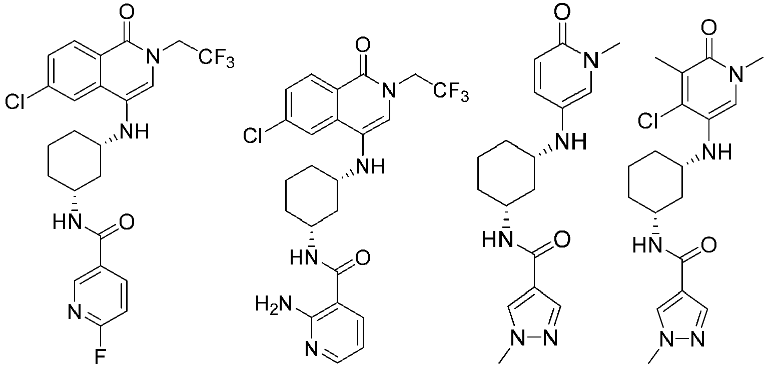 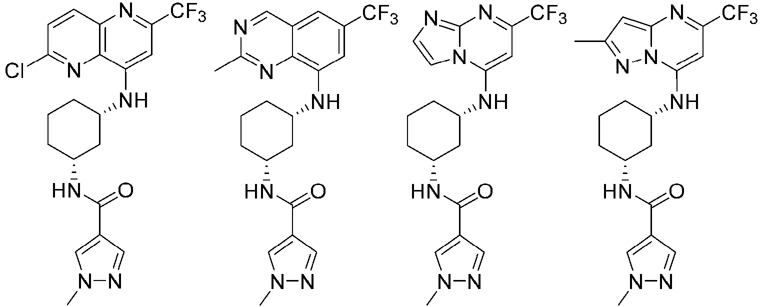 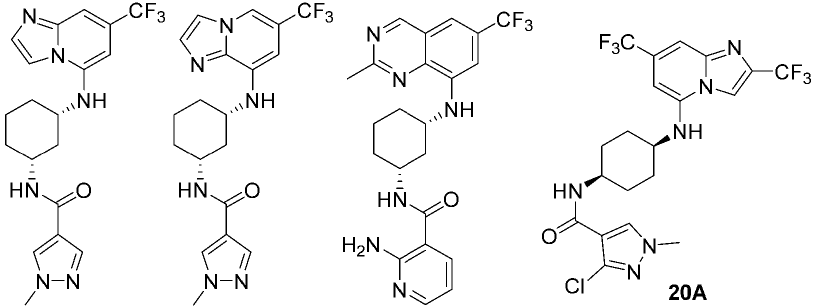 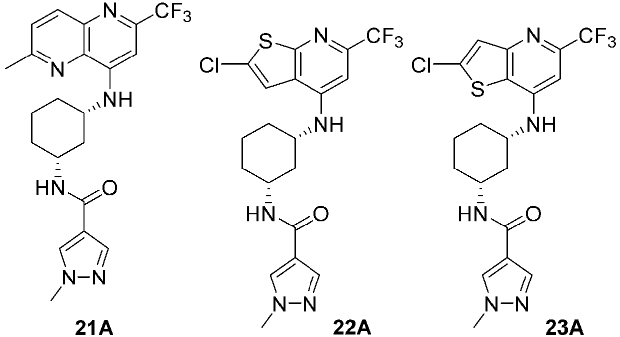 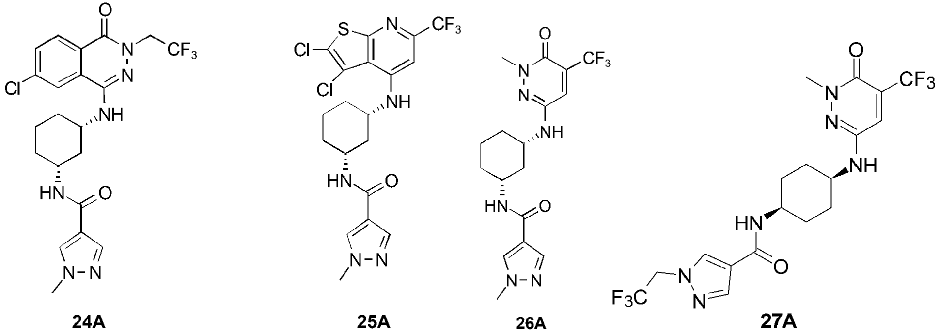 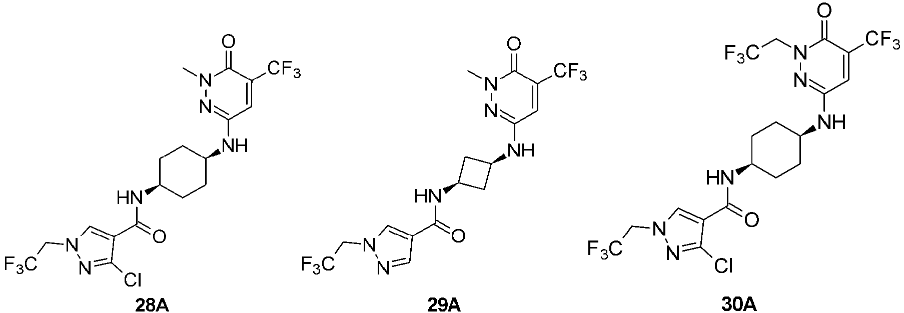 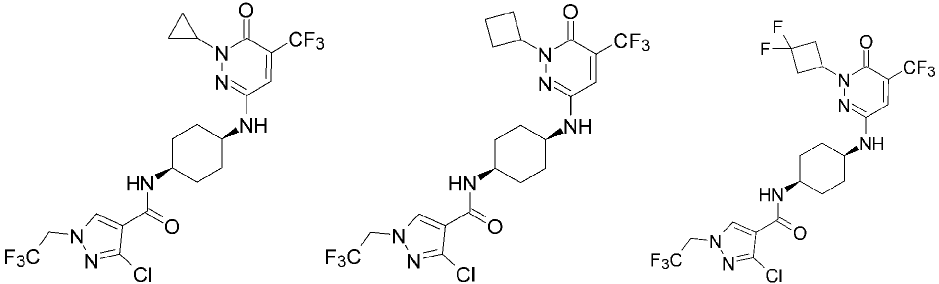 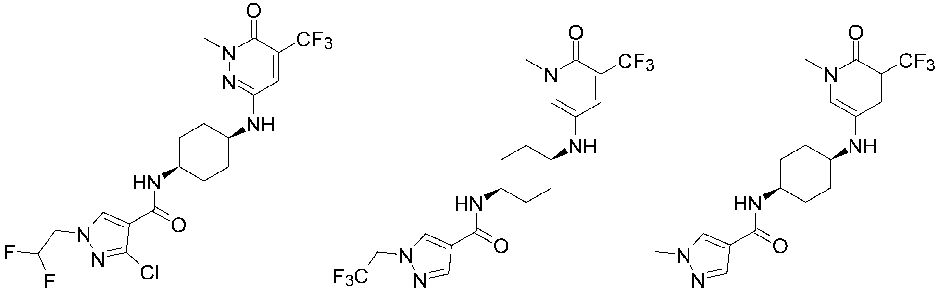 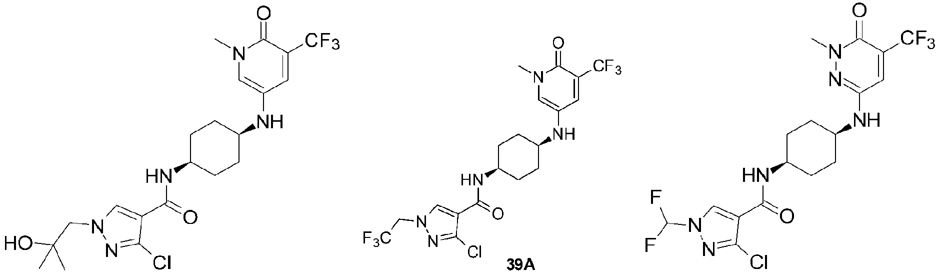 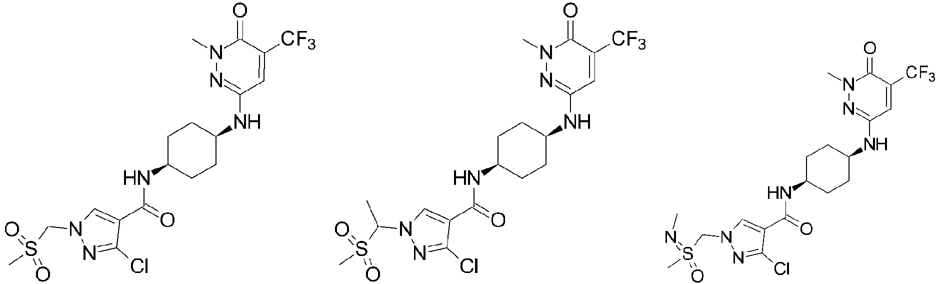 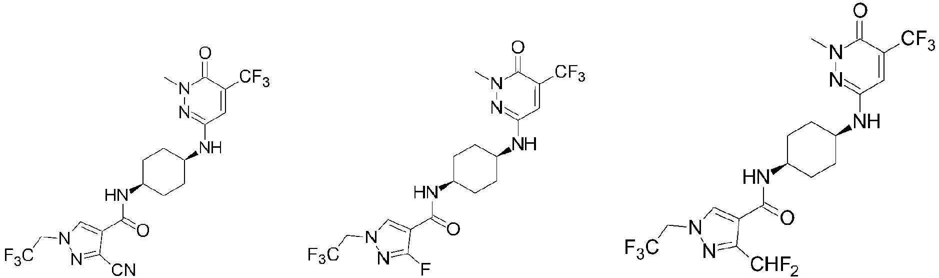 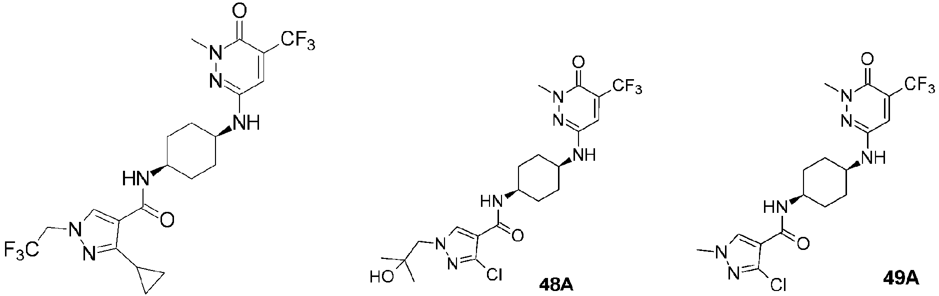 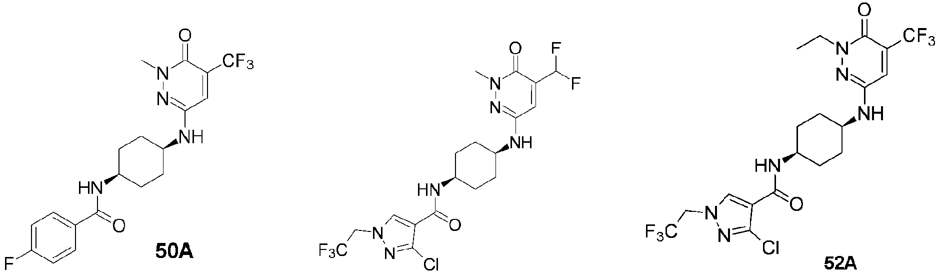 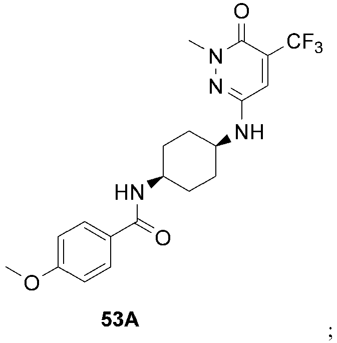 optionally, the pharmaceutically acceptable salt of the compound is a trifluoroacetate.

8. A pharmaceutical composition, comprising: the compound represented by formula IA, or the tautomer, the stereoisomer, the hydrate, the solvate, the pharmaceutically acceptable salt, or the prodrug thereof according to any one of claims 1-7; and a pharmaceutically acceptable carrier.

9. Use of the compound represented by formula IA, or the tautomer, the stereoisomer, the hydrate, the solvate, the pharmaceutically acceptable salt, or the prodrug thereof according to any one of claims 1-7, or use of the pharmaceutical composition according to claim 8, comprising:
antagonizing MRGPRX2;
and/or, preventing and/or treating a disease associated with MRGPRX2;
and/or, manufacturing a medicament, a pharmaceutical composition, or a formulation for use in antagonizing MRGPRX2 and/or for use in preventing and/or treating a disease associated with MRGPRX2.

10. The use according to claim 9, wherein the disease associated with MRGPRX2 comprises: a mast cell-related disease;
or the disease associated with MRGPRX2 comprises: skin diseases (e.g., atopic dermatitis, contact dermatitis, urticaria, chronic spontaneous urticaria, induced urticaria, and pruritus), autoimmune diseases (e.g., allergy, mastocytosis, rheumatoid arthritis, asthma, ulcerative colitis, and interstitial cystitis), and neurological diseases (e.g., pain).
